# EUROPEAN PATENT APPLICATION

(11) **EP 4 464 720 A2**
(43) Date of publication of application: **20.11.2024**
(21) Application number: 24203669.7
(22) Date of filing: 14.06.2019
(51) Int. Cl.: C07K 14/705

(54) **NON-HUMAN ANIMALS CAPABLE OF DH-DH REARRANGEMENT IN THE IMMUNOGLOBULIN HEAVY CHAIN CODING SEQUENCES**

(30) Priority: 14.06.2018 US 201862685203 P; 23.07.2018 US 201862702206 P; 01.03.2019 US 201962812580 P
(62) Divisional of application: 21213824.2
(71) Applicant: Regeneron Pharmaceuticals, Inc., Tarrytown, NY 10591 (US)
(72) Inventor: MURPHY, Andrew J, Tarrytown, 10591 (US); MACDONALD, Lynn, Tarrytown, 10591 (US); GUO, Chunguang, Tarrytown, 10591 (US); VORONINA, Vera, North Bethesda (US); MCWHIRTER, John, 18038 Sanremo IM (IT)
(74) Representative: J A Kemp LLP

(57) **Abstract**

Non-human animals and methods and compositions for making and using them are provided, which non-human animals have a genome comprising an engineered or recombinant diversity cluster within an immunoglobulin heavy chain variable region, which engineered or recombinant diversity cluster comprises an insertion of one or more D_{H} segments that are each operably linked to a 23-mer recombination signal sequence. Methods for producing antibodies from non-human animals are also provided, which antibodies optionally contain human variable regions and rodent, e.g., constant regions.

## Description

### CROSS-REFERENCE TO RELATED APPLICATIONS

This application claims the benefit under 35 U.S.C. § 119(3) of U.S. Provisional Application Serial Nos. 62/685,203 (filed June 14, 2018), 62/702,206 (filed July 23, 2018), and 62/812,580 (filed March 1, 2019), each of which application is hereby incorporated by reference in its entirety.

### SEQUENCE LISTING

The official copy of the sequence listing is submitted electronically via EFS-Web as an ASCII formatted sequence listing with a file named "10347_ST25.txt", which file was created on June 13, 2019, has a size of about 50 kilobytes, and is filed concurrently with the specification. The sequence listing contained in this ASCII formatted document is part of the specification and is herein incorporated by reference in its entirety.

### BACKGROUND

Monoclonal antibody products have revolutionized the biopharmaceutical industry and achieved significant advances in the treatment of several diseases. Many of these monoclonal antibody products have leveraged the natural features of antibody molecules (i.e., traditional immunoglobulin gene segments) and, in some instances, incorporated other features such as labeling (e.g., pegylated, radiolabeled) or conjugation with other drugs. At the current rate of approval, approximately 70 monoclonal antibody products are expected to be on the market by 2020. Despite these advances and the knowledge gained by the use of monoclonal antibodies for therapeutic use, diseases linked to targets that are difficult for monoclonal antibodies to bind and/or access persist, which highlights the need for different approaches for developing effective treatments.

### SUMMARY

The present invention encompasses the recognition that it is desirable to engineer non-human animals (e.g., a rodent (e.g., a rat, e.g., a mouse) to establish additional *in vivo* systems for identifying and developing new antibody-based therapeutics and, in some embodiments, antibody agents (e.g., monoclonal antibodies and/or fragments thereof), which can be used for the treatment of a variety of diseases. Further, the present invention also encompasses the desirability of non-human animals having an engineered heavy chain diversity (D_{H}) cluster (or engineered D_{H} region) within an immunoglobulin heavy chain variable region (e.g., a heterologous immunoglobulin heavy chain variable region, e.g., a human immunoglobulin heavy chain variable region) comprising one or more D_{H} segments engineered to be operably linked to a recombination signal sequence that permits D_{H}-to-D_{H} rearrangement, which may lead to the increased expression of antibodies containing complementary determining region three (CDR3s) that are characterized by a longer amino acid length as compared to wild-type (or reference) CDR3s and by diversity that, in some embodiments, directs binding to particular antigens. In some embodiments, non-human animals described herein provide *in vivo* systems for development of antibodies and/or antibody-based therapeutics for administration to humans.

Described herein are nucleotide molecules comprising at least one immunoglobulin heavy chain diversity (D_{H}) gene segment operably linked to a 23-mer recombination signal sequence (RSS), optionally wherein the D_{H} gene segment may be located within an engineered D_{H} region, e.g., of an immunoglobulin heavy chain variable region (e.g., a human or humanized immunoglobulin heavy chain variable region). As such, in some embodiments, a nucleotide molecule described herein comprises an engineered immunoglobulin heavy chain diversity (D_{H}) region that comprises at least one D_{H} gene segment operably linked to a 23-mer (RSS). In some embodiments, an engineered D_{H} region comprises (i) at least one D_{H} gene segment operably linked to a 23-mer RSS and (ii) an unrearranged D_{H} gene segment flanked on one side by a 12-mer RSS and on the other side by another 12-mer RSS, wherein the (i) at least one D_{H} gene segment operably linked to a 23-mer RSS and the (ii) germline D_{H} gene segment flanked on one side by a 12-mer RSS and on the other side by another12-mer RSS are operably linked such that (i) and (ii) are able to join in a D_{H}-D_{H} recombination event according to the 12/23 rule. Also provided are targeting vectors, non-human animals (e.g., rodents (e.g., rats or mice)) and non-human animal cells (e.g., rodent cells (e.g., rat cells or mouse cells) comprising a nucleotide molecule described herein, methods of using a nucleotide molecule described herein, etc.

In some embodiments, a D_{H} gene segment operably linked to a 23-mer RSS comprises a human D_{H} gene segment operably linked to a 23-mer RSS. In some embodiments, the human D_{H} gene segment comprises at least 19 nucleotides and/or encodes two cysteines. In some embodiments, the human D_{H} gene segment comprises at least 20 nucleotides and/or encodes two cysteines. In some embodiments, the human D_{H} gene segment comprises at least 23 nucleotides and/or encodes two cysteines. In some embodiments, the human D_{H} gene segment comprises at least 28 nucleotides and/or encodes two cysteines. In some embodiments, the human D_{H} gene segment comprises at least 31 nucleotides and/or encodes two cysteines. In some embodiments, the human D_{H} gene segment comprises at least 37 nucleotides and/or encodes two cysteines. In some embodiments, the human D_{H} gene segment operably linked to a 23-mer RSS comprises a human D_{H}2 gene segment operably linked to a 23-mer RSS. In some embodiments, the D_{H}2 gene segment comprises at least 30 nucleotides and/or encodes two cysteines. In some embodiments, the human D_{H} gene segment operably linked to a 23-mer RSS comprises a D_{H} gene segment selected from the group consisting of a human D_{H}3-3 gene segment, a D_{H}3-9 gene segment, a D_{H}3-10 gene segment, a D_{H}3-16 gene segment, a D_{H}3-22 gene segment, a human D_{H}2-2 gene segment, a human D_{H}2-8 gene segment, or a human D_{H}2-15 gene segment. In some embodiments, the human D_{H} gene segment operably linked to a 23-mer RSS comprises a human D_{H} gene segment selected from the group consisting of a human D_{H}3-3 gene segment, a human D_{H}2-2 gene segment, a human D_{H}2-8 gene segment, and a human D_{H}2-15 gene segment. In some embodiments, the human D_{H} gene segment operably linked to a 23-mer RSS comprises a human D_{H} gene segment selected from the group consisting of a human D_{H}3-3 gene segment, a human D_{H}2-2 gene segment, a human D_{H}2-8 gene segment, and a human D_{H}2-15 gene segment. In some embodiments, the human D_{H} gene segment comprises a human D_{H}3-3 gene segment. In some embodiments, the human D_{H} gene segment comprises human D_{H}2-2 gene segment. In some embodiments, the human D_{H} gene segment comprises a human D_{H}2-8 gene segment. In some embodiments, the human D_{H} gene segment comprises a human D_{H}2-15 gene segment.

In some embodiments, a D_{H} gene segment operably linked to a 23-mer RSS comprises: (a) a human D_{H}3-3 gene segment operably linked to a 23-mer RSS, optionally wherein the 23-mer RSS is contiguous to the 5'-end of the human D_{H}3-3 gene segment, (b) a human D_{H}2-2 gene segment operably linked to a 23-mer RSS, optionally wherein the 23-mer RSS is contiguous to the 3'-end of the human D_{H}2-2 gene segment, (c) a human D_{H}2-8 gene segment operably linked to a 23-mer RSS, optionally wherein the 23-mer RSS is contiguous to the 3'-end of the human D_{H}2-8 gene segment, (d) a human 2-15 gene segment operably linked to a 23-mer RSS, optionally wherein the 23-mer RSS is contiguous to the 3'-end of the human D_{H}2-15 gene segment, or (e) any combination of (a)-(d)

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:52.

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:61.

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:70.

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:71.

In some embodiments, a nucleotide molecule described herein (e.g., a D_{H} gene segment operably linked to a 23-mer RSS, an engineered D_{H} region, an immunoglobulin heavy chain variable region, etc.) comprises a nucleotide sequence comprising the sequence set forth as SEQ ID NO:72.

In some embodiments, a D_{H} gene segment operably linked to a 23-mer RSS comprises from 5' to 3' the 23-mer RSS and the D_{H} gene segment, e.g., comprises from 5' to 3' a 23-mer RSS, a (human) D_{H} gene segment, and a 12-mer RSS, e.g., the 23-mer RSS is contiguous to the 5'-end of the D_{H} gene segment, e.g., the D_{H} gene segment is operably linked to a 5'-end 23-mer RSS. In some embodiments, the D_{H} gene segment operably linked to a 23-mer RSS comprises a human D_{H}3-3 gene segment operably linked to a 5' end 23-mer RSS, e.g., the nucleotide molecule comprises from 5' to 3' a 23-mer RSS, the human D_{H}3-3 gene segment, and a 12-mer RSS.

Engineered D_{H} regions comprising at least one D_{H} gene segment with a 5'-end 23-mer RSS may further comprise an unrearranged D_{H} gene segment that is flanked on one side by a 12-mer RSS and on the other side by another12-mer RSS (e.g., the unrearranged D_{H} gene segment that is flanked on one side by a 12-mer RSS and on the other side by another12-mer RSS comprises a germline D_{H} gene segment, e.g., a D_{H} gene segment in its germline configuration, etc.) wherein the unrearranged D_{H} gene segment is upstream of and operably linked to the at least one D_{H} gene segment operably linked to a 5'-end 23-mer. In some embodiments, the unrearranged D_{H} gene segment flanked on one side by a 12-mer RSS and on the other side by another12-mer RSS comprises an unrearranged human D_{H} gene segment flanked on one side by a 12-mer RSS and on the other side by another12-mer RSS.

In some embodiments, a D_{H} gene segment operably linked to a 23-mer RSS comprises from 5' to 3' the D_{H} gene segment and the 23-mer RSS, e.g., comprises from 5' to 3' a 12-mer RSS, a (human) D_{H} gene segment, and a 23-mer RSS, e.g., the 23-mer RSS is contiguous to the 3'-end of the D_{H} gene segment, e.g., the D_{H} gene segment is operably linked to a 3'-end 23-mer, etc. In some embodiments, the D_{H} gene segment operably linked to a 3' end 23-mer RSS comprises a human D_{H}2 gene segment operably linked to a 3'-end 23-mer RSS, wherein the D_{H}2 gene segment is selected from the group consisting of a human D_{H}2-2 gene segment, a human D_{H}2-8 gene segment, and a human D_{H}2-15 gene segment. In some embodiments, a D_{H} gene segment operably linked to a 23-mer RSS comprises from 5' to 3': a human D_{H}2-2 gene segment operably linked to a 3' end 23-mer RSS, a human D_{H}2-8 gene segment operably linked to a 3' end 23-mer RSS, and a human D_{H}2-15 gene segment operably linked to a 3' end 23-mer RSS. In some embodiments, a D_{H} gene segment operably linked to a 3' end 23-mer RSS comprises from 5' to 3': a first contiguous nucleotide sequence comprising a 12-mer RSS, a human D_{H}2-2 gene segment, and a 23-mer RSS, a second contiguous nucleotide sequence comprising a 12-mer RSS, a human D_{H}2-8 gene segment, and a 23-mer RSS, and a third contiguous nucleotide sequence comprising a 12-mer RSS, a human D_{H}2-15 gene segment, and a 23-mer RSS.

Engineered D_{H} regions comprising at least one D_{H} gene segment operably linked to a 3'-end 23-mer RSS may further comprise an unrearranged D_{H} gene segment that is flanked on one side by a 12-mer RSS and on the other side by another12-mer RSS (e.g., the unrearranged D_{H} gene segment that is flanked on one side by a 12-mer RSS and on the other side by another12-mer RSS may comprise a germline D_{H} gene segment, e.g., a D_{H} gene segment in its germline configuration, etc.) wherein the unrearranged D_{H} gene segment flanked on one side by a 12-mer RSS and on the other side by another12-mer RSS is downstream of and operably linked to the at least one D_{H} gene segment operably linked to a 3'-end 23-mer RSS. In some embodiments, the unrearranged D_{H} gene segment flanked on one side by a 12-mer RSS and on the other side by another12-mer RSS comprises an unrearranged human D_{H} gene segment flanked on one side by a 12-mer RSS and on the other side by another12-mer RSS.

In some embodiments, an engineered D_{H} region as described herein comprises (i) one or more unrearranged human D_{H} gene segments, wherein each of the one or more of human D_{H} gene segments is flanked on its 5' and 3' ends by a 12-mer RSS, and (ii) at least one D_{H} gene segment operably linked to a 23-mer RSS comprising a human D_{H} gene segment, e.g., a human D_{H}3-3 gene segment) operably linked at its 5'-end to a 23-mer RSS. In some embodiments, an engineered DH region as described herein comprises from 5' to 3'(i) at least one D_{H} gene segment operably linked to a 23-mer RSS, e.g., at least one human D_{H}2 gene segment operably linked at its 3' end to a 23-mer RSS, optionally wherein the at least one human D_{H}2 gene segment operably linked at its 3'end to a 23-mer RSS comprises a human D_{H}2-2 gene segment operably linked at its 3' end to a 23-mer RSS, a human D_{H}2-8 gene segment operably linked at its 3' end to a 23-mer RSS, a human D_{H}2-15 gene segment operably linked at its 3' end to a 23-mer RSS, or any combination thereof, and (ii) one or a plurality of human D_{H} gene segments, wherein each of the one or plurality of human D_{H} gene segments is flanked on its 5' and 3' ends by a 12-mer RSS.

In some embodiments, an engineered D_{H} region as described herein comprises only human D_{H} gene segments.

In some embodiments, a nucleotide molecule described herein (e.g., engineered D_{H} regions, immunoglobulin heavy chain variable regions, etc.) comprises a D_{H} gene segment operably linked to a 23-mer RSS and an unrearranged D_{H} gene segment flanked on one side by a 12-mer RSS and on the other side by another12-mer RSS, wherein the D_{H} gene segment operably linked to a 23-mer RSS and the unrearranged D_{H} gene segment flanked on one side by a 12-mer RSS and on the other side by another12-mer RSS have not undergone recombination with (i) another D_{H} gene segment, (ii) a V_{H} gene segment, (iii) a J_{H} gene segment, or (iv) any combination thereof. In some embodiments, nucleotide molecules described herein include molecules comprising an engineered D_{H} regions comprising one or more D_{H} gene segments that have recombined with (i) another D_{H} gene segment, (ii) a V_{H} gene segment, (iii) a J_{H} gene segment, or any combination thereof, e.g., nucleotide molecules comprising a rearranged VDJ or VDDJ encoding sequence that encodes an immunoglobulin heavy chain variable region.

Accordingly, in some embodiments, a nucleotide molecule described herein comprising an engineered D_{H} region as described herein further comprises in operable linkage: (a) at least one unrearranged immunoglobulin heavy chain variable (V_{H}) gene segment (e.g., an unrearranged human V_{H}6-1 gene segment) that is upstream of and operably linked to an engineered D_{H} region, (b) at least one unrearranged immunoglobulin heavy chain joining (J_{H}) gene segment (e.g., an unrearranged human J_{H}6) gene segment that is downstream of and operably linked to the engineered D_{H} region, or a combination of (a) and (b).

In some embodiments, the at least one unrearranged V_{H} gene segment comprises the full repertoire of functional unrearranged human V_{H} gene segments spanning between and including the unrearranged human V_{H}3-74 and unrearranged human V_{H}1-6 gene segments. In some embodiments, the at least one unrearranged V_{H} gene segment comprises the full repertoire of functional unrearranged human V_{H} gene segments spanning between and including the unrearranged human V_{H}3-74 and unrearranged human V_{H}1-6 gene segments in germline configuration. In some embodiments, the at least one unrearranged J_{H} gene segment comprises an unrearranged human J_{H}4 gene segment, an unrearranged human J_{H}5 gene segment, and an unrearranged human J_{H}6 gene segment. In some embodiments, the at least one unrearranged J_{H} gene segment comprises an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment. In some embodiments, the at least one unrearranged J_{H} gene segment comprises an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment in germline configuration.

In some embodiments, a nucleotide molecule described herein comprises an immunoglobulin heavy chain variable (V_{H}) region comprising an engineered D_{H} region as described herein, e.g., comprises in operable linkage from 5' to 3':
(a) at least one unrearranged immunoglobulin heavy chain variable (V_{H}) gene segment,
(b) an engineered D_{H} region comprising at least one D_{H} gene segment operably linked to a 23-mer RSS, and
(c) at least one unrearranged immunoglobulin heavy chain joining (J_{H}) gene segment.

In some embodiments,
(a) the at least one unrearranged V_{H} gene segment comprises
   (i) an unrearranged human V_{H}6-1 gene segment,
   (ii) an unrearranged human V_{H}2-1 gene segment and an unrearranged human V_{H}6-1 gene segment and/or
   (iii) all functional unrearranged human V_{H} gene segments spanning between and including the unrearranged human V_{H}3-74 to unrearranged human V_{H}6-1 gene segment, e.g., all functional unrearranged human V_{H} gene segments in germline configuration, optionally, wherein a rodent Adam6 gene replaces a pseudogene between the unrearranged human V_{H}2-1 and V_{H}6-1 gene segments;
(b) the engineered D_{H} region comprises from 5' to 3':
   (i) one or more, e.g., a plurality of, unrearranged human D_{H} gene segments, wherein each of the plurality of unrearranged human D_{H} gene segments is flanked on its 5' and 3' ends by a 12-mer RSS, and a human D_{H} gene segment operably linked at its 5'-end to a 23-mer RSS, optionally wherein the plurality of unrearranged human D_{H} gene segments comprises the unrearranged human D_{H} gene segments spanning between and including the unrearranged human D_{H}1-1 gene segment and unrearranged human D_{H}1-26 gene segment in germline configuration and/or wherein human gene segment operably linked at its 5'-end to a 23-mer RSS is a human D_{H}3-3 gene segment, e.g., wherein the engineered D_{H} comprises the full repertoire of unrearranged human D_{H} gene segments in germline configuration with the exception of the unrearranged human D_{H}7-27 gene segment, which is replaced with the D_{H}3-3 gene segment operably linked to a 5'-end 23-mer RSS;
   (ii) at least one human D_{H} gene segment operably linked at its 3'-end with a 23-mer RSS, and one or more, e.g., a plurality of, human D_{H} gene segments, wherein each of the one or plurality of human D_{H} gene segment(s) is flanked on its 5' and 3' ends by a 12-mer RSS, optionally wherein the at least one human D_{H} gene segment operably linked at its 3'-end with a 23-mer RSS comprises a D_{H}2-2 gene segment operably linked at its 3' end to a 23-mer RSS, a human D_{H}2-8 gene segment operably linked at its 3' end to a 23-mer RSS, a human D_{H}2-15 gene segment operably linked at its 3' end to a 23-mer RSS and/or wherein the one or plurality of human D_{H} gene segment(s) comprises the D_{H} gene segments spanning between and including the unrearranged human D_{H}1-1 gene segment and the unrearranged human D_{H}7-27 gene segment, optionally wherein the engineered D_{H} comprises the full repertoire of unrearranged human D_{H} gene segments in germline configuration with the exception that the unrearranged human D_{H}2-2, D_{H}2-8, and D_{H}2-15 gene segments are respectively replaced with a D_{H}2-2 gene segment operably linked at its 3' end to a 23-mer RSS, a human D_{H}2-8 gene segment operably linked at its 3' end to a 23-mer RSS, and a human D_{H}2-15 gene segment operably linked at its 3' end to a 23-mer RSS;
   (iii) or a combination of (b)(i) and (b)(ii);and
(c) the at least one unrearranged J_{H} gene segment comprises
   (i) an unrearranged human J_{H}6 gene segment,
   (ii) an unrearranged human J_{H}4 gene segment, an unrearranged human J_{H}5 gene segment, and an unrearranged human J_{H}6 gene segment, and/or
   (iii) the full repertoire of unrearranged human J_{H} gene segments, e.g., an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment, optionally wherein the unrearranged human J_{H}1, J_{H}2, J_{H}3, J_{H}4, J_{H}5, and J_{H}6 gene segments are in germline configuration. In some embodiments, the immunoglobulin V_{H} region (comprising at least one functional V_{H} gene segment, the engineered D_{H} region, and at least one functional J_{H} gene segment) is a human immunoglobulin V_{H} region, e.g., each gene segment (e.g., each V_{H}, D_{H}, and J_{H} gene segment therein), including the D_{H} gene segment operably linked to a 23-mer RSS, is a human (V_{H}, D_{H}, or J_{H}) gene segment.

In some embodiments, a nucleotide molecule comprising an immunoglobulin V_{H} region as described herein comprises in operable linkage from 5' to 3'
(a) at least an unrearranged human V_{H}6-1 gene segment, e.g., all or a portion of all the functional unrearranged human V_{H} gene segments spanning between and including the unrearranged human V_{H}3-74 to unrearranged human V_{H}6-1 gene segment, e.g., the full repertoire of functional unrearranged human V_{H} gene segments, optionally including a rodent Adam6 gene between two unrearranged human V_{H} gene segments, e.g., (e.g., wherein the rodent Adam6 gene is located between a human V_{H}1-2 gene segment and a human V_{H}6-1 gene segment)
(b) a human engineered D_{H} region comprising from 5' to 3' the unrearranged human D_{H} gene segments spanning between and including the unrearranged human D_{H}1-1 gene segment and unrearranged human D_{H}1-16 gene segment in germline configuration and an unrearranged human D_{H}3-3 gene segment operably linked to a 5'-end 23-mer RSS, e.g., the full repertoire of unrearranged human D_{H} gene segments in germline configuration with the exception of the unrearranged human D_{H}7-27 gene segment, which is replaced with an unrearranged human D_{H}3-3 gene segment operably linked to a 5'-end 23-mer RSS, and
(c) at least an unrearranged human J_{H}6 gene segment.

In some embodiments, a nucleotide molecule comprising an immunoglobulin V_{H} region as described herein comprises in operable linkage from 5' to 3':
(a) at least an unrearranged human V_{H}6-1 gene segment, e.g., all or a portion of all the functional unrearranged human V_{H} gene segments spanning between and including the unrearranged human V_{H}3-74 to unrearranged human V_{H}6-1 gene segment, e.g., the full repertoire of functional unrearranged human V_{H} gene segments, optionally including a rodent Adam6 gene between two unrearranged human V_{H} gene segments, e.g., (e.g., wherein the rodent Adam6 gene is located between a human V_{H}1-2 gene segment and a human V_{H}6-1 gene segment)
(b) a human engineered D_{H} region comprising from 5' to 3' the unrearranged human D_{H} gene segments spanning between and including the unrearranged human D_{H}1-1 gene segment and unrearranged human D_{H}1-26 gene segment in germline configuration, and an unrearranged human D_{H}3-3 gene segment operably linked to a 5'-end 23-mer RSS, e.g., the full repertoire of unrearranged human D_{H} gene segments in germline configuration with the exception of the unrearranged human D_{H}7-27 gene segment, which is replaced with an unrearranged human D_{H}3-3 gene segment operably linked to a 5'-end 23-mer RSS, and
(c) an unrearranged human J_{H}4 gene segment, an unrearranged human J_{H}5 gene segment, and an unrearranged human J_{H}6 gene segment.

In some embodiments, a nucleotide molecule comprising an immunoglobulin V_{H} region as described herein comprises in operably linkage from 5' to 3'
(a) at least an unrearranged human V_{H}6-1 gene segment, e.g., all or a portion of all the functional unrearranged human V_{H} gene segments spanning between and including the unrearranged human V_{H}3-74 to unrearranged human V_{H}6-1 gene segment, e.g., the full repertoire of functional unrearranged human V_{H} gene segments, optionally including a rodent Adam6 gene between two unrearranged human V_{H} gene segments, e.g., (e.g., wherein the rodent Adam6 gene is located between a human V_{H}1-2 gene segment and a human V_{H}6-1 gene segment)
(b) a human engineered D_{H} region comprising from 5' to 3' an unrearranged human D_{H}1-1 gene segment, a D_{H}2-2 gene segment operably linked at its 3' end to a 23-mer RSS, a human D_{H}2-8 gene segment operably linked at its 3' end to a 23-mer RSS, a human D_{H}2-15 gene segment operably linked at its 3' end to a 23-mer RSS, and the unrearranged human D_{H} gene segments spanning between and including the unrearranged human D_{H}3-16 gene segment and the unrearranged human D_{H}7-27 gene segment, optionally wherein the engineered D_{H} comprises the full repertoire of unrearranged human D_{H} gene segments in germline configuration with the exception that the unrearranged human D_{H}2-2, D_{H}2-8, and D_{H}2-15 gene segments are respectively replaced with a D_{H}2-2 gene segment operably linked at its 3' end to a 23-mer RSS, a human D_{H}2-8 gene segment operably linked at its 3' end to a 23-mer RSS, and a human D_{H}2-15 gene segment operably linked at its 3' end to a 23-mer RSS, and
(c) the full repertoire of unrearranged human J_{H} gene segments, e.g., an unrearranged human J_{H}1 gene segment, an unrearranged J_{H}2 human gene segment, an unrearranged J_{H}3 human gene segment, an unrearranged J_{H}4 human gene segment, an unrearranged J_{H}5 human gene segment, and an unrearranged J_{H}6 human gene segment, optionally wherein the unrearranged human J_{H}1, J_{H}2, J_{H}3, J_{H}4, J_{H}5, and J_{H}6 gene segments are in germline configuration.

In some embodiments, a nucleotide molecule as described herein comprises from 5' to 3' (a) a (human) immunoglobulin heavy chain variable (V_{H}) region comprising at least one (human) V_{H} gene segment, a (human) engineered D_{H} region as described herein, and at least one (human) J_{H} gene segment operably linked to (b) a heavy chain immunoglobulin constant region (C_{H}) or a portion thereof, optionally wherein the C_{H} is a rodent C_{H} that comprises a rodent intronic enhancer region, a rodent IgM gene, a rodent IgD gene, a rodent IgG gene, a rodent IgA gene, a rodent IgE gene, or any combination thereof. In some embodiments, a nucleotide molecule as described herein comprises from 5' to 3' (a) a human immunoglobulin heavy chain V_{H} region comprising at least one human V_{H} gene segment, a human engineered D_{H} region as described herein, and at least one human J_{H} gene segment operably linked to (b) a rodent C_{H} region comprising at least a rodent intronic enhancer region, and optionally a rodent IgM gene. In some embodiments, a nucleotide molecule described herein comprises from 5' to 3' (a) human V_{H} region comprising at least one human V_{H} gene segment, a human engineered D_{H} region as described herein, and at least one human J_{H} gene segment operably linked to (b) a rodent C_{H} region comprising at least a rodent intronic enhancer region and a rodent IgM gene. In some embodiments, a nucleotide molecule described herein comprises from 5' to 3' (a) human V_{H} region comprising at least one human V_{H} gene segment, a human engineered D_{H} region as described herein, and at least one human J_{H} gene segment operably linked to (b) an endogenous rodent C_{H} region, e.g., at an endogenous rodent immunoglobulin heavy chain locus. In some embodiments, the rodent may be a rat. In some embodiments, the rodent may be a mouse.

In some embodiments, a nucleotide molecule as described herein further comprises a rodent Adam6 gene. In some embodiments, the rodent Adam6 gene is located between human V_{H}2-1 and V_{H}6-1 gene segments, e.g., replaces a human Adam6 gene located between a human V_{H}2-1 and V_{H}6-1 gene segment in germline configuration. In some embodiments, the rodent may be a rat. In some embodiments, the rodent may be a mouse.

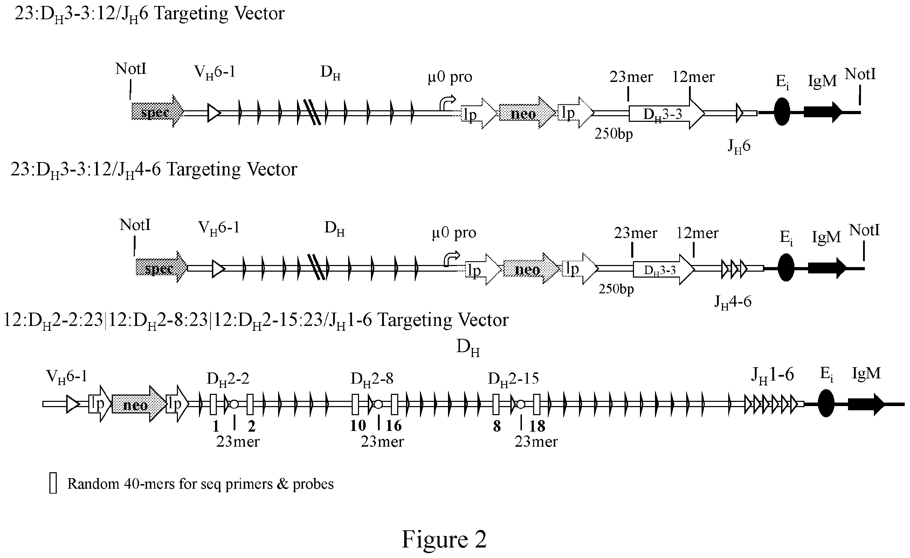

In some embodiments, a nucleotide molecule as described herein comprises one or more drug selection cassettes, e.g., a drug resistance gene flanked by one or more site-specific recombination sites, e.g., a neomycin drug resistance gene flanked by a *lox*P site-specific recombination recognition site, wherein at least one of the one or more drug resistance cassettes is optionally immediately upstream of the at least one D_{H} gene segment operably linked to a 23-mer RSS. In some embodiments the nucleotide molecule comprises a sequence illustrated in Figure 2.

Also described herein are targeting vectors, e.g., for modifying the genome (e.g., the germline genome) of a non-human animal (e.g., a rodent such as a rat or a mouse) to comprise an engineered D_{H} region as described herein. Generally, a targeting vector as described herein comprises any of the nucleotide molecules described herein, and optionally comprises 5'- and 3'-homology arms for homologous recombination within immunoglobulin heavy chain variable region, optionally wherein the immunoglobulin heavy chain variable region is a human or humanized immunoglobulin heavy chain variable region. In some embodiments, a targeting vector as described herein comprises a 5' homology arm comprising an unrearranged human gene segment, e.g., a V_{H}6-1 gene segment and/or a 3' homology arm comprising a rodent (e.g., mouse) C_{H} region or portion thereof, e.g., a rodent (mouse) C_{H} intronic enhancer region and/or rodent (mouse) IgM gene.

In some embodiments, a targeting vector comprises a nucleotide molecule as described herein, and 5'- and 3'- homology arms configured to allow homologous recombination with an immunoglobulin heavy chain sequence, which immunoglobulin heavy chain sequence may optionally be located at an endogenous rodent immunoglobulin heavy chain locus and/or comprise a human or humanized immunoglobulin heavy chain variable region. In some embodiments, a targeting vector as described herein comprising a 5' homology arm that comprises an unrearranged human gene segment (e.g., a V_{H}6-1 gene segment), a human engineered D_{H} region, at least one unrearranged human J_{H} gene segment, and a 3' homology arm comprising a rodent (mouse) C_{H} intronic enhancer region and/or rodent (mouse) may be useful in engineering a D_{H} region in a rodent comprising a humanized immunoglobulin heavy chain locus, e.g., a mouse comprising replacement of mouse immunoglobulin variable sequences with human immunoglobulin variable sequences, e.g., VELOCIMMUNE^{®} mice, which may optionally comprise a functional ADAM6 gene that restores their fertility, a modified endogenous heavy chain constant region gene sequence comprising an intact endogenous IgM gene and another endogenous modified constant region gene (e.g., IgG) for the production of reverse chimeric non-IgM antibodies lacking a functional CH1 domain, a sequence encoding a reverse chimeric humanized common light chain, a sequence encoding a reverse chimeric humanized kappa light chain, a sequence encoding a reverse chimeric humanized lambda light chain, a sequence encoding a hybrid kappa/lambda or kappa/lambda light chain, unrearranged germline human heavy gene segments and/or (un)rearranged germline light chain gene segments modified with a histidine codon for the expression of variable domains that have histidine amino acids and may exhibit pH sensitive antigen binding, and/or terminal deoxynucleotidyl transferase (TdT) for increased antigen receptor diversity. *See, e.g.,* U.S. Patent Nos. 9,035,128; 9,066,502; 9,163,092; 9,150,662; 9,334,333; 9,850,462; 9,844,212; 9,029,628; 9,006,511; 9,394,373; 9,206,261; 9,206,262; 9,206,263; 9,226,484; 9,399,683; 9,540,452; 9,012,717; 9,796,788, 8,697,940; 8,754,287; 9,334,334; 9,801,362; 9,332,742; 9,969,814; U.S. Patent Publications 2011/0195454, 2012/0021409, 2012/0192300, 2013/0185821, 2013/0302836, 2013/0045492, and 2018/0125043; PCT Publication Nos. WO2017210586, and WO2019/113065 each of which is incorporated herein in its entirety by reference.

As such, described herein are methods of engineering a D_{H} region for D_{H}-D_{H} recombination, e.g., in a rodent. In some embodiments, the method comprises modifying a D_{H} region comprising one or a plurality of unrearranged D_{H} gene segments to comprise at least one D_{H} gene segment operably linked to a 23-mer RSS. In some embodiments, a method of modifying an immunoglobulin heavy chain variable region to engineer D_{H}-D_{H} recombination comprises obtaining an immunoglobulin heavy chain variable region comprising a D_{H} region comprising one or more unrearranged D_{H} gene segments each of which unrearranged D_{H} gene segments is flanked on one side by a 12-mer RSS and on the other side by another12-mer RSS, and modifying the D_{H} region to further comprise at least one D_{H} gene segment operably linked to a 23-mer RSS. In some embodiments, the D_{H} region is a human D_{H} region comprising one or a plurality of unrearranged human D_{H} gene segments, e.g., wherein the plurality of unrearranged human D_{H} gene segments optionally comprises all functional unrearranged human D_{H} gene segments spanning between, and including, the D_{H}1-1 and the D_{H}7-27 gene segments, e.g., in germline configuration. In some embodiments, modifying comprises replacing one or more of the one or plurality of unrearranged D_{H} gene segment flanked on one side by a 12-mer RSS and on the other side by another12-mer RSS, e.g., a functional unrearranged human D_{H} gene segment, with the at least one D_{H} gene segment operably linked to a 23-mer RSS. In some embodiments, the most 3' unrearranged D_{H} gene segment flanked on one side by a 12-mer RSS and on the other side by another12-mer RSS of the D_{H} region is replaced with the D_{H} gene segment operably linked to a 23-mer RSS, wherein the D_{H} gene segment operably linked to a 23-mer RSS comprises from 5' to 3' the 23-mer RSS, the D_{H} gene segment, and the 12-mer RSS. In some embodiments, an unrearranged D_{H} gene segment flanked on one side by a 12-mer RSS and on the other side by another12-mer RSS is replaced with a corresponding D_{H} gene segment engineered to be operably linked to a 23-mer RSS. In some embodiments, wherein the D_{H} region comprises an unrearranged human D_{H}7-27 gene segment (e.g., a germline D_{H}7-17 gene segment), the unrearranged human D_{H}7-27 gene segment is replaced by a human D_{H} gene segment (e.g., an unrearranged human D_{H}3-3 gene segment) operably linked to a 5'-end 23-mer RSS. In some embodiments, wherein the D_{H} region comprises an unrearranged D_{H}2-2 gene segment, an unrearranged D_{H}2-8 gene segment, and/or an unrearranged D_{H}2-15 gene segment (e.g., wherein the engineered D_{H} comprises the full repertoire of unrearranged human D_{H} gene segments in germline configuration) the unrearranged D_{H}2-2 gene segment, the unrearranged D_{H}2-8 gene segment, and/or the unrearranged D_{H}2-15 gene segment are respectively replaced with a D_{H}2-2 gene segment operably linked at its 3' end to a 23-mer RSS, a human D_{H}2-8 gene segment operably linked at its 3' end to a 23-mer RSS, and/or a human D_{H}2-15 gene segment operably linked at its 3' end to a 23-mer RSS. In some embodiments, modifying comprises replacing one of two 12-mer RSS flanking an unrearranged D_{H} gene segment (e.g., a human germline D_{H} gene segment) with a 23-mer RSS. In some embodiments, the immunoglobulin heavy chain variable region to be modified comprises, in addition to a D_{H} region, a J_{H} region (optionally comprising a full repertoire of human germline J_{H} gene segments comprising a human germline J_{H}1 gene segment, a human germline J_{H}2 gene segment, a human germline J_{H}3 gene segment, a human germline J_{H}4 gene segment, a human germline J_{H}5 gene segment, and a human germline J_{H}6 gene segment, optionally wherein the human germline J_{H}1, J_{H}2, J_{H}3, J_{H}4, J_{H}5, and J_{H}6 gene segments are in germline configuration) operably linked to the D_{H} region, and replacing an unrearranged D_{H} gene segment flanked on one side by a 12-mer RSS and on the other side by another12-mer RSS comprises deleting at least one unrearranged J_{H} gene segment comprised in the J_{H} region, e.g., results in deletion of unrearranged human J_{H}1, J_{H}2, J_{H}3, J_{H}4, and/or J_{H}5 gene segments contiguous with the D_{H} region. In some embodiments, deleting at least one germline J_{H} gene segment comprised in the J_{H} region comprises deleting the unrearranged human J_{H}1, J_{H}2, and J_{H}3 gene segments, and optionally further deleting the J_{H}4 and J_{H}5 gene segments. In some embodiments, replacing one or more of the all functional D_{H} gene segments, e.g., replacing a D_{H}7-27 gene segment with a D_{H} gene segment operably linked to a 5'-end 23-mer RSS, e.g., a D_{H}3-3 gene segment operably linked to a 5'-end 23-mer RSS, results in deletion of unrearranged human J_{H}1, J_{H}2, and J_{H}3 gene segments contiguous with the D_{H} region. In some embodiments, replacing one or more of the all functional D_{H} gene segments, e.g., replacing a D_{H}7-27 gene segment with a D_{H} gene segment operably linked to a 5'-end 23-mer RSS, e.g., a D_{H}3-3 gene segment operably linked to a 5'-end 23-mer RSS, results in deletion of unrearranged human J_{H}1, J_{H}2, J_{H}3, J_{H}4, and J_{H}5 gene segments contiguous with the D_{H} region. In some embodiments, a D_{H} region is modified to comprise at least one D_{H} gene segment operably linked to a 23-mer RSS, wherein the at least one D_{H} gene segment operably linked to a 23-mer RSS comprises (a) a human D_{H}3-3 gene segment operably linked to a 23-mer RSS, optionally wherein the 23-mer RSS is contiguous to the 5'-end of the D_{H}3-3 gene segment, (b) a human D_{H}2-2 gene segment operably linked to a 23-mer RSS, optionally wherein the 23-mer RSS is contiguous to the 3'-end of the D_{H}2-2 gene segment, (c) a human D_{H}2-8 gene segment operably linked to a 23-mer RSS, optionally wherein the 23-mer RSS is contiguous to the 3'-end of the D_{H}2-8 gene segment, (d) a human D_{H}2-15 gene segment operably linked to a 23-mer RSS, optionally wherein the 23-mer RSS is contiguous to the 3'-end of the D_{H}2-15 gene segment, or (e) any combination of (a)-(d).

Such methods may result in an immunoglobulin heavy chain variable region comprising an engineered D_{H} region, e.g., a nucleotide molecule comprising a (human) immunoglobulin heavy chain variable region as described herein, wherein the (un)rearranged (human) immunoglobulin heavy chain region may be optionally linked to a non-human immunoglobulin heavy chain constant region or portion thereof, e.g., a rodent immunoglobulin heavy chain constant region comprising at least a rodent C_{H} intronic enhancer region and/or a rodent IgM gene, e.g., optionally at an endogenous non-human immunoglobulin heavy chain locus. In some embodiments, upon recombination, such an immunoglobulin heavy chain locus, e.g., endogenous immunoglobulin heavy chain locus, comprises a rearranged immunoglobulin heavy chain variable region coding sequence encodes an immunoglobulin heavy chain variable domain, e.g., an immunoglobulin heavy chain variable domain having a complementarity determining region 3 (CDR3) amino acid length of more than 20 amino acids, which length may be the result of a V_{H}(D_{H}A-D_{H}B)J_{H}, a V_{H}D_{H}J_{H}6, or a V_{H}(D_{H}A-D_{H}B)J_{H}6 recombination. Accordingly, provided herein are rodents, rodent cells, loci and/or nucleotide molecules comprising a rearranged immunoglobulin heavy chain V_{H}(D_{R}A-D_{H}B)J_{H}, a V_{H}(D_{H})J_{H}6, or a V_{H}(D_{H}A-D_{H}B)J_{H}6 sequence encoding an immunoglobulin heavy chain variable domain comprising a CDR3, wherein the length of the CDR3 is at least 20 amino acids in length.

Also described herein are non-human animals, e.g., rodents, comprising an engineered D_{H} region of the invention, e.g., the nucleic acids, targeting vectors and/or immunoglobulin heavy chain loci as described herein, e.g., in its genome, e.g., germline genome. Also described herein are such non-human animal genomes, e.g., rodent genomes. In some embodiments, described herein is a rodent whose germline genome comprises, or a rodent germline genome comprising, an immunoglobulin heavy chain variable region as described, wherein the immunoglobulin heavy chain variable region comprises: (i) at least one unrearranged heavy chain variable (V_{H}) gene segment, (ii) an engineered heavy chain variable region diversity (D_{H}) region, wherein the engineered D_{H} region comprises one or more unrearranged D_{H} gene segments each flanked on one side by a 12-mer RSS and on the other side by another 12-mer RSS and one or more D_{H} gene segments each operably linked to a 23-mer recombination signal sequence (RSS), and (iii) at least one unrearranged heavy chain joining (J_{H}) gene segment, wherein (i)-(iii) are in operable linkage such that, upon recombination, the immunoglobulin heavy chain variable region comprises a rearranged heavy chain variable region sequence encoding an immunoglobulin heavy chain variable domain, optionally wherein the rearranged heavy chain variable region sequence is formed after a V_{H}(D_{H}-D_{H})J_{H} recombination event, optionally wherein at least one of the one or more D_{H} gene segments each operably linked to a 23-mer recombination signal sequence (RSS) joins one of the one or more unrearranged D_{H} gene segments each flanked on one side by a 12-mer RSS and on the other side by another12-mer RSS during the V_{H}(D_{H}-D_{H}) recombination event. In some embodiments, the one or more D_{H} segments operably linked to a 23-mer RSS comprises a D_{H} gene segment operably linked to a 3' 23-mer RSS. In some embodiments, the one or more D_{H} segments operably linked to a 23-mer RSS comprises a D_{H} gene segment operably linked to a 5' 23-mer RSS. In some embodiments, the immunoglobulin heavy chain variable region is a human immunoglobulin heavy chain variable region comprising only human V_{H}, D_{H}, and J_{H} gene segments. In some embodiments, the human immunoglobulin heavy chain variable region is operably linked to an immunoglobulin heavy chain constant region. In some embodiments, the immunoglobulin heavy chain constant region is an endogenous immunoglobulin heavy chain constant region of the non-human animal or non-human animal genome, e.g., at an endogenous immunoglobulin heavy chain locus. In some embodiments, the immunoglobulin heavy chain variable region comprises human V_{H} gene segments spanning between from V_{H}3-74 to V_{H}6-1 in germline configuration. In some embodiments, the immunoglobulin heavy chain variable region comprises a human J_{H}6 gene segment. In some embodiments, the immunoglobulin heavy chain variable region comprises one or more nucleotide molecules encoding one or more rodent Adam6 polypeptides (e.g., a rodent Adam6 gene), which may optionally be inserted between two human V_{H} gene segment (e.g., inserted between a human V_{H}1-2 gene segment and a human V_{H}6-1 gene segment) and/or inserted in the place of a human Adam6 pseudogene. In some embodiments, the non-human animal is a rodent. In some embodiments, the rodent genome is heterozygous for the engineered D_{H} region. In some embodiments, the rodent genome is homozygous for the engineered D_{H} region. In some embodiments, the rodent may be a rat. In some embodiments, the rodent may be a mouse. In some embodiments, the rodent, rodent genome, or rodent cell is a rat, a mouse, a rat genome, a mouse genome, a rat cell, or a mouse cell, e.g., a rodent (rat or mouse) embryonic stem cell.

In some embodiments, a rodent, rodent genome, or rodent cell described herein further comprises rearranged heavy chain VDJ and/or V_{H}(D_{H}A-D_{H}B)J_{H} coding sequences encoding immunoglobulin heavy chain variable domains. In some embodiments, described herein is a non-human animal, e.g., a rodent, e.g., a rat or a mouse, that comprises (1) in its germline genome, e.g., in a germ cell, an immunoglobulin heavy chain locus comprising an engineered D_{H} region comprising a D_{H} gene segment operably linked to a 23-mer RSS, and (2) in its somatic genome, e.g., in a B cell, a rearranged heavy chain variable region V_{H}(D_{H}A-D_{H}B)J_{H} coding sequence, wherein the first or second D_{H} gene segment (D_{H}A or D_{H}B, respectively) is derived from the D_{H} gene segment operably linked to a 23-mer RSS, or a portion thereof (e.g., comprises a sequence identical to that of at least a portion the D_{H} gene segment operably linked to a 23-mer RSS, a somatically hypermutated variant thereof and/or a degenerate variant thereto). In some embodiments, wherein the B cell is a naive B cell and/or the rearranged heavy chain variable region coding sequence is operably linked to an IgM constant region sequence, at least one of D_{H}A and D_{H}B gene segment comprises at least 9 consecutive nucleotides that align with a nucleotide molecule encoded by the D_{H} gene segment operably linked to a 23-mer RSS, and each of the D_{H}A and D_{H}B gene segments comprises at least 5 consecutive nucleotides that align with a nucleotide molecule of a germline D_{H} gene segment. In some embodiments, where the B cell is a plasma or memory B cell and/or the rearranged heavy chain variable region coding sequence is somatically hypermutated and/or operably linked to an non-IgM constant region sequence (e.g., an IgG, IgA, IgE, etc.), each of D_{H}A and D_{H}B respectively shows 40% identity to a first and second germline D_{H} gene segment, with a maximum of 1 nucleotide mutation. In some embodiments, at least 95% of all rearranged heavy chain VDJ and/or V_{H}(D_{H}A-D_{H}B)J_{H} coding sequences in the rodent have a CDR3 length of at least 10 amino acids, optionally wherein at least 70% of all rearranged heavy chain VDJ and/or V_{H}(D_{H}A-D_{H}B)J_{H} coding sequences in the rodent have a CDR3 length of at least 11 amino acids, optionally at least 15% of all rearranged heavy chain VDJ and/or V_{H}(D_{H}A-D_{H}B)J_{H} coding sequences in the rodent have a CDR3 length of at least 14 amino acids. In some embodiments, a population of VDJ and/or V_{H}(D_{H}A-D_{H}B)J_{H} coding sequences in the rodent have a CDR3 length of at least 15 amino acids, optionally at least 16 amino acids, optionally at least 17 amino acids, and optionally at least 18 amino acids. In some embodiments, described herein is a rodent or a rodent cell that expresses, or a nucleic acid or immunoglobulin locus that comprises, a rearranged heavy chain V_{H}(D_{H}A-D_{H}B)J_{H} coding sequence, wherein the second germline D_{H} gene segment is D_{H}3-3, optionally wherein the rearranged heavy chain V_{H}(D_{H}A-D_{H}B)J_{H} coding sequence encodes a CDR3 length of over 20 amino acids. In some embodiments, described herein is a rodent or rodent cell that expresses, or a nucleic acid or immunoglobulin locus that comprises, a rearranged heavy chain V_{H}(D_{H}A-D_{H}B)J_{H} coding sequence, wherein the first germline D_{H} gene segment is D_{H}2-2, D_{H}2-8 or D_{H}2-15, optionally wherein the rearranged heavy chain V_{H}(D_{H}A-D_{H}B)J_{H} coding sequence encodes a CDR3 length of over 20 amino acids. In some embodiments, described herein is a rodent or rodent cell that expresses a rearranged heavy chain V_{H}(D_{H}A-D_{H}B)J_{H}6 coding sequence encoding CDR3 length of over 20 amino acids.

In some embodiments provided is a rodent genome, nucleic acid or immunoglobulin locus comprising a rearranged human immunoglobulin heavy chain V_{H}(D_{H}A-D_{H}B)J_{H} coding sequence operably linked to a rodent immunoglobulin heavy chain constant region sequence. In some embodiments, the D_{H}B gene segment is derived from a human germline D_{H}3-3 gene segment. In some embodiments, the D_{H}A gene segment is derived from a human germline D_{H}2-2, D_{H}2-8 or D_{H}2-15 gene segment. In some embodiments, the rearranged heavy chain is determined to be a V_{H}(D_{R}A-D_{H}B)J_{H} coding sequence since each of D_{H}A and D_{H}B respectively shows 40% identity to a first and second germline D_{H} gene segment, with a maximum of 1 nucleotide mutation. In some embodiments, the rearranged heavy chain V_{H}(D_{H}A-D_{H}B)J_{H} coding sequence encodes a CDR3 length of over 20 amino acids. In some embodiments, the J_{H} gene segment is derived from a human germline J_{H}6 gene segment. In some embodiments, a rodent or rodent cell comprising a V_{H}(D_{R}A-D_{H}B)J_{H} coding sequence is provided. In some embodiments, the rodent is a rat or a mouse or the rodent cell is a rat cell or a mouse cell. In some embodiments, the rodent cell is a rodent B cell. In some embodiments, a hybridoma comprising a rodent B cell expressing a rearranged heavy chain V_{H}(D_{H}A-D_{H}B)J_{H} coding sequence fused with a myeloma cell is provided. In some embodiments, a V_{H}(D_{H}A-D_{H}B)J_{H} sequence is confirmed to be the result of a D_{H}-D_{H} recombination event when the sequences identified as D_{H}A and D_{H}B each respectively shows 40% identity to a first and second germline D_{H} gene segment, with a maximum of 1 nucleotide mutation.

In some embodiments, a non-human animal or cell is provided, whose genome comprises an immunoglobulin heavy chain variable region that includes an engineered D_{H} region, wherein the engineered D_{H} region comprises at least one D_{H} segment that is operably linked to a first and second recombination signal sequences (RSS). In some embodiments, the first RSS is a 23-mer RSS and the second RSS is a 12-mer RSS. In some embodiments, the first RSS is a 12-mer RSS and the second RSS is a 23-mer RSS.

In some embodiments, a non-human animal is provided, whose genome comprises an immunoglobulin heavy chain variable region that includes an engineered D_{H} region, which engineered D_{H} region comprises one or more D_{H} segments that are each operably linked to a 23-mer recombination signal sequence (RSS).

In some embodiments, a non-human cell or tissue is provided, whose genome comprises an immunoglobulin heavy chain variable region that includes an engineered D_{H} region, which engineered D_{H} region comprises one or more D_{H} segments that are each operably linked to a 23-mer recombination signal sequence (RSS). In some embodiments, a cell is from a lymphoid or myeloid lineage. In some embodiments, a cell is a lymphocyte. In some embodiments, a cell is selected from a B cell, dendritic cell, macrophage, monocyte, and a T cell. In some embodiments, a tissue is selected from adipose, bladder, brain, breast, bone marrow, eye, heart, intestine, kidney, liver, lung, lymph node, muscle, pancreas, plasma, serum, skin, spleen, stomach, thymus, testis, ovum, or any combination thereof.

In some embodiments, an immortalized cell made from a non-human cell as described herein is provided, e.g., a hybridoma cell made from fusing a B cell isolated from a non-human animal as described herein with a myeloma cell.

In some embodiments, a non-human cell is a non-human embryonic stem (ES) cell. In some embodiments, a non-human embryonic stem cell is a rodent embryonic stem cell. In some embodiments, a rodent embryonic stem cell is a mouse embryonic stem cell and is from a 129 strain, C57BL strain, or a mixture thereof. In some embodiments, a rodent embryonic stem cell is a mouse embryonic stem cell and is a mixture of 129 and C57BL strains.

In some embodiments, use of a non-human embryonic stem cell as described herein to make a non-human animal is provided. In some embodiments, a non-human embryonic stem cell is a mouse embryonic stem cell and is used to make a mouse comprising an immunoglobulin heavy chain variable region that includes an engineered D_{H} region as described herein. In some embodiments, a non-human embryonic stem cell is a rat embryonic stem cell and is used to make a rat comprising an immunoglobulin heavy chain variable region that includes an engineered D_{H} region as described herein. In some embodiments, a non-limiting exemplary method for making the rat comprising an immunoglobulin heavy chain variable region that includes an engineered D_{H} region can include the methods disclosed in US20140309487, incorporated herein by reference in its entirety.

In some embodiments, a non-human embryo comprising, made from, obtained from, or generated from a non-human embryonic stem cell as described herein is provided. In some embodiments, a non-human embryo is a rodent embryo; in some embodiments, a mouse embryo; in some embodiments, a rat embryo.

In some embodiments, use of a non-human embryo described herein to make a non-human animal is provided. In some embodiments, a non-human embryo is a mouse embryo and is used to make a mouse comprising an immunoglobulin heavy chain variable region that includes an engineered D_{H} region as described herein. In some embodiments, a non-human embryo is a rat embryo and is used to make a rat comprising an immunoglobulin heavy chain variable region that includes an engineered D_{H} region as described herein.

Provided herein are methods of making a rodent whose genome comprises an engineered D_{H} region, the method comprising (a) modifying the genome of a rodent embryonic stem cell to comprise a DNA fragment comprising one or more D_{H} segments that are each operably linked to a 23-mer RSS, e.g., wherein the DNA fragment comprises a nucleotide molecule, targeting vector, and/or engineered D_{H} region described herein, and (b) generating a rodent using the modified rodent embryonic stem cell of (a). In some embodiments, the methods comprise modifying an unrearranged D_{H} region of an immunoglobulin heavy chain variable region to comprise at least one D_{H} segment operably linked to a 23-mer RSS, wherein the unrearranged D_{H} region further comprises one or more unrearranged D_{H} gene segments each of which is flanked on one side by a 12-mer RSS and on the other side by another 12-mer RSS, thereby making said rodent. In some embodiments, modifying comprises replacing the one or more unrearranged human D_{H} gene segments, and optionally replacing or deleting one or more J_{H} gene segments, with the at least one D_{H} segments operably linked to a 23-mer RSS. In some embodiments, the immunoglobulin heavy chain variable region is a human immunoglobulin heavy chain variable region, e.g., wherein the human immunoglobulin heavy chain variable region comprises an unrearranged human immunoglobulin heavy chain V_{H} gene cluster comprising at least one V_{H} gene segment, an unrearranged human immunoglobulin heavy chain D_{H} region comprising one or more unrearranged human D_{H} gene segments, and an unrearranged human immunoglobulin heavy chain J_{H} gene cluster comprising at one unrearranged human J_{H} gene segment, wherein the unrearranged human immunoglobulin heavy chain D_{H} region is modified to comprise at least one D_{H} segment operably linked to a 23-mer RSS. In some embodiments, the modifying step results in the immunoglobulin heavy chain variable region comprising (i) the full repertoire of functional human V_{H} gene segments, e.g., all functional V_{H} gene segments spanning between and including V_{H}3-74 to V_{H}6-1, (ii) the full repertoire of unrearranged human D_{H} gene segments except for D_{H}7-27 which is replaced with the at least one D_{H} gene segment operably linked to a 23-mer RSS, and (iii) at least an unrearranged human J_{H}6 gene segment, and optionally at least an unrearranged J_{H}4 gene segment, an unrearranged J_{H}5 gene segment, and an unrearranged J_{H}6 gene segment. In some embodiments, the at least one D_{H} gene segment operably linked to a 23-mer RSS comprises a D_{H}3-3 gene segment operably linked to a 5'- 23-mer RSS. In some embodiments, the modifying step results in the immunoglobulin heavy chain variable region comprising (i) a full repertoire of functional human V_{H} gene segments, e.g., all functional V_{H} gene segments spanning between and including V_{H}3-74 to V_{H}6-1, (ii) the full repertoire of unrearranged human D_{H} gene segments except that the unrearranged human D_{H}2-2, D_{H}2-8, and D_{H}2-15 gene segments are respectively replaced with a D_{H}2-2 gene segment operably linked at its 3' end to a 23-mer RSS, a human D_{H}2-8 gene segment operably linked at its 3' end to a 23-mer RSS, and a human D_{H}2-15 gene segment operably linked at its 3' end to a 23-mer RSS, and (iii) the full repertoire of unrearranged human J_{H} gene segments, e.g., an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment. In some embodiments, the immunoglobulin heavy chain variable region (a) further comprises one or more rodent Adam6 genes, optionally wherein the one or more rodent Adam6 genes are located between two unrearranged V_{H} gene segments, e.g., between an unrearranged human V_{H}1-2 gene segment and an unrearranged human V_{H}6-1 gene segment, and/or (b) is operably linked to an immunoglobulin heavy chain constant region, optionally wherein the immunoglobulin heavy chain constant region is an endogenous rodent immunoglobulin heavy chain constant region, e.g., an endogenous rodent immunoglobulin heavy chain constant region at an endogenous immunoglobulin heavy chain locus. In some embodiments, the rodent is a rat or a mouse.

In some embodiments, a kit is provided, comprising anon-human animal as described herein, a non-human cell or tissue as described herein, an immortalized cell as described herein, a non-human embryonic stem cell as described herein, or a non-human embryo as described herein.

In some embodiments, a kit as described herein is provided, for use in the manufacture and/or development of a drug (e.g., an antibody or antigen-binding fragment thereof) for therapy or diagnosis. In some embodiments, a kit as described herein is provided, for use in the manufacture and/or development of a drug (e.g., an antibody or antigen-binding fragment thereof) for the treatment, prevention or amelioration of a disease, disorder or condition.

In some embodiments, a transgene, nucleic acid construct, DNA construct, or targeting vector as described herein is provided. In some embodiments, a transgene, nucleic acid construct, DNA construct, or targeting vector comprises an engineered D_{H} region as described herein. In some embodiments, a transgene, nucleic acid construct, DNA construct, or targeting vector comprises a DNA fragment that includes one or more D_{H} segments operably linked to a 23-mer RSS. In some embodiments, a transgene, nucleic acid construct, DNA construct, or targeting vector further comprises one or more selection markers. In some embodiments, a transgene, nucleic acid construct, DNA construct, or targeting vector further comprises one or more site-specific recombination sites (e.g., *lox*P, Frt, or combinations thereof). In some embodiments, a transgene, nucleic acid construct, DNA construct, or targeting vector is depicted in Figure 2.

In some embodiments, use of a transgene, nucleic acid construct, DNA construct, or targeting vector as described herein to make anon-human animal, non-human cell, non-human embryonic stem cell, and/or non-human embryo is provided.

In some embodiments, one or more D_{H} segments are each operably linked to a 3' 23-mer RSS. In some embodiments, one or more D_{H} segments are each operably linked to a 5' 23-mer RSS.

In some embodiments, an engineered D_{H} region comprises one D_{H} segment operably linked to a 5' 23-mer RSS. In some embodiments, an engineered D_{H} region comprises one D_{H} segment operably linked to a 3' RSS. In some embodiments of one D_{H} segment operably linked to a 5' 23-mer RSS, the one D_{H} segment is a synthetic D_{H} segment; in some embodiments, a synthetic human D_{H} segment; in some embodiments, a synthetic human D_{H} segment having a sequence that is identical or substantially identical to a human D_{H}3-3 segment. In some embodiments of one D_{H} segment operably linked to a 3' 23-mer RSS, the one D_{H} segment is a synthetic D_{H} segment; in some embodiments, a synthetic human D_{H} segment; in some embodiments, a synthetic human D_{H} segment having a sequence that is identical or substantially identical to a human D_{H}3-3 segment.

In some embodiments, an engineered D_{H} region comprises three D_{H} segments each operably linked to a 5' 23-mer RSS. In some embodiments of three D_{H} segments each operably linked to a 5' 23-mer RSS, the three D_{H} segments are synthetic D_{H} segments. In some embodiments of three D_{H} segments each operably linked to a 5' 23-mer RSS, the three D_{H} segments are human D_{H}2 family segments. In some embodiments of three D_{H} segments each operably linked to a 5' 23-mer RSS, the three D_{H} segments are selected from human D_{H}2-2, human D_{H}2-8, human D_{H}2-15, human D_{H}2-21 and combinations thereof. In some embodiments of three D_{H} segments each operably linked to a 5' 23-mer RSS, the three D_{H} segments are human D_{H}2-2, human D_{H}2-8 and human D_{H}2-15.

In some embodiments, an engineered D_{H} region comprises three D_{H} segments each operably linked to a 3' 23-mer RSS. In some embodiments of three D_{H} segments each operably linked to a 3' 23-mer RSS, the three D_{H} segments are synthetic D_{H} segments. In some embodiments of three D_{H} segments each operably linked to a 3' 23-mer RSS, the three D_{H} segments are human D_{H}2 family segments. In some embodiments of three D_{H} segments each operably linked to a 3' 23-mer RSS, the three D_{H} segments are selected from human D_{H}2-2, human D_{H}2-8, human D_{H}2-15, human D_{H}2-21 and combinations thereof. In some embodiments of three D_{H} segments each operably linked to a 3' 23-mer RSS, the three D_{H} segments are human D_{H}2-2, human D_{H}2-8 and human D_{H}2-15.

In some embodiments, an engineered D_{H} region as described herein includes a plurality of human D_{H} segments, wherein at least one of the plurality of human D_{H} gene segments is operably linked to a 5' or a 3' RSS; in some embodiments, a 5' 23-mer RSS. In some embodiments, an engineered D_{H} region as described herein includes a plurality of human D_{H} segment, wherein at least three of the plurality of human D_{H} gene segments are each operably linked to a 5' or a 3' R_SS; in some embodiments, a 3' 23-mer RSS.

In some embodiments, the genome of a provided non-human animal, non-human cell or non-human tissue lacks one or more wild-type D_{H} segments. In some embodiments, the genome of a provided non-human animal, non-human cell or non-human tissue lacks all or substantially all wild-type D_{H} segments. In some embodiments, the genome of a provided non-human animal, non-human cell or non-human tissue contains only human D_{H} segments.

In some embodiments, an immunoglobulin heavy chain variable region is a human immunoglobulin heavy chain variable region. In some embodiments, a human immunoglobulin heavy chain variable region is operably linked to an immunoglobulin heavy chain constant region. In some embodiments, an immunoglobulin heavy chain constant region is an endogenous (e.g., non-human) immunoglobulin heavy chain constant region.

In some embodiments, a human immunoglobulin heavy chain variable region includes the human V_{H} gene segments from V_{H}3-74 to V_{H}6-1. In some embodiments, a human immunoglobulin heavy chain variable region includes at least human J_{H} gene segment J_{H}6. In some embodiments, a human immunoglobulin heavy chain variable region includes at least human J_{H} gene segments J_{H}4, J_{H}5 and J_{H}6. In some embodiments, a human immunoglobulin heavy chain variable region includes human J_{H} gene segments J_{H}1, J_{H}2, J_{H}3, J_{H}4, J_{H}5 and J_{H}6.

In some embodiments of a non-human animal, non-human cell or non-human tissue, the genome lacks an endogenous Adam6 gene. In some embodiments of a non-human animal, non-human cell or non-human tissue, the genome further comprises insertion of one or more nucleotide sequences encoding one or more rodent Adam6 polypeptides; in some embodiments, the one or more nucleotide sequences are inserted between a first and a second human V_{H} gene segment; in some embodiments, the one or more nucleotide sequences are inserted in the place of a human Adam6 pseudogene; in some embodiments, the one or more nucleotide sequences are inserted between a human V_{H} gene segment and a human D_{H} gene segment. In some embodiments, a first human V_{H} gene segment is human V_{H}1-2 and a second human V_{H} gene segment is human V_{H}6-1.

In some embodiments, a provided non-human animal, non-human cell or non-human tissue is homozygous, heterozygous or hemizygous for an engineered D_{H} region as described herein. In some embodiments, a provided non-human animal, non-human cell or non-human tissue is transgenic for an engineered D_{H} region as described herein.

In some embodiments, a method of making a non-human animal whose genome comprises an immunoglobulin heavy chain variable region that includes an engineered D_{H} region is provided, the method comprising(a) inserting a DNA fragment into a non-human embryonic stem cell, which DNA fragment comprises one or more D_{H} segments that are each operably linked to a 23-mer RSS; (b) obtaining the non-human embryonic stem cell generated in (a); and (c) creating a non-human animal using the non-human embryonic stem cell of (b).

In some embodiments, a DNA fragment comprises one or more D_{H} segments are each operably linked to a 3' 23-mer RSS. In some embodiments, a DNA fragment comprises one D_{H} segment operably linked to a 3' 23-mer RSS. In some embodiments, a DNA fragment comprises one synthetic human D_{H} segment operably linked to a 3' 23-mer RSS. In some embodiments, a DNA fragment comprises one synthetic human D_{H}3-3 segment operably linked to a 3' 23-mer RSS. In some embodiments, a DNA fragment comprises three D_{H} segments each operably linked to a 3' 23-mer RSS. In some embodiments, a DNA fragment comprises three human D_{H} segments each operably linked to a 3' 23-mer RSS. In some embodiments, a DNA fragment comprises three human D_{H} segments each operably linked to a 3' 23-mer RSS, which human D_{H} segments are human D_{H}2-2, human D_{H}2-8 and human D_{H}2-15.

In some embodiments, a DNA fragment comprises one or more D_{H} segments, each of which is operably linked to a 5' 23-mer RSS. In some embodiments, a DNA fragment comprises one D_{H} segment operably linked to a 5' 23-mer RSS. In some embodiments, a DNA fragment comprises one synthetic human D_{H} segment operably linked to a 5' 23-mer. In some embodiments, a DNA fragment comprises one synthetic human D_{H}3-3 segment operably linked to a 5' 23-mer RSS. In some embodiments, a DNA fragment comprises one synthetic human D_{H}3-3 segment operably linked to a 5' 23-mer RSS, which synthetic human D_{H}3-3 segment is positioned in a human D_{H} region in the place of a human D_{H}7-27 segment.

In some embodiments, a DNA fragment comprises one or more selection markers. In some embodiments, a DNA fragment comprises one or more site-specific recombination sites.

In some embodiments, a method of making a non-human animal whose genome comprises an immunoglobulin heavy chain variable region that includes an engineered D_{H} region is provided, the method comprising a step of modifying the genome of a non-human animal or non-human animal cell so that it comprises an immunoglobulin heavy chain variable region that includes an engineered D_{H} region, which engineered D_{H} region comprises one or more D_{H} segments that are each operably linked to a 23-mer RSS, thereby making said non-human animal.

In some embodiments of making a non-human animal, the genome of the non-human animal or non-human animal cell is modified to include one or more D_{H} segments that are each operably linked to a 5' 23-mer RSS. In some embodiments of making a non-human animal, the genome of the non-human animal or non-human animal cell is modified to include one or more D_{H} segments that are each operably linked to a 3' 23-mer RSS.

In some embodiments, a method of producing an antibody in a non-human animal is provided. In some embodiments, a method of producing an antibody or obtaining a nucleic acid encoding same comprises immunizing a non-human animal (e.g., a rodent (e.g., a rat or a mouse) with an antigen, which rodent has a germline genome comprising an engineered D_{H} region that comprises one or more D_{H} segments that are each operably linked to a 23-mer RSS, and allowing the rodent to produce an immune response to the antigen including an antibody, or nucleic acid encoding same, that binds the antigen. In some embodiments, the method further comprises recovering the antibody, or nucleic acid encoding same, from the rodent or a rodent cell, e.g., is a B cell or a hybridoma. In some embodiments, the one or more D_{H} segments are each operably linked to a 5' 23-mer RSS. In some embodiments, one or more D_{H} segments are each operably linked to a 3' 23-mer RSS.

In some embodiments, the method comprising the steps of (a) immunizing a non-human animal with an antigen, which non-human animal has a genome comprising an immunoglobulin heavy chain variable region that includes an engineered D_{H} region, which engineered D_{H} region comprises one or more D_{H} segments that are each operably linked to a 23-mer RSS; (b) maintaining the rodent under conditions sufficient that the non-human animal produces an immune response to the antigen; and (c) recovering an antibody from the non-human animal, or a non-human animal cell, that binds the antigen. In some embodiments, a non-human animal cell is a B cell. In some embodiments, a non-human animal cell is a hybridoma.

In some embodiments, a non-human animal is provided whose genome comprises a human immunoglobulin heavy chain variable region that comprises one or more human V_{H} gene segments, an engineered D_{H} region that includes at least one human D_{H} segment operably linked to a 23-mer RSS, and at least one human J_{H} gene segment, wherein the human immunoglobulin heavy chain variable region is operably linked to one or more endogenous immunoglobulin constant region genes so that the rodent is characterized in that when it is immunized with an antigen, it generates antibodies comprising human heavy chain variable domains that include CDR3 regions generated by human D_{H}-D_{H} recombination and/or enhanced recombination to a J_{H}6 gene segment, and wherein the antibodies show specific binding to the antigen. In some embodiments, the at least one human D_{H} segment operably linked to a 23-mer RSS is positioned in the place of a human D_{H}7-27 segment. In some embodiments, a human immunoglobulin heavy chain variable region comprises less than all six human J_{H} gene segments. In some embodiments, a human immunoglobulin heavy chain variable region comprises only one of the six human J_{H} gene segment. In some embodiments, a human immunoglobulin heavy chain variable region comprises the human J_{H}6 gene segment and lacks a functional J_{H}1 gene segment, lacks a functional J_{H}2 gene segment, lacks a functional J_{H}3 gene segment, lacks a functional J_{H}4 gene segment, and lacks a functional J_{H}5 gene segment. In some embodiments, a human immunoglobulin heavy chain variable region comprises only three of the six human J_{H} gene segments. In some embodiments, a human immunoglobulin heavy chain variable region comprises only human J_{H}4 gene segment, the human J_{H}5 gene segment, and the human J_{H}6 gene segment, and lacks functional J_{H}1 gene segment, lacks a functional J_{H}2 gene segment, and lacks a functional J_{H}3 gene segment. In some embodiments, wherein a non-human animal whose genome comprises a human immunoglobulin heavy chain variable region comprising less than all six human J_{H} gene segments, e.g., a human immunoglobulin heavy chain variable region that comprises only one of the six human J_{H} gene segments (e.g., a human immunoglobulin heavy chain variable region that comprises the human J_{H}6 gene segment and lacks a functional J_{H}1 gene segment, lacks a functional J_{H}2 gene segment, lacks a functional J_{H}3 gene segment, lacks a functional J_{H}4 gene segment, and lacks a functional J_{H}5 gene segment), exhibits enhanced recombination to a J_{H}6 gene segment compared to a control non-human animal whose genome comprises a human immunoglobulin heavy chain variable region comprising all six human J_{H} gene segments, e.g., the non-human animal comprises a greater percentage of rearranged immunoglobulin heavy chain sequences comprising the J_{H}6 gene segment sequence or portion thereof and/or encoding a CDR3 length of at least 20 amino acids thereof than the control non-human animal. In some embodiments, wherein a non-human animal whose genome comprises a human immunoglobulin heavy chain variable region comprising less than all six human J_{H} gene segments, e.g., a human immunoglobulin heavy chain variable region that comprises only three of the six human J_{H} gene segment (e.g., a human immunoglobulin heavy chain variable region that comprises a human immunoglobulin heavy chain variable region comprises human J_{H}4 gene segment, the human J_{H}5 gene segment, and the human J_{H}6 gene segment, and lacks functional J_{H}1 gene segment, lacks a functional J_{H}2 gene segment, and lacks a functional J_{H}3 gene segment), exhibits enhanced recombination to a J_{H}6 gene segment compared to a control non-human animal whose genome comprises a human immunoglobulin heavy chain variable region comprising all six human J_{H} gene segments, e.g., the non-human animal comprises a greater percentage of rearranged immunoglobulin heavy chain sequences comprising the J_{H}6 gene segment sequence or portion thereof and/or encoding a CDR3 length of at least 20 amino acids compared than the control non-human animal

In some embodiments, a non-human animal is provided whose genome comprises a human immunoglobulin heavy chain variable region that comprises one or more human V_{H} gene segments, an engineered D_{H} region that includes at least one human D_{H} segment flanked 3' by a 23-mer RSS, and at least two human J_{H} gene segments, wherein the human immunoglobulin heavy chain variable region is operably linked to one or more endogenous immunoglobulin constant region genes so that the rodent is characterized in that when it is immunized with an antigen, it generates antibodies comprising human heavy chain variable domains that include CDR3 regions generated by human D_{H}-D_{H} recombination, and wherein the antibodies show specific binding to the antigen. In some embodiments, an engineered D_{H} region includes at least three human D_{H} segments that are each flanked 3' by a 23-mer RSS. In some embodiments, an engineered D_{H} region includes three human D_{H} segments that are each flanked 3' by a 23-mer RSS, which three human D_{H} segments are human D_{H}2-2, human D_{H}2-8 and human D_{H}2-15. In some embodiments, a human immunoglobulin heavy chain variable region includes the human V_{H} gene segments from V_{H}3-74 to V_{H}6-1.

In some embodiments, the antigen is a pathogen, e.g., a bacterial, fungal, or viral pathogen. In some embodiments, immunization of a non-human animal herein comprises infecting the non-human animal with a pathogen, e.g., a bacterial, fungal, or viral pathogen. In some embodiments, immunization of a non-human animal herein comprises administering to the non-human animal genomic or proteinaceous material isolated from the pathogen, e.g., bacterial, fungal, or viral pathogen. In some embodiments, the antigen is a receptor (e.g., a complement receptor, a chemokine receptor, etc.), or portion thereof. In some embodiments, the antigen is a nucleic acid encoding the receptor, or portion thereof. In some embodiments, the antigen is a cell expressing the receptor, or portion thereof. In some embodiments, the antigen is an ion channel, or portion thereof. In some embodiments, the antigen is a nucleic acid encoding the ion channel or portion thereof. In some embodiments, the antigen is a cell expressing the ion channel, or portion thereof.

In some embodiments, a provided non-human animal, non-human cell or non-human tissue has a genome that further comprises an insertion of one or more human V_{L} gene segments and one or more human J_{L} gene segments into an endogenous light chain locus. In some embodiments, human V_{L} and J_{L} segments are Vκ and Jκ gene segments and are inserted into an endogenous κ light chain locus. In some embodiments, human Vκ and Jκ gene segments are operably linked to a rodent Cκ gene (e.g. a mouse or a rat Cκ gene). In some embodiments, human V_{L} and J_{L} segments are Vλ and Jλ gene segments and are inserted into an endogenous λ light chain locus. In some embodiments, human Vλ and Jλ gene segments are operably linked to a rodent Cλ gene (e.g., a mouse or a rat Cλ gene).

In some embodiments, use of a non-human animal, non-human cell or non-human tissue as described herein in the manufacture and/or development of a drug or vaccine for use in medicine, such as use as a medicament, is provided. In some embodiments, use of a non-human animal, non-human cell or non-human tissue as described herein in the manufacture and/or development of an antibody for administration to humans is provided. In some embodiments, use of a non-human animal, non-human cell or non-human tissue as described herein in the manufacture of a medicament for the treatment, prevention or amelioration of a disease, disorder or condition is provided.

In some embodiments, a non-human animal, non-human cell or non-human tissue as described herein is provided for use in the manufacture and/or development of a drug for therapy or diagnosis. In some embodiments, a non-human animal, non-human cell or non-human tissue as described herein is provided for use in the manufacture of a medicament for the treatment, prevention or amelioration of a disease, disorder or condition.

In some embodiments, a non-human animal provided herein is a rodent; in some embodiments, a mouse; in some embodiments, a rat. In many embodiments, a non-human animal cell provided herein is a rodent cell; in some embodiments, a mouse cell; in some embodiments, a rat cell. In many embodiments, a non-human animal tissue provided herein is a rodent tissue; in some embodiments, a mouse tissue; in some embodiments, a rat tissue.

In some embodiments, a 23-mer RSS comprises a nucleotide sequence comprising a sequence set forth as SEQ ID NO:151.

### BRIEF DESCRIPTION OF THE DRAWING

The Drawing included herein, which is composed of the following Figures, is for illustration purposes only and not for limitation.

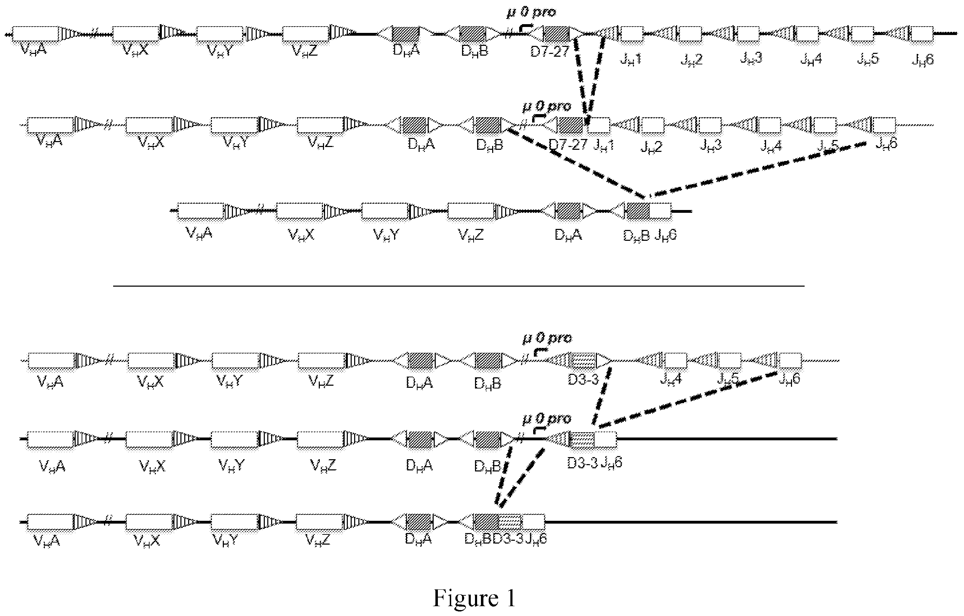

***Figure 1*** shows a general illustration, not to scale, of embodiments of the present invention showing ordered assembly of gene segments in a DJ recombination event for immunoglobulin heavy chain variable region gene segments with an unmodified D_{H} region (top panel) and engineered D_{H} region (bottom panel). 12-mer recombination signal sequences (RSS) are depicted as unfilled triangles. 23-mer RSS are depicted as triangles with vertical stripes. Illustrative unmodified V_{H} gene segments (unfilled boxes) and D_{H} gene segments (filled boxes) are respectively depicted as unfilled and filled boxes and are provided generic nomenclature using letters of the alphabet. D gene segments (e.g., D_{H}3-3) operably linked to a 23-mer RSS and unmodified J_{H} gene segments are respectively depicted as boxes filled with horizontal stripes and unfilled boxes and provided their proper nomenclature. Also shown is the µ0 promoter (µ 0 pro). Not depicted is recombination of a V_{H} gene segment to the recombined DJ gene segment.
***Figure 2*** shows illustrations, not to scale, of exemplary embodiments of targeting vectors made according to Example 1. Hash lines represent D_{H} gene segments that are included in the targeting vector but are not specifically depicted.

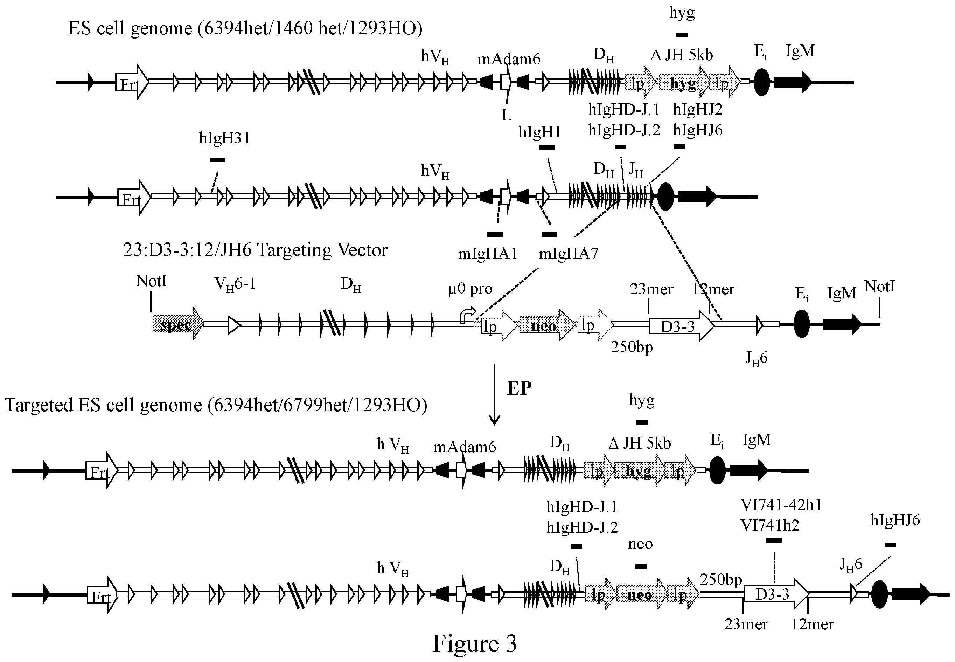

***Figure 3*** shows an illustration, not to scale, of a non-limiting exemplary embodiment of inserting the 23:D_{H}3-3:12/J_{H}6 targeting vector into a humanized immunoglobulin heavy chain variable region locus in the genome of mouse ES cells via electroporation (EP). Hash lines represent V_{H} gene segments or D_{H} gene segments that are included in the heavy chain variable region locus but are not specifically depicted.

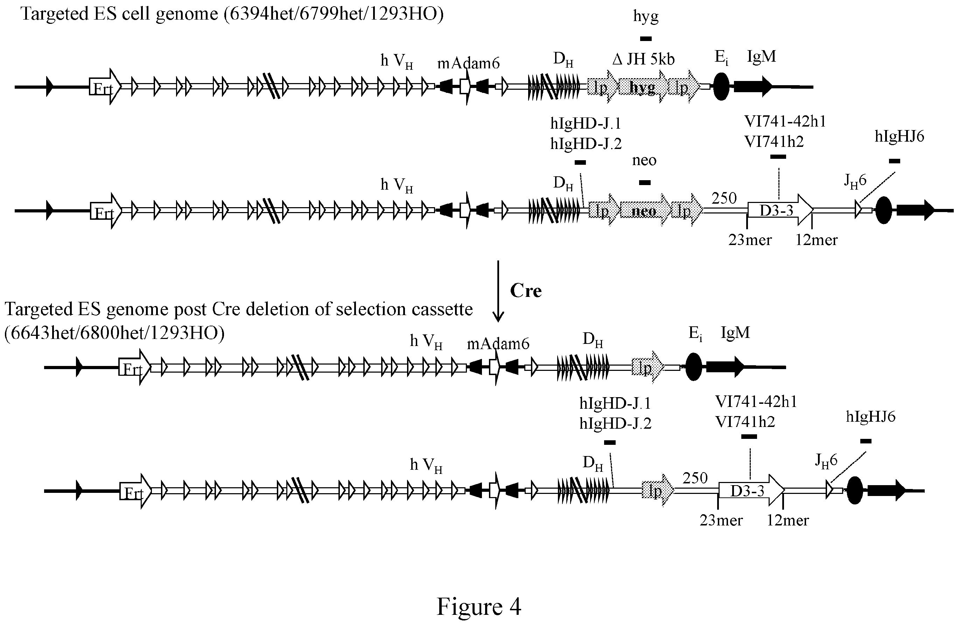

***Figure* 4** shows an illustration, not to scale, of a non-limiting exemplary embodiment of Cre-mediated deletion of selection cassettes in humanized immunoglobulin heavy chain loci after electroporation and integration of the 23:D_{H}3-3:12/J_{H}6 targeting vector as described in Examples 1 and 2. Hash lines represent V_{H} gene segments or D_{H} gene segments that are included in the heavy chain variable region locus but are not specifically depicted.

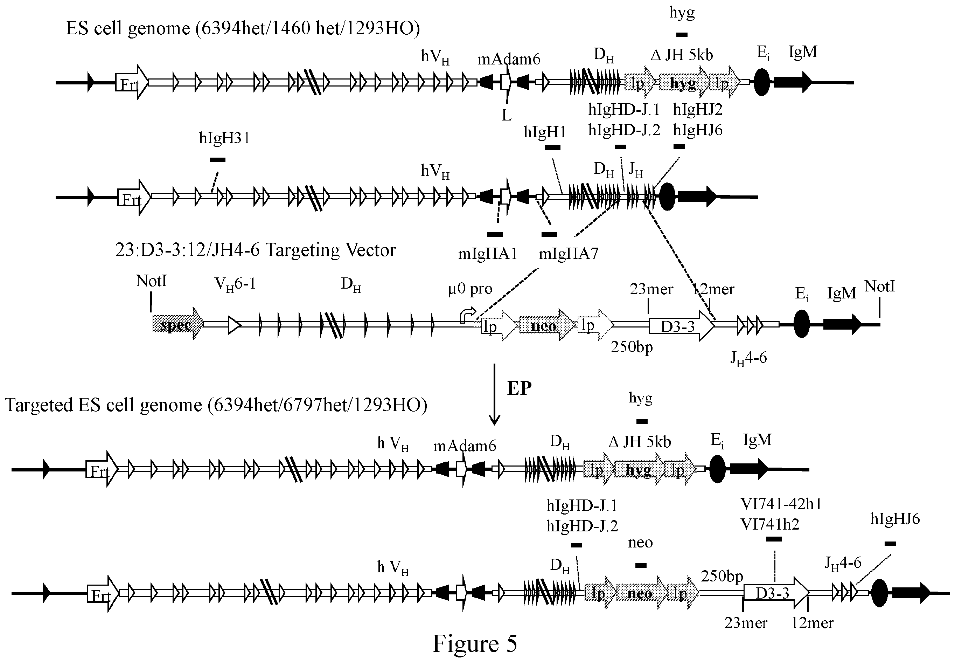

***Figure 5*** shows an illustration, not to scale, of a non-limiting exemplary embodiment of inserting the 23:D_{H}3-3:12/J_{H}4-6 targeting vector into a humanized immunoglobulin heavy chain variable region locus in the genome of mouse ES cells via electroporation (EP). Hash lines represent V_{H} gene segments or D_{H} gene segments that are included in the heavy chain variable region locus but are not specifically depicted.

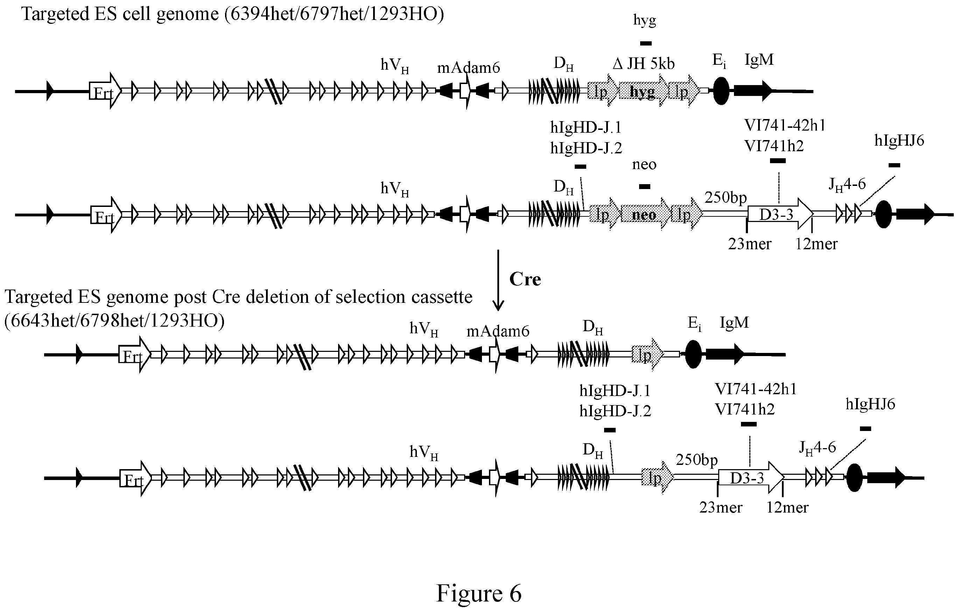

***Figure* 6** shows an illustration, not to scale, of a non-limiting exemplary embodiment of Cre-mediated deletion of selection cassettes in humanized immunoglobulin heavy chain loci after electroporation and integration of the 23:D_{H}3-3:12/J_{H}4-6 targeting vector as described in Examples 1 and 2. Hash lines represent V_{H} gene segments or D_{H} gene segments that are included in the heavy chain variable region locus but are not specifically depicted.

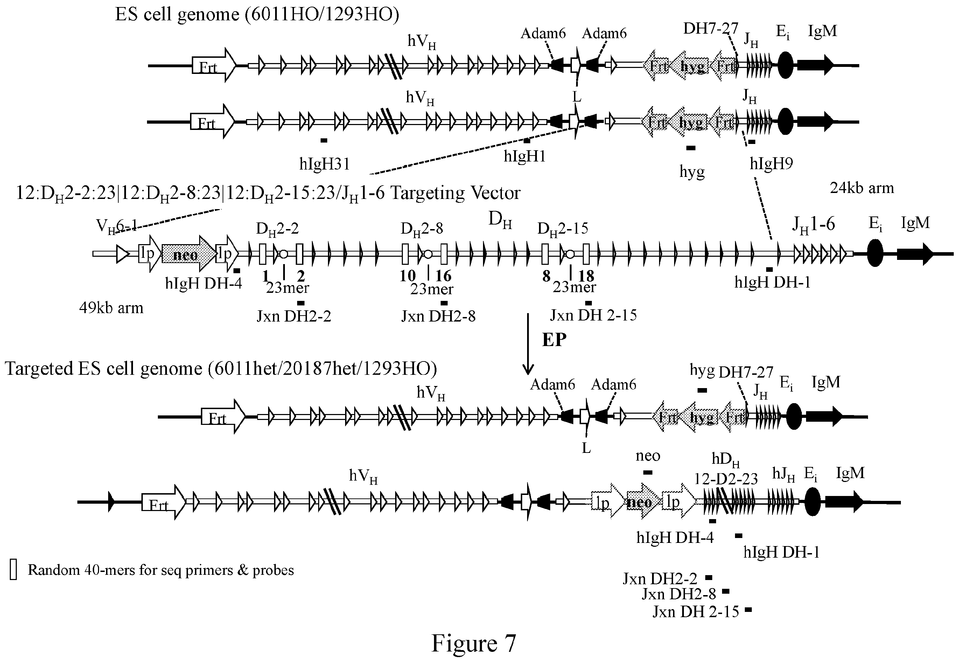

***Figure* 7** shows an illustration, not to scale, of a non-limiting exemplary embodiment of inserting the 12:D_{H}2-2:23|12:D_{H}2-8:23|12:D_{H}2-15:23/J_{H}1-6 targeting vector into a humanized immunoglobulin heavy chain variable region locus in the genome of mouse ES cells via electroporation (EP). Hash lines represent V_{H} gene segments or D_{H} gene segments that are included in the heavy chain variable region locus but are not specifically depicted. Filled triangles represent pseudogenes.

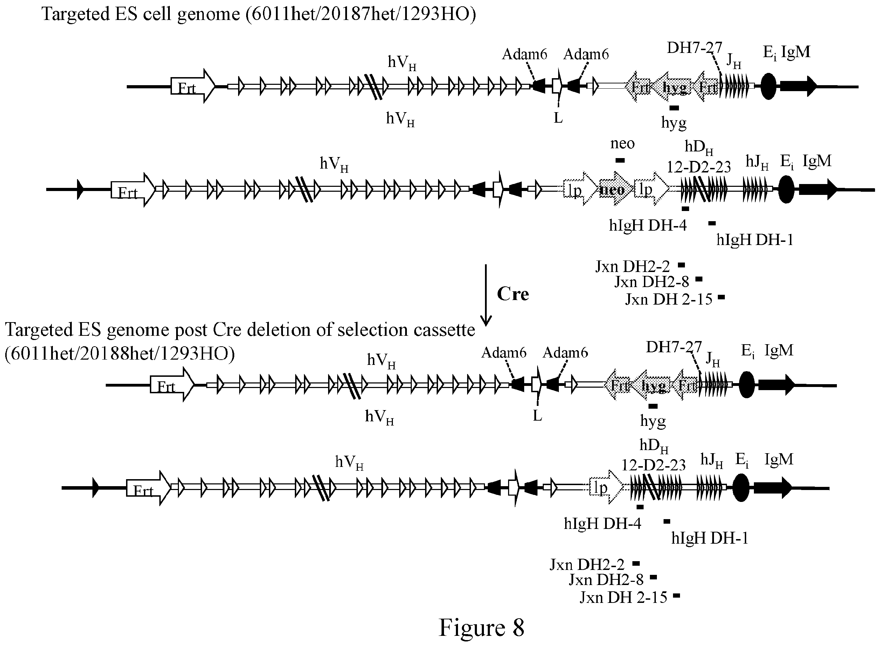

***Figure 8*** shows an illustration, not to scale, of an exemplary non-limiting embodiment of Cre-mediated deletion of selection cassettes in humanized immunoglobulin heavy chain loci after electroporation and integration of the 12:D_{H}2-2:23|12:D_{H}2-8:23|12:D_{H}2-15:23 targeting vector as described in Examples 1 and 2. Hash lines represent V_{H} gene segments or D_{H} gene segments that are included in the heavy chain variable region locus but are not specifically depicted

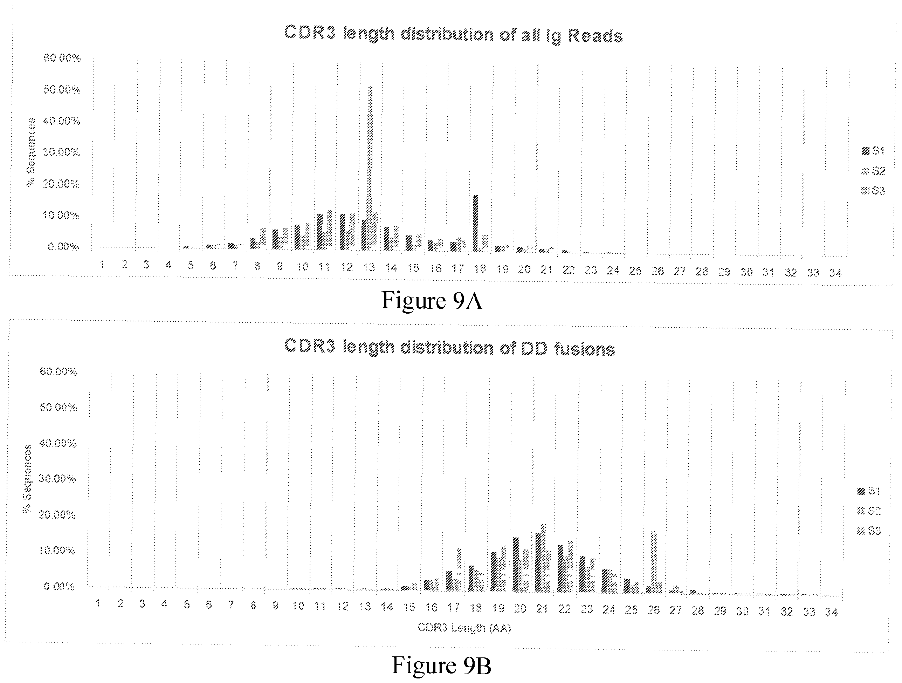

***Figure 9A*** shows results in connection with an embodiment of the invention, graphing the percentages (y-axes) of all functional immunoglobulin (Ig) reads resulting from a D_{H}-D_{H} recombination event (bottom panel) that have a CDR3 of a particular amino acid length (x-axes) isolated from animals modified with the 12:D_{H}2-2:23|12:D_{H}2-8:23|12:D_{H}2-15:23 targeting vector. "S1," "S2," and "S3" each represent a different experimental mouse.
***Figure 9B*** shows results in connection with an embodiment of the invention, graphing the percentages (y-axes) of all Ig reads resulting from a D_{H}-D_{H} recombination event (bottom panel) that have a CDR3 of a particular amino acid length (x-axes) isolated from animals modified with the 12:D_{H}2-2:23|12:D_{H}2-8:23|12:D_{H}2-15:23 targeting vector. "S1," "S2," and "S3" each represent a different experimental mouse.

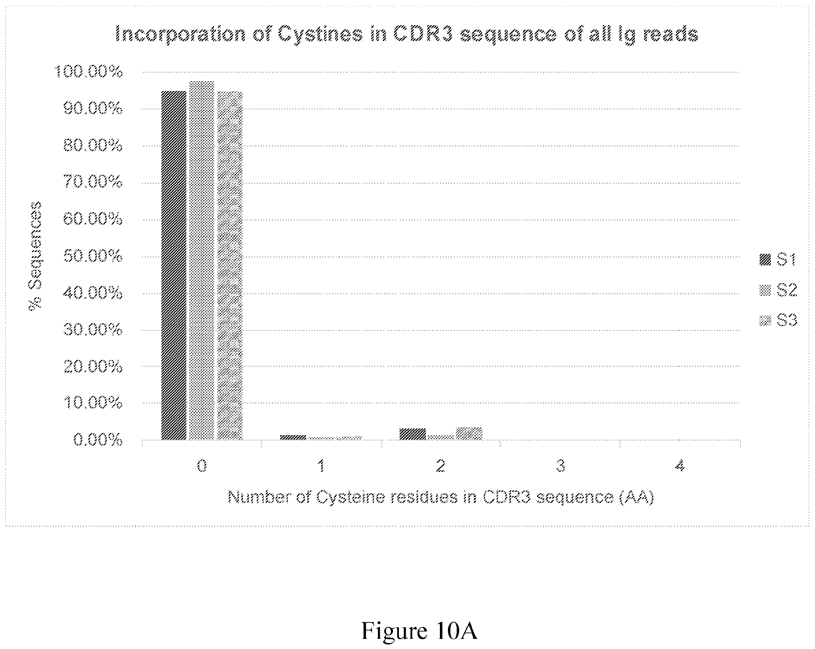

***Figure 10A*** shows results in connection with an embodiment of the invention, graphing the percentages (y-axes) of all functional immunoglobulin (Ig) reads resulting from a D_{H}-D_{H} recombination event that have a CDR3 comprising a certain number of cysteine residues (x-axes) isolated from animals modified with the 12:D_{H}2-2:23|12:D_{H}2-8:23|12:D_{H}2-15:23 targeting vector. "S1," "S2," and "S3" each represent a different experimental mouse.

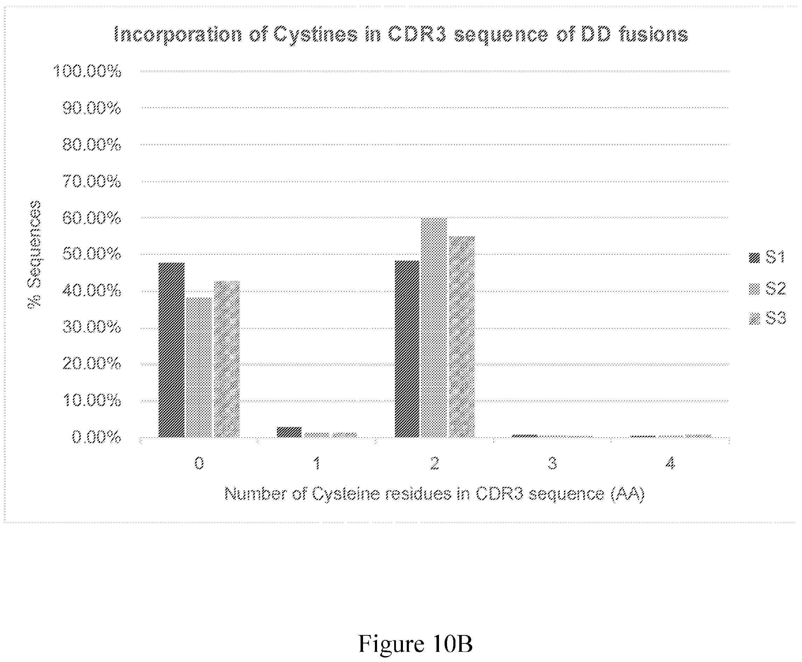

***Figure 10B*** results in connection with an embodiment of the invention, graphing the percentages (y-axes) of all immunoglobulin (Ig) reads resulting from a D_{H}-D_{H} recombination event that have a CDR3 comprising a certain number of cysteine residues (x-axes) isolated from animals modified with the 12:D_{H}2-2:23|12:D_{H}2-8:23|12:D_{H}2-15:23 targeting vector. "S1," "S2," and "S3" each represent a different experimental mouse.

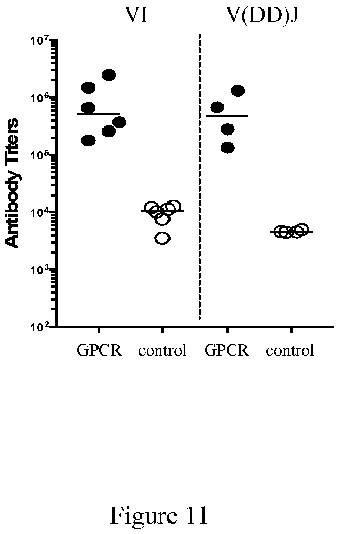

***Figure 11*** shows results in connection with an embodiment of the present invention, graphing representative antibody titers (y-axis) in individual mice having humanized immunoglobulin heavy and κ light chain variable region loci (VI; see, e.g., U.S. Patent Nos. 8,697,940 and 8,642,835; each of which is incorporated herein by reference in its entireties) and mice modified with the 23:D_{H}3-3:12/J_{H}6 targeting vector as described herein (V(DD)J), both cohorts of which were immunized with a DNA immunogen encoding a G protein-coupled receptor (GPCR). Antibody titers were determined by MSD cell binding on 293 cells engineered to express the GPCR (GPCR; x-axis) and on cells that do not express GPCR (control; x-axis).

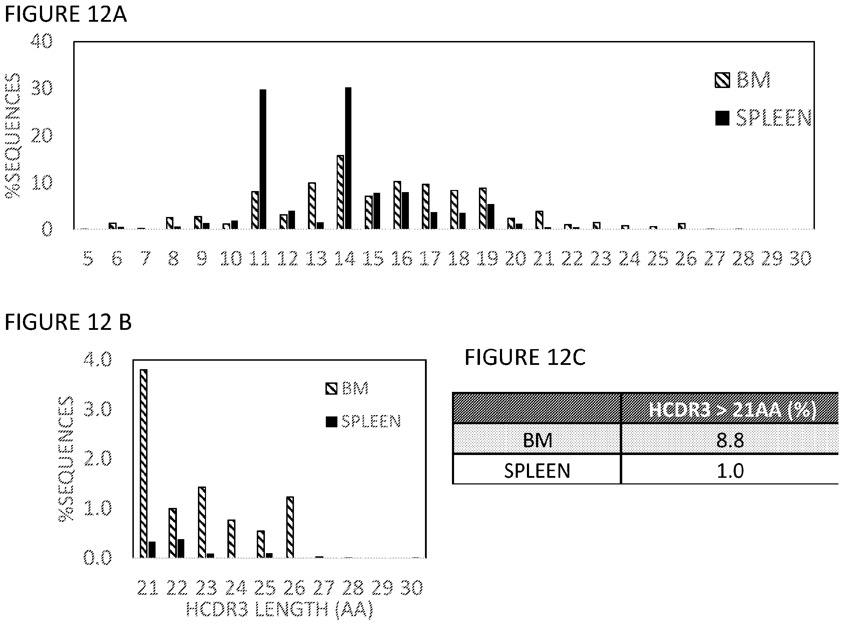

***Figure 12A*** shows results in connection with an embodiment of the present invention, showing the percentage (%; y-axes) of rearranged immunoglobulin heavy chain V_{H}D_{H}J_{H} gene sequences and gene sequences presumed to be the result of V_{H}D_{H}A-D_{H}BJ_{H} rearrangement according to the stringent criterion set forth in Table 7 isolated from the bone marrow (BM) or spleen of mice modified with the 23:D_{H}3-3:12/J_{H}6 targeting vector and encoding a heavy chain CDR3 (HCDR3) having an amino acid (AA) length of 5 to 30 amino acids (x-axes). BM and spleen cell numbers are not normalized to each other. n=1.
***Figure 12B*** shows results in connection with an embodiment of the present invention, providing an enlargement of the graph shown in panel Figure 12A. BM and spleen cell numbers are not normalized to each other. n=1.
***Figure 12C*** shows results in connection with an embodiment of the present invention, showing the percentage of reads having a CDR3 greater than 21 amino acids in length in both the bone marrow or spleen of mice modified with the 23:D_{H}3-3:12/J_{H}6 targeting vector. BM and spleen cell numbers are not normalized to each other. n=1.

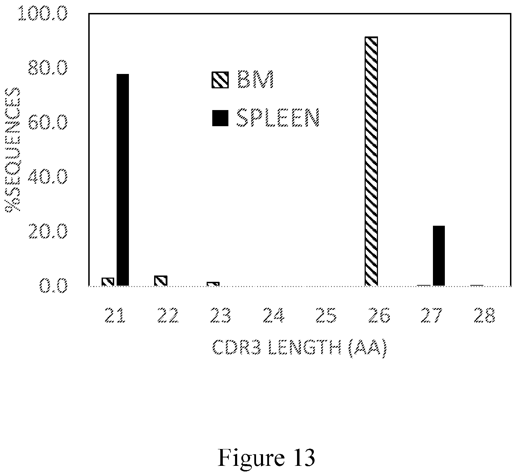

***Figure 13*** shows results in connection with an embodiment of the present invention, showing the percentage (%; y-axes) of presumed rearranged immunoglobulin heavy chain V_{H}D_{H}A-D_{H}BJ_{H} gene sequences (according to the stringent criterion set forth in Table 7) isolated from the bone marrow (BM) or spleen of mice modified with the 23:D_{H}3-3:12/J_{H}6 targeting vector and encoding a heavy chain CDR3 (HCDR3) having an amino acid (AA) length of, all of which were over 20 amino acids in length (x-axes). BM and spleen cell numbers are not normalized to each other. n=1.

### DEFINITIONS

The scope of the present invention is defined by the claims appended hereto and is not limited by particular embodiments described herein; those skilled in the art, reading the present disclosure, will be aware of various modifications that may be equivalent to such described embodiments, or otherwise within the scope of the claims. In general, terminology is in accordance with its understood meaning in the art, unless clearly indicated otherwise. Explicit definitions of certain terms are provided herein and below; meanings of these and other terms in particular instances throughout this specification will be clear to those skilled in the art from context. Additional definitions for the following terms and other terms are set forth throughout the specification. References cited within this specification, or relevant portions thereof, are incorporated herein by reference in their entireties.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

The articles "a" and "an" in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or the entire group members are present in, employed in, or otherwise relevant to a given product or process. Furthermore, it is to be understood that the invention encompasses all variations, combinations, and permutations in which one or more limitations, elements, clauses, descriptive terms, etc., from one or more of the listed claims is introduced into another claim dependent on the same base claim (or, as relevant, any other claim) unless otherwise indicated or unless it would be evident to one of ordinary skill in the art that a contradiction or inconsistency would arise. Where elements are presented as lists, (e.g., in Markush group or similar format) it is to be understood that each subgroup of the elements is also disclosed, and any element(s) can be removed from the group. It should be understood that, in general, where the invention, or aspects of the invention, is/are referred to as comprising particular elements, features, etc., embodiments of the invention or aspects of the invention consist, or consist essentially of, such elements, features, etc. For purposes of simplicity those embodiments have not in every case been specifically set forth in so many words herein. It should also be understood that any embodiment or aspect of the invention can be explicitly excluded from the claims, regardless of whether the specific exclusion is recited in the specification.

As used in this application, the terms "about" and "approximately" are used as equivalents. Any numerals used in this application with or without about/approximately are meant to cover any normal fluctuations, e.g., +/- 5%, appreciated by one of ordinary skill in the relevant art.

***Administration*:** refers to the administration of a composition to a subject or system (e.g., to a cell, organ, tissue, organism, or relevant component or set of components thereof). Those of ordinary skill will appreciate that route of administration may vary depending, for example, on the subject or system to which the composition is being administered, the nature of the composition, the purpose of the administration, etc.

For example, in some embodiments, administration to an animal subject (e.g., to a human or a rodent) may be bronchial (including by bronchial instillation), buccal, enteral, intradermal, intra-arterial, intradermal, intragastric, intramedullary, intramuscular, intranasal, intraperitoneal, intrathecal, intravenous, intraventricular, mucosal, nasal, oral, rectal, subcutaneous, sublingual, topical, tracheal (including by intratracheal instillation), transdermal, vaginal and/or vitreal. In some embodiments, administration may involve intermittent dosing. In some embodiments, administration may involve continuous dosing (e.g., perfusion) for at least a selected period of time. In some embodiments, an antibody produced by a non-human animal disclosed herein can be administered to a subject (e.g., a human subject or rodent). In some embodiments, a pharmaceutical composition includes an antibody produced by a non-human animal disclosed herein. In some embodiments, a pharmaceutical composition can include a buffer, a diluent, an excipient, or any combination thereof. In some embodiments, a pharmaceutical composition including an antibody produced by a non-human animal disclosed herein can be included in a container for storage or administration, for example, a vial, a syringe (e.g., an IV syringe), or a bag (e.g., an IV bag).

***Biologically active*:** refers to a characteristic of any agent that has activity in a biological system, *in vitro* or *in vivo* (e.g., in an organism). For instance, an agent that, when present in an organism, has a biological effect within that organism is considered to be biologically active.

In particular embodiments where a protein or polypeptide is biologically active, a portion of that protein or polypeptide that confers at least one biological activity of the protein or polypeptide is typically referred to as a "*biologically active*" portion.

*Comparable,* refers to two or more agents, entities, situations, sets of conditions, etc. that may not be identical to one another but that are sufficiently similar to permit comparison there between so that conclusions may reasonably be drawn based on differences or similarities observed. Those of ordinary skill in the art will understand, in context, what degree of identity is required in any given circumstance for two or more such agents, entities, situations, sets of conditions, etc. to be considered comparable.

***Conservative*:** refers to a conservative amino acid substitution, i.e., a substitution of an amino acid residue by another amino acid residue having a side chain R group with similar chemical properties (e.g., charge or hydrophobicity). In general, a conservative amino acid substitution will not substantially change the functional properties of interest of a protein, for example, the ability of a receptor to bind to a ligand. Examples of groups of amino acids that have side chains with similar chemical properties include: aliphatic side chains such as glycine, alanine, valine, leucine, and isoleucine; aliphatic-hydroxyl side chains such as serine and threonine; amide-containing side chains such as asparagine and glutamine; aromatic side chains such as phenylalanine, tyrosine, and tryptophan; basic side chains such as lysine, arginine, and histidine; acidic side chains such as aspartic acid and glutamic acid; and sulfur-containing side chains such as cysteine and methionine. Conservative amino acids substitution groups include, for example, valine/leucine/isoleucine, phenylalanine/tyrosine, lysine/arginine, alanine/valine, glutamate/aspartate, and asparagine/glutamine.

In some embodiments, a conservative amino acid substitution can be a substitution of any native residue in a protein with alanine, as used in, for example, alanine scanning mutagenesis. In some embodiments, a conservative substitution is made that has a positive value in the PAM250 log-likelihood matrix disclosed in Gonnet, G. H. et al., 1992, Science 256: 1443-1445, hereby incorporated by reference in its entirety. In some embodiments, a substitution is a moderately conservative substitution wherein the substitution has a nonnegative value in the PAM250 log-li kelihood matrix.

***Control*:** refers to the art-understood meaning of a "*control*" being a standard against which results are compared. Typically, controls are used to augment integrity in experiments by isolating variables in order to make a conclusion about such variables. In some embodiments, a control is a reaction or assay that is performed simultaneously with a test reaction or assay to provide a comparator. A "*control*" may refer to a "*control animal.*" A "*control animal*" may have a modification as described herein, a modification that is different as described herein, or no modification (i.e., a wild-type animal). In one experiment, a "*test*" (i.e., a variable being tested) is applied. In a second experiment, the "*control*," the variable being tested is not applied. A control may be a positive control or a negative control.

In some embodiments, a control is a historical control (i.e., of a test or assay performed previously, or an amount or result that is previously known). In some embodiments, a control is or comprises a printed or otherwise saved record.

***Disruption*:** refers to the result of a homologous recombination event with a DNA molecule (e.g., with an endogenous homologous sequence such as a gene or gene locus).

In some embodiments, a disruption may achieve or represent an insertion, deletion, substitution, replacement, missense mutation, or a frame-shift of a DNA sequence(s), or any combination thereof. Insertions may include the insertion of entire genes, fragments of genes, e.g., exons, which may be of an origin other than the endogenous sequence (e.g., a heterologous sequence), or coding sequences derived or isolated from a particular gene of interest. In some embodiments, a disruption may increase expression and/or activity of a gene or gene product (e.g., of a protein encoded by a gene). In some embodiments, a disruption may decrease expression and/or activity of a gene or gene product. In some embodiments, a disruption may alter the sequence of a gene or an encoded gene product (e.g., an encoded protein). In some embodiments, a disruption may alter sequence of a chromosome or chromosome position in a genome. In some embodiments, a disruption may truncate or fragment a gene or an encoded gene product (e.g., an encoded protein). In some embodiments, a disruption may extend a gene or an encoded gene product. In some such embodiments, a disruption may achieve assembly of a fusion protein. In some embodiments, a disruption may affect level, but not activity, of a gene or gene product. In some embodiments, a disruption may affect activity, but not level, of a gene or gene product. In some embodiments, a disruption may have no significant effect on level of a gene or gene product. In some embodiments, a disruption may have no significant effect on activity of a gene or gene product. In some embodiments, a disruption may have no significant effect on either level or activity of a gene or gene product. In some embodiments, a significant effect can be measured by, e.g., but not limited to, a Student's T-test.

***Endogenous locus*** or ***endogenous gene*:** refers to a genetic locus found in a parent or reference organism (or cell) prior to introduction of an alteration, disruption, deletion, insertion, modification, substitution or replacement as described herein.

In some embodiments, an endogenous locus comprises a sequence, in whole or in part, found in nature. In some embodiments, the endogenous locus is a wild-type locus. In some embodiments, a reference organism is a wild-type organism. In some embodiments, a reference organism is an engineered organism. In some embodiments, a reference organism is a laboratory-bred organism (whether wild-type or engineered).

***Endogenous promoter*:** refers to a promoter that is naturally associated, e.g., in a wild-type organism, with an endogenous gene or genetic locus.

***Engineered*:** refers, in general, to the aspect of having been manipulated by the hand of man. As is common practice and is understood by those in the art, progeny of an engineered polynucleotide or cell are typically still referred to as "*engineered*" even though the actual manipulation was performed on a prior entity. Furthermore, as will be appreciated by those skilled in the art, a variety of methodologies are available through which "*engineering*" as described herein may be achieved.

In some embodiments, a polynucleotide may be considered to be "*engineered*" when two or more sequences that are not linked together in that order in nature are manipulated by the hand of man to be directly linked to one another in the engineered polynucleotide. In some embodiments, an engineered polynucleotide may comprise a regulatory sequence, which is found in nature in operative linkage with a first coding sequence but not in operative linkage with a second coding sequence, linked by the hand of man so that it is operatively linked with the second coding sequence. In engineered D_{H} gene segment embodiments herein, a D_{H} gene segment is manipulated by the hand of man to be operably linked to (e.g., flanked at least one side by, contiguous to, abutting against, immediately adjacent to) a 23-mer RSS. In some embodiments a D_{H} gene segment engineered to be operably linked to a 23-mer RSS is derived from another D_{H} gene segment, e.g., the D_{H} gene segment operably linked to a 23-mer RSS comprises a nucleotide sequence identical to that of the other D_{H} gene segment but for differences due to the degeneracy of the genetic code and/or the replacement of a 12-mer RSS with a 23-mer RSS. An other D_{H} gene segment and an engineered D_{H} gene segment derived therefrom (e.g., a D_{H} gene segment operably linked to a 23-mer RSS) may be considered corresponding gene segments. For example a human D_{H}3-3 gene segment operably linked to a 23-mer RSS may be considered to correspond to a D_{H}3-3 gene segment flanked on one side by a 12-mer RSS and on the other side by another12-mer RSS, wherein the D_{H}3-3 gene segment operably linked to a 23-mer RSS and the D_{H}3-3 gene segment flanked on one side by a 12-mer RSS and on the other side by another 12-mer RSS share an identical nucleotide sequence except for differences due to degeneracy of the genetic code and/or the replacement of one of the two 12-mer RSS with the 23-mer RSS. Alternatively, or additionally, in some embodiments, first and second nucleic acid sequences that each encodes polypeptide elements or domains that in nature are not linked to one another may be linked to one another in a single engineered polynucleotide. Comparably, in some embodiments, a cell or organism may be considered to be "*engineered*" if it has been manipulated so that its genetic information is altered (e.g., new genetic material not previously present has been introduced, or previously present genetic material has been altered or removed).

In some embodiments, "*engineering*" may involve selection or design (e.g., of nucleic acid sequences, polypeptide sequences, cells, tissues, and/or organisms) through use of computer systems programmed to perform analysis or comparison, or otherwise to analyze, recommend, and/or select sequences, alterations, etc.). Alternatively, or additionally, in some embodiments, "*engineering*" may involve use of *in vitro* chemical synthesis methodologies and/or recombinant nucleic acid technologies such as, for example, for example, nucleic acid amplification (e.g., via the polymerase chain reaction) hybridization, mutation, transformation, transfection, etc., and/or any of a variety of controlled mating methodologies. As will be appreciated by those skilled in the art, a variety of established such techniques (e.g., for recombinant DNA, oligonucleotide synthesis, and tissue culture and transformation (e.g., electroporation, lipofection, etc.) are well known in the art and described in various general and more specific references that are cited and/or discussed throughout the present specification. See e.g., Sambrook et al., Molecular Cloning: A Laboratory Manual (2d ed., Cold Spring Harbor Laboratory Press, Cold Spring Harbor, N.Y., 1989; incorporated herein in its entirety by reference).

***Gene*:** refers to a DNA sequence in a chromosome that codes for a product (e.g., an RNA product and/or a polypeptide product). For the purpose of clarity, the term "*gene*" generally refers to a portion of a nucleic acid that encodes a polypeptide; the term may optionally encompass regulatory sequences, as will be clear from context to those of ordinary skill in the art. This definition is not intended to exclude application of the term "*gene*" to non-protein-coding expression units but rather to clarify that, in most cases, the term as used in this document refers to a polypeptide-coding nucleic acid.

In some embodiments, a gene includes coding sequence (i.e., sequence that encodes a particular product). In some embodiments, a gene includes non-coding sequence. In some embodiments, a gene may include both coding (e.g., exonic) and non-coding (e.g., intronic) sequence. In some embodiments, a gene may include one or more regulatory sequences (e.g., promoters, enhancers, *etc*.) and/or intron sequences that, for example, may control or impact one or more aspects of gene expression (e.g., cell-type-specific expression, inducible expression, etc.).

***Heterologous*:** refers to an agent or entity from a different source. For example, when used in reference to a polypeptide, gene, or gene product present in a particular cell or organism, the term clarifies that the relevant polypeptide or fragment thereof, gene or fragment thereof, or gene product or fragment thereof: (1) was engineered by the hand of man; (2) was introduced into the cell or organism (or a precursor thereof) through the hand of man (e.g., via genetic engineering); and/or (3) is not naturally produced by or present in the relevant cell or organism (e.g., the relevant cell type or organism type). Another example includes a polypeptide or fragment thereof, gene or fragment thereof, or gene product or fragment thereof that is normally present in a particular native cell or organism, but has been modified, for example, by mutation or placement under the control of non-naturally associated and, in some embodiments, non-endogenous regulatory elements (e.g., a promoter).

***Host cell*:** refers to a cell into which a heterologous (e.g., exogenous) nucleic acid or protein has been introduced. Persons of skill upon reading this disclosure will understand that such terms refer not only to the particular subject cell, but also is used to refer to the progeny of such a cell. Because certain modifications may occur in succeeding generations due to either mutation or environmental influences, such progeny may not, in fact, be identical to the parent cell, but are still included within the scope of the term "*host cell*"*.*

In some embodiments, a host cell is or comprises a prokaryotic or eukaryotic cell. In embodiments, a host cell is or comprises a mammalian cell. In general, a host cell is any cell that is suitable for receiving and/or producing a heterologous nucleic acid or protein, regardless of the Kingdom of life to which the cell is designated. Exemplary cells include those of prokaryotes and eukaryotes (single-cell or multiple-cell), bacterial cells (e.g., strains of *Escherichia coli, Bacillus* spp., *Streptomyces* spp., etc.), mycobacteria cells, fungal cells, yeast cells (e.g., *Saccharomyces cerevisiae, Schizosaccharomyces pombe, Pichia pastor is, Pichia methanolica,* etc.), plant cells, insect cells (e.g., SF-9, SF-21, baculovirus-infected insect cells, *Trichoplusia ni,* etc.), non-human animal cells, human cells, or cell fusions such as, for example, hybridomas or quadromas.

In some embodiments, the cell is a human, monkey, ape, hamster, rat, or mouse cell. In some embodiments, the cell is eukaryotic and is selected from the following cells: CHO (e.g., CHO K1, DXB-11 CHO, Veggie-CHO), COS (e.g., COS-7), retinal cell, Vero, CV1, kidney (e.g., HEK293, 293 EBNA, MSR 293, MUCK, HaK, BHK), HeLa, HepG2, WI38, MRC 5, Colo205, HB 8065, HL-60, (e.g., BHK21), Jurkat, Daudi, A43 1 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some embodiments, the cell comprises one or more viral genes, e.g., a retinal cell that expresses a viral gene (e.g., a PER.C6^{®} cell). In some embodiments, a host cell is or comprises an isolated cell. In some embodiments, a host cell is part of a tissue. In some embodiments, a host cell is part of an organism.

***Humanized*:** refers to a molecule (e.g., a nucleic acid, protein, etc.) that was non-human in origin and for which a portion has been replaced with a corresponding portion of a corresponding human molecule in such a manner that the modified (e.g., humanized) molecule retains its biological function and/or maintains the structure that performs the retained biological function. In contrast "human" and the like encompasses molecules having only a human origin, e.g., human nucleotides or protein comprising only human nucleotide and amino acid sequences respectively. The term "human(ized)" is used to reflect that the human(ized) molecule may be (a) a human molecule or (b) a humanized molecule.

***Identity*:** in connection with a comparison of sequences, refers to identity as determined by a number of different algorithms known in the art that can be used to measure nucleotide and/or amino acid sequence identity.

In some embodiments, identities as described herein are determined using a ClustalW v. 1.83 (slow) alignment employing an open gap penalty of 10.0, an extend gap penalty of 0.1, and using a Gonnet similarity matrix (MACVECTOR^{™} 10.0.2, MacVector Inc., 2008).

***In vitro*:** refers to events that occur in an artificial environment, e.g., in a test tube or reaction vessel, in cell culture, etc., rather than within a multi-cellular organism.

***In vivo*:** refers to events that occur within a multi-cellular organism, such as a human and/or a non-human animal. In the context of cell-based systems, the term may be used to refer to events that occur within a living cell (as opposed to, for example, *in vitro* systems).

***Isolated*:** refers to a substance and/or entity that has been (1) separated from at least some of the components with which it was associated when initially produced (whether in nature and/or in an experimental setting), and/or (2) designed, produced, prepared, and/or manufactured by the hand of man. Isolated substances and/or entities may be separated from about 10 or more of the other components with which they were initially associated. In some embodiments, isolated agents are at least about 80% or more pure. A substance is "*pure*" if it is substantially free of other components. In some embodiments, as will be understood by those skilled in the art, a substance may still be considered "*isolated*" or even "*pure*", after having been combined with certain other components such as, for example, one or more carriers or excipients (e.g., buffer, solvent, water, etc.); in such embodiments, percent isolation or purity of the substance is calculated without including such carriers or excipients.

To give but one example, in some embodiments, a biological polymer such as a polypeptide or polynucleotide that occurs in nature is considered to be *"isolated"* when: (a) by virtue of its origin or source of derivation is not associated with some or all of the components that accompany it in its native state in nature; (b) it is substantially free of other polypeptides or nucleic acids of the same species from the species that produces it in nature; or (c) is expressed by or is otherwise in association with components from a cell or other expression system that is not of the species that produces it in nature. Thus, for instance, in some embodiments, a polypeptide that is chemically synthesized or is synthesized in a cellular system different from that which produces it in nature is considered to be an *"isolated"* polypeptide. Alternatively, or additionally, in some embodiments, a polypeptide that has been subjected to one or more purification techniques may be considered to be an "*isolated*" polypeptide to the extent that it has been separated from other components: a) with which it is associated in nature; and/or b) with which it was associated when initially produced.

***Non-human* animal:** refers to any vertebrate organism that is not a human.

In some embodiments, a non-human animal is a cyclostome, a bony fish, a cartilaginous fish (e.g., a shark or a ray), an amphibian, a reptile, a mammal, and a bird. In some embodiments, a non-human mammal is a primate, a goat, a sheep, a pig, a dog, a cow, or a rodent. In some embodiments, a non-human animal is a rodent such as a rat or a mouse.

***Nucleic acid*:** in its broadest sense, refers to any compound and/or substance that is or can be incorporated into an oligonucleotide chain and is generally interchangeable with nucleic acid molecule, nucleic acid sequence, nucleotide molecule, nucleotide molecule, which terms are also interchangeable with each other.

In some embodiments, a *"nucleic acid"* is a compound and/or substance that is or can be incorporated into an oligonucleotide chain via a phosphodiester linkage. As will be clear from context, in some embodiments, *"nucleic acid"* refers to individual nucleic acid residues (e.g., nucleotides and/or nucleosides); in some embodiments, "*nucleic acid*" refers to an oligonucleotide chain comprising individual nucleic acid residues. In some embodiments, a "*nucleic acid*'*'* is or comprises RNA; in some embodiments, a "*nucleic acid*" is or comprises DNA. In some embodiments, a "*nucleic acid*" is, comprises, or consists of one or more natural nucleic acid residues. In some embodiments, a *"nucleic acid"* is, comprises, or consists of one or more nucleic acid analogs. In some embodiments, a nucleic acid analog differs from a *"nucleic acid"* in that it does not utilize a phosphodiester backbone. For example, in some embodiments, a "*nucleic acid*" is, comprises, or consists of one or more "*peptide nucleic acids*", which are known in the art and have peptide bonds instead of phosphodiester bonds in the backbone, are considered within the scope of the present invention. Alternatively, or additionally, in some embodiments, a *"nucleic acid"* has one or more phosphorothioate and/or 5'-N-phosphoramidite linkages rather than phosphodiester bonds. In some embodiments, a "*nucleic acid*" is, comprises, or consists of one or more natural nucleosides (e.g., adenosine, thymidine, guanosine, cytidine, uridine, deoxyadenosine, deoxythymidine, deoxyguanosine, and deoxycytidine). In some embodiments, a "*nucleic acid*" is, comprises, or consists of one or more nucleoside analogs (e.g., 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, CS-bromouridine, C5-fluorouridine, CS-iodouridine, C5-propynyl-uridine, C5-propynyl-cytidine, C5-methyl cytidine, 2-aminoadenosine, 7-deazaadenosine, 7-deazaguanosine, 8-oxoadenosine, 8-oxoguanosine, O(6)-methylguanine, 2-thiocytidine, methylated bases, intercalated bases, and combinations thereof). In some embodiments, a "*nucleic acid*" comprises one or more modified sugars (e.g., 2'-fluororibose, ribose, 2'-deoxyribose, arabinose, and hexose) as compared with those in natural nucleic acids. In some embodiments, a *"nucleic acid"* has a nucleotide sequence that encodes a functional gene product such as an RNA or protein. In some embodiments, a "*nucleic acid*" has a nucleotide sequence that encodes polypeptide fragment (e.g., a peptide). In some embodiments, a *"nucleic acid"* includes one or more introns. In some embodiments, a "*nucleic acid*" includes one or more exons. In some embodiments, a "*nucleic acid*" includes one or more coding sequences. In some embodiments, a "*nucleic acid*" is prepared by one or more of isolation from a natural source, enzymatic synthesis by polymerization based on a complementary template (*in two* or *in vitro*)*,* reproduction in a recombinant cell or system, and chemical synthesis. In some embodiments, a "*nucleic acid*" is at least 3 or more residues long. In some embodiments, a "*nucleic acid*" is single stranded; in some embodiments, a "*ucleic acid*" is double stranded. In some embodiments, a "*nucleic acid*" has a nucleotide sequence comprising at least one element that encodes, or is the complement of a sequence that encodes, a polypeptide or fragment thereof. In some embodiments, a "*nucleic acid*" has enzymatic activity.

***Operably linked*:** refers to a juxtaposition wherein the components described are in a relationship permitting them to function in their intended manner.

In some embodiments, operably linked nucleotide sequences are contiguous with one another, e.g., a nucleic acid sequence comprising an immunoglobulin gene segment operably linked to an RSS comprises the immunoglobulin gene segment nucleotide sequence contiguous with the RSS nucleotide sequence, e.g., the immunoglobulin gene segment is flanked at least on one side by (e.g., abuts) the RSS nucleotide sequence in a contiguous manner such that the immunoglobulin gene segment is immediately adjacent to the RSS nucleotide sequence.

In other embodiments, operable linkage does not require contiguity. For example, unrearranged variable region gene segments "operably linked" to each other are capable of rearranging to form a rearranged variable region gene, which unrearranged variable region gene segments may not necessarily be contiguous with one another. Unrearranged variable region gene segments operably linked to each other and to a contiguous constant region gene are capable of rearranging to form a rearranged variable region gene that is expressed in conjunction with the constant region gene as a polypeptide chain of an antigen binding protein. A control sequence "*operably linked*" to a coding sequence is ligated in such a way that expression of the coding sequence is achieved under conditions compatible with the control sequences. "*Operably linked*" sequences include both expression control sequences that are contiguous with the gene of interest and expression control sequences that act in trans or at a distance to control the gene of interest.

The term "*expression control sequence*", refers to polynucleotide sequences, which are necessary to affect the expression and processing of coding sequences to which they are ligated. "*Expression control sequences*" include: appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. The nature of such control sequences differs depending upon the host organism. For example, in prokaryotes, such control sequences generally include promoter, ribosomal binding site and transcription termination sequence, while in eukaryotes typically, such control sequences include promoters and transcription termination sequence. The term "*control sequences*" is intended to include components whose presence is essential for expression and processing and can also include additional components whose presence is advantageous, for example, leader sequences and fusion partner sequences.

Generally, each unrearranged immunoglobulin V gene segment, D gene segment, or J gene segment is operably linked to (e.g., associated with, flanked on by one or both sides with, contiguous with, etc.) a recombination signal sequence (RSS), which may be a 12-mer RSS or a 23-mer RSS. Any gene segment flanked on each side by an RSS (including a D_{H} gene segment operably linked to a 23-mer RSS) has not undergone recombination, and thus, may be considered an "unrearranged" gene segment. In some embodiments, an unrearranged gene segment herein may comprise a gene segment in its germline (e.g., wildtype) configuration, e.g., a germline V_{H} gene segment and a germline J_{H} gene segment are each flanked on both sides by 23-mer RSS. In contrast, a germline D_{H} gene segment, e.g., an unrearranged D_{H} gene segment, is flanked on each side by a 12-mer RSS. A D_{H} gene segment operably linked to a 23-mer RSS, e.g., an engineered D_{H} gene segment, also has not undergone recombination, and thus, comprises (i) a 23-mer RSS and (ii) a 12-mer RSS. An engineered D_{H} gene segment (e.g., a D_{H} gene segment operably linked to a 23-mer RSS) is able to rearrange with another D_{H} gene segment operably linked with a 12-mer RSS in accordance with the 12/23 rule of recombination.

***Physiological conditions*:** has its art-understood meaning referencing conditions under which cells or organisms live and/or reproduce. In some embodiments, the term refers to conditions of the external or internal milieu that may occur in nature for an organism or cell system. In some embodiments, physiological conditions are those conditions present within the body of a human or non-human animal, especially those conditions present at and/or within a surgical site. Physiological conditions typically include, e.g., a temperature range of 20-40°C, atmospheric pressure of 1, pH of 6-8, glucose concentration of 1-20 mM, oxygen concentration at atmospheric levels, and gravity as it is encountered on earth. In some embodiments, conditions in a laboratory are manipulated and/or maintained at physiological conditions. In some embodiments, physiological conditions are encountered in an organism (e.g., non-human animal).

***Polypeptide*:** refers to any polymeric chain of amino acids.

In some embodiments, a polypeptide has an amino acid sequence that occurs in nature. In some embodiments, a polypeptide has an amino acid sequence that does not occur in nature. In some embodiments, a polypeptide has an amino acid sequence that contains portions that occur in nature separately from one another (i.e., from two or more different organisms, for example, human and non-human portions). In some embodiments, a polypeptide has an amino acid sequence that is engineered in that it is designed and/or produced through action of the hand of man. In some embodiments, a polypeptide may comprise or consist of a plurality of fragments, each of which is found in the same parent polypeptide in a different spatial arrangement relative to one another than is found in the polypeptide of interest (e.g., fragments that are directly linked in the parent may be spatially separated in the polypeptide of interest or vice versa, and/or fragments may be present in a different order in the polypeptide of interest than in the parent), so that the polypeptide of interest is a derivative of its parent polypeptide.

***Recombinant*:** refers to polypeptides that are designed, engineered, prepared, expressed, created or isolated by recombinant means, such as polypeptides expressed using a recombinant expression vector transfected into a host cell, polypeptides isolated from a recombinant, combinatorial human polypeptide library (Hoogenboom H. R., 1997 TIB Tech. 15:62-70; Hoogenboom H., and Chames P., 2000, Immunology Today 21:371-378; Azzazy H., and Highsmith W. E., 2002, Clin. Biochem. 35:425-445; Gavilondo J. V., and Larrick J. W., 2002, BioTechniques 29:128-145), antibodies isolated from an animal (e.g., a mouse) that is transgenic for human immunoglobulin genes (see e.g., Taylor, L. D., et al., 1992, Nucl. Acids Res. 20:6287-6295; Little M. et al., 2000, Immunology Today 21:364-370; Kellermann S. A. and Green L. L., 2002, Current Opinion in Biotechnology 13:593-597; Murphy, A.J., et al., 2014, Proc. Natl. Acad. Sci. U. S. A. 111(14):5153-5158; each of which is incorporated herein in its entirety by reference) or polypeptides prepared, expressed, created or isolated by any other means that involves splicing selected sequence elements to one another.

In some embodiments, one or more of such selected sequence elements is found in nature. In some embodiments, one or more of such selected sequence elements is designed *in silico.* In some embodiments, one or more such selected sequence elements result from mutagenesis (e.g., *in vivo* or *in vitro*) of a known sequence element, e.g., from a natural or synthetic source. For example, in some embodiments, a recombinant polypeptide comprises sequences found in the genome (or polypeptide) of a source organism of interest (e.g., human, mouse, etc.). In some embodiments, a recombinant polypeptide comprises sequences that occur in nature separately from one another (i.e., from two or more different organisms, for example, human and non-human portions) in two different organisms (e.g., a human and a non-human organism). In some embodiments, a recombinant polypeptide has an amino acid sequence that resulted from mutagenesis (e.g., *in vitro* or *in vivo,* for example in a non-human animal), so that the amino acid sequences of the recombinant polypeptides are sequences that, while originating from and related to polypeptide sequences, may not naturally exist within the genome of a non-human animal *in vivo.*

***Reference*:** refers to a standard or control agent, animal, cohort, individual, population, sample, sequence or value against which an agent, animal, cohort, individual, population, sample, sequence or value of interest is compared. A "*reference*" may refer to a "*reference animal*". A "*reference animal*" may have a modification as described herein, a modification that is different as described herein or no modification (i.e., a wild-type animal). Typically, as would be understood by those skilled in the art, a reference agent, animal, cohort, individual, population, sample, sequence or value is determined or characterized under conditions comparable to those utilized to determine or characterize the agent, animal (e.g., a mammal), cohort, individual, population, sample, sequence or value of interest.

In some embodiments, a reference agent, animal, cohort, individual, population, sample, sequence or value is tested and/or determined substantially simultaneously with the testing or determination of the agent, animal, cohort, individual, population, sample, sequence or value of interest. In some embodiments, a reference agent, animal, cohort, individual, population, sample, sequence or value is a historical reference, optionally embodied in a tangible medium. In some embodiments, a reference may refer to a control.

***Substantially*:** refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "*substantially*" is therefore used to capture the potential lack of completeness inherent in many biological and chemical phenomena.

***Substantial homology*:** refers to a comparison between amino acid or nucleic acid sequences. As will be appreciated by those of ordinary skill in the art, two sequences are generally considered to be "*substantially homologous*" if they contain homologous residues in corresponding positions. Homologous residues may be identical residues. Alternatively, homologous residues may be non-identical residues with appropriately similar structural and/or functional characteristics. For example, as is well known by those of ordinary skill in the art, certain amino acids are typically classified as "*hydrophobic*" or "*hydrophilic*" amino acids, and/or as having "*polar*" or "*non-polar*" side chains. Substitution of one amino acid for another of the same type may often be considered a "*homologous*" substitution. Typical amino acid categorizations are summarized below:

| | | | | | |
|---|---|---|---|---|---|
| Alanine | Ala | A | Nonpolar | Neutral | 1.8 |
| Arginine | Arg | R | Polar | Positive | -4.5 |
| Asparagine | Asn | N | Polar | Neutral | -3.5 |
| Aspartic acid | Asp | D | Polar | Negative | -3.5 |
| Cysteine | Cys | C | Nonpolar | Neutral | 2.5 |
| Glutamic acid | Glu | E | Polar | Negative | -3.5 |
| Glutamine | Gln | Q | Polar | Neutral | -3.5 |
| Glycine | Gly | G | Nonpolar | Neutral | -0.4 |
| Histidine | His | H | Polar | Positive | -3.2 |
| Isoleucine | Ile | I | Nonpolar | Neutral | 4.5 |
| Leucine | Leu | L | Nonpolar | Neutral | 3.8 |
| Lysine | Lys | K | Polar | Positive | -3.9 |
| Methionine | Met | M | Nonpolar | Neutral | 1.9 |
| Phenylalanine | Phe | F | Nonpolar | Neutral | 2.8 |
| Proline | Pro | P | Nonpolar | Neutral | -1.6 |
| Serine | Ser | S | Polar | Neutral | -0.8 |
| Threonine | Thr | T | Polar | Neutral | -0.7 |
| Tryptophan | Trp | W | Nonpolar | Neutral | -0.9 |
| Tyrosine | Tyr | Y | Polar | Neutral | -1.3 |
| Valine | Val | V | Nonpolar | Neutral | 4.2 |

| Ambiguous Amino Acids | 3-Letter | 1-Letter |
|---|---|---|
| Asparagine or aspartic acid | Asx | B |
| Glutamine or glutamic acid | Glx | Z |
| Leucine or Isoleucine | Xle | J |
| Unspecified or unknown amino acid | Xaa | X |

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, S. F. et al., 1990, J. Mol. Biol., 215(3): 403-410; Altschul, S. F. et al., 1996, Methods in Enzymol. 266:460-80; Altschul, S. F. et al., 1997, Nucleic Acids Res., 25:3389-402; Baxevanis, A.D., and B. F. F. Ouellette (eds.) Bioinformatics: A Practical Guide to the Analysis of Genes and Proteins, Wiley, 1998; and Misener et al. (eds.) Bioinformatics Methods and Protocols (Methods in Molecular Biology, Vol. 132), Humana Press, 1998. In addition to identifying homologous sequences, the programs mentioned above typically provide an indication of the degree of homology.

In some embodiments, two sequences are considered to be substantially homologous if at least 95% or more of their corresponding residues are homologous over a relevant stretch of residues. In some embodiments, the relevant stretch is a complete sequence. In some embodiments, the relevant stretch is at least 9 or more residues. In some embodiments, the relevant stretch includes contiguous residues along a complete sequence. In some embodiments, the relevant stretch includes discontinuous residues along a complete sequence, for example, noncontiguous residues brought together by the folded conformation of a polypeptide or a portion thereof. In some embodiments, the relevant stretch is at least 10 or more residues.

***Substantial identity*:** refers to a comparison between amino acid or nucleic acid sequences. As will be appreciated by those of ordinary skill in the art, two sequences are generally considered to be *"substantially identical*" if they contain identical residues in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, S. F. et al., 1990, J. Mol. Biol., 215(3): 403-410; Altschul, S. F. et al., 1996, Methods in Enzymol. 266:460-80; Altschul, S. F. et al., 1997, Nucleic Acids Res., 25:3389-3402; Baxevanis, A.D., and B. F. F. Ouellette (eds.) Bioinformatics: A Practical Guide to the Analysis of Genes and Proteins, Wiley, 1998; and Misener et al. (eds.) Bioinformatics Methods and Protocols (Methods in Molecular Biology, Vol. 132), Humana Press, 1998. In addition to identifying identical sequences, the programs mentioned above typically provide an indication of the degree of identity.

In some embodiments, two sequences are considered to be substantially identical if at least 95% or more of their corresponding residues are identical over a relevant stretch of residues. In some embodiments, the relevant stretch is a complete sequence. In some embodiments, the relevant stretch is at least 10 or more residues.

***Targeting vector*** or ***targeting construct*:** refers to a polynucleotide molecule that comprises a targeting region. A targeting region comprises a sequence that is identical or substantially identical to a sequence in a target cell, tissue or animal and provides for integration of the targeting construct into a position within the genome of the cell, tissue or animal via homologous recombination. Targeting regions that target using site-specific recombinase recognition sites (e.g., *loxP* or Frt sites) are also included.

In some embodiments, a targeting construct as described herein further comprises a nucleic acid sequence or gene of particular interest, a selectable marker, control and or regulatory sequences, and other nucleic acid sequences that allow for recombination mediated through exogenous addition of proteins that aid in or facilitate recombination involving such sequences. In some embodiments, a targeting construct as described herein further comprises a gene of interest in whole or in part, wherein the gene of interest is a heterologous gene that encodes a polypeptide, in whole or in part, that has a similar function as a protein encoded by an endogenous sequence. In some embodiments, a targeting construct as described herein further comprises a humanized gene of interest, in whole or in part, wherein the humanized gene of interest encodes a polypeptide, in whole or in part, that has a similar function as a polypeptide encoded by an endogenous sequence. In some embodiments, a targeting construct (or targeting vector) may comprise a nucleic acid sequence manipulated by the hand of man. For example, in some embodiments, a targeting construct (or targeting vector) may be constructed to contain an engineered or recombinant polynucleotide that contains two or more sequences that are not linked together in that order in nature yet manipulated by the hand of man to be directly linked to one another in the engineered or recombinant polynucleotide.

***Transgene*** or ***transgene construct*:** refers to a nucleic acid sequence (encoding e.g., a polypeptide of interest, in whole or in part) that has been introduced into a cell by the hand of man such as by the methods described herein. A transgene could be partly or entirely heterologous, i.e., foreign, to the transgenic animal or cell into which it is introduced. A transgene can include one or more transcriptional regulatory sequences and any other nucleic acid, such as introns or promoters, which may be necessary for expression of a selected nucleic acid sequence. A transgene can include one or more selectable markers that allow for subsequent selection of progeny (e.g., cells) that have taken up the transgene.

***Transgenic animal*, *transgenic* n*on-human animal*** or ***Tg⁺*:** are used interchangeably herein and refer to any non-naturally occurring non-human animal in which one or more of the cells of the non-human animal contain heterologous nucleic acid and/or gene encoding a polypeptide of interest, in whole or in part.

In some embodiments, a heterologous nucleic acid sequence and/or gene is introduced into the cell, directly or indirectly by introduction into a precursor cell, by way of deliberate genetic manipulation, such as by microinjection or by infection with a recombinant virus. The term genetic manipulation does not include classic breeding techniques, but rather is directed to introduction of recombinant DNA molecule(s). This molecule may be integrated within a chromosome, or it may be extrachromosomally replicating DNA. The term "*Tg*⁺" includes animals that are heterozygous or homozygous for a heterologous nucleic acid and/or gene, and/or animals that have single or multi-copies of a heterologous nucleic acid and/or gene.

***Vector*:** refers to a nucleic acid molecule capable of transporting another nucleic acid to which it is associated.

In some embodiment, vectors are capable of extra-chromosomal replication and/or expression of nucleic acids to which they are linked in a host cell such as a eukaryotic and/or prokaryotic cell. Vectors capable of directing the expression of operably linked genes are referred to herein as "*expression vectors*."

***Wild-type*:** has its art-understood meaning that refers to an entity having a structure and/or activity as found in nature in a "*normal*'*'* (as contrasted with mutant, diseased, engineered, altered, etc.) state or context. Those of ordinary skill in the art will appreciate that wild-type genes and polypeptides often exist in multiple different forms (e.g., alleles).

Other features, objects, and advantages of the present invention are apparent in the detailed description of some embodiments that follows. It should be understood, however, that the detailed description, while indicating some embodiments of the present invention, is given by way of illustration only, not limitation. Various changes and modifications within the scope of the invention will become apparent to those skilled in the art from the detailed description.

### DETAILED DESCRIPTION OF CERTAIN EMBODIMENTS

The present invention provides, among other things, transgenic or engineered non-human animals having heterologous genetic material encoding one or more portions (functional fragments, binding portions, etc.) of human immunoglobulins, which heterologous genetic material is inserted into an immunoglobulin heavy chain variable region locus so that the heterologous genetic material is operably linked with heavy chain constant (C_{H}) genes. It is contemplated that such non-human animals demonstrate a capacity to generate antibodies encoded by rearranged V(DD)J sequences. It is also contemplated that such non-human animals demonstrate an antibody population characterized by heavy chain variable regions having an increase in CDR3 diversity as compared to an antibody population having immunoglobulin heavy chain variable CDR3 diversity generated from a wild-type immunoglobulin heavy chain variable region locus (or an immunoglobulin heavy chain variable region locus that appears in nature). Therefore, provided non-human animals are particularly useful for the development of antibody-based therapeutics that bind particular antigens, in particular, antigens associated with low and/or poor immunogenicity or antigens that are characterized by one or more epitopes that are unfavorable for binding by traditional (or wild-type) antibodies. In particular, the present invention encompasses the introduction of a 23-mer recombination signal sequence adjacent to (e.g., 5' or 3' of) one or more D_{H} segments of a D_{H} region in an immunoglobulin heavy chain variable region resulting in the capacity for D_{H}-to-D_{H} segment rearrangement during VDJ recombination, and expression of antibodies having heavy chain variable regions and, in particular, CDR3 regions, which may be characterized by a longer amino acid length as compared to antibodies generated from VDJ recombination involving single D_{H} segments. Such transgenic non-human animals provide an *in vivo* system for identifying and developing antibodies and/or antibody-based therapeutics that bind disease targets beyond the targeting capabilities of established drug discovery technologies. Further, such transgenic non-human animals provide a useful animal model system for the development of antibodies and/or antibody-based therapeutics centered on or designed for disrupting protein-protein interactions that are central to various diseases and/or disease pathologies that affect humans.

As shown in ***Figure 1******,* top panel,** recombination between immunoglobulin gene segments follows a rule commonly referred to as the 12/23 rule, in which gene segments flanked by recombination signal sequences (RSS) are joined by an ordered process. Each RSS consists of a conserved block of seven nucleotides (heptamer; 5'-CACAGTG-3'; SEQ ID NO: 144) that is contiguous with a coding sequence (e.g., a V_{H}, D_{H} or J_{H} segment) followed by a non-conserved region, known as the spacer which is either 12bp or 23bp in length, and a second conserved block of nine nucleotides (nonamer; 5'-ACAAAAACC-3'; SEQ ID NO: 145). The spacer may vary in sequence, but its conserved length corresponds to one or two turns of the DNA double helix. This brings the heptamer and nonamer sequences to the same side of the DNA helix, where they can be bound by a complex of proteins that catalyzes recombination. An RSS with a 23bp spacer is a 23-mer RSS and an RSS with a 12bp spacer is a 12-mer RSS. The 12/23 rule of recombination generally promotes recombination between a 12-mer RSS and a 23-mer RSS and prohibits recombination between a 23-mer RSS and another 23-mer RSS, or between a 12-mer RSS and another 12-mer RSS, e.g., prohibits direct germline V_{H}-to-germline J_{H} recombination (i.e., 23-mer-to-23-mer joining) or germline D_{H}-germlitie D_{H} recombination (i.e., 12-mer-to-12-mer joining), although exceptions have been reported.

In some embodiments, non-human animals as described herein comprise an immunoglobulin heavy chain variable region containing an engineered diversity cluster (i.e., an engineered D_{H} region) characterized by the presence of one or more D_{H} segments each of which are operably linked to a 23-mer RSS and accordingly are capable of D_{H}-D_{H} recombination. In some embodiments, antibodies containing CDR3s generated from such recombination may be characterized as having increased diversity resulting from longer amino acid length to direct binding to particular antigens (e.g., viruses, membrane channels, etc.). In some embodiments, non-human animals described herein comprise human heavy chain variable (V_{H}) and joining (J_{H}) gene segments operably linked with the engineered D_{H} region so that VDJ recombination occurs between said V_{H}, J_{H} and more than one D_{H} segment to create a heavy chain variable region that binds an antigen of interest. In some embodiments, an engineered D_{H} region as described herein contains one or more (e.g., 1, 2, 3, 4, 5, 10 or more) human D_{H} segments engineered to allow for (or promote) D_{H}-to-D_{H} recombination at an increased frequency as compared to a reference immunoglobulin heavy chain variable region locus. In some embodiments, non-human animals as described herein comprise a plurality of V_{H} and J_{H} gene segments operably linked to one or more D_{H} segments that are each operably linked to a 23-mer recombination signal sequence (RSS) at an immunoglobulin heavy chain variable region in the genome of the non-human animal. In many embodiments, V_{H} and J_{H} segments are human V_{H} and human J_{H} gene segments.

In some embodiments, non-human animals as described herein further comprise a human or humanized immunoglobulin light chain locus (e.g., κ and/or λ) such that the non-human animals produce antibodies comprising human variable regions (i.e., heavy and light) and non-human constant regions. In some embodiments, said human or humanized immunoglobulin light chain locus comprises human V_{L} and J_{L} gene segments operably linked to a rodent light chain constant region (e.g., a rodent Cκ or Cλ). In some embodiments, non-human animals described herein further comprise an immunoglobulin light chain locus as described in U.S. Patent Nos. 9,796,788; 9,969,814; U.S. Patent Application Publication Nos. 2011/0195454 A1, 2012/0021409 A1, 2012/0192300 A1, 2013/0045492 A1, 2013/0185821 A1, 2013/0302836 A1, 2018/0125043; International Patent Application Publication Nos. WO 2011/097603, WO 2012/148873, WO 2013/134263, WO 2013/184761, WO 2014/160179, WO 2014/160202; and "WO2019/113065 each of which are hereby incorporated by reference in its entireties).

Various aspects of the invention are described in detail in the following sections. The use of sections is not meant to be limiting to embodiments described herein. Each section can apply to any aspect or embodiment described herein. In this application, the use of "or" means "and/or" unless stated otherwise.

### VDJ Recombination

Genes responsible for immunoglobulin synthesis are found in all cells of an animal and are arranged in gene segments, sequentially situated along the chromosome. The configuration of human gene segments that are inherited, e.g., the germline configuration of human gene segments, e.g., the order of human gene segments in the germline genome (e.g., the genome passed down to the next generation) of a human, may be found at Lefranc, M.-P., Exp. Clin. Immunogenet., 18, 100-116 (2001), incorporated herein in its entirety by reference, which also shows functional gene segments and pseudogenes found within the human immunoglobulin heavy chain locus in germline configuration. A series of recombination events, involving several genetic components, serves to assemble immunoglobulins from ordered arrangement of gene segments (e.g., V, D and J). This assembly of gene segments is known to be imprecise and, therefore, immunoglobulin diversity is achieved both by combination of different gene segments and formation of unique junctions through imprecise joining. Further diversity is generated through a process known as somatic hypermutation in which the variable region sequence of immunoglobulins is altered to increase affinity and specificity for antigen. The immunoglobulin molecule is a Y-shaped polypeptide composed of two identical heavy and two identical light chains, each of which have two structural components: one variable domain and one constant domain. It is the variable domains of heavy and light chains that are formed by the assembly of gene segments, while constant domains are fused to variable domains through RNA splicing. Although the mechanism of assembling (or joining) gene segments is similar for heavy and light chains, only one joining event is required for light chains (i.e., V to J) while two are required for heavy chains (i.e., D to J and V to DJ).

The assembly of gene segments for heavy and light chain variable regions (referred to respectively as VDJ recombination and VJ recombination) is guided by conserved noncoding DNA sequences that flank each gene segment, termed recombination signal sequences (RSSs), which ensure DNA rearrangements at precise locations relative to V, D and J coding sequences (see, e.g., Ramsden, D.A. et al., 1994, Nuc. Acids Res. 22(10):1785-96; incorporated herein in its entirety by reference). A representative schematic of the sequences involved in VDJ recombination of heavy chain gene segments as understood by a skilled artisan is set forth in Figure 1. Each RSS consists of a conserved block of seven nucleotides (heptamer) that is contiguous with a coding sequence (e.g., a V, D or J segment) followed by a spacer (either 12bp or 23bp) and a second conserved block of nine nucleotides (nonamer). Although considerable sequence divergence in the 12bp or 23Bp spacer among individuals is tolerated, the length of these sequences typically does not vary. Recombination between immunoglobulin gene segments follows a rule commonly referred to as the 12/23 rule, in which gene segments flanked by an RSS with a 12bp spacer (or 12-mer) are typically joined to a gene segment flanked by a 23bp spacer (or 23-rner; see, e.g., Hiom, K. and M. Gellert, 1998, Mol. Cell. 1(7):1011-9; incorporated herein in its entirety by reference). The sequence of an RSS has been reported to influence the efficiency and/or the frequency of recombination with a particular gene segment (see, e.g., Ramsden, D.A and G.E. Wu, 1991, Proc. Natl. Acad. Sci. U.S.A. 88:10721-5; Boubnov, N.V. et al., 1995, Nuc. Acids Res. 23:1060-7; Ezekiel, U.R. et al., 1995, Immunity 2:381-9; Sadofsky, M. et al., 1995, Genes Dev. 9:2193-9; Cuomo, C.A. et al., 1996, Mol. Cell Biol. 16:5683-90; Ramsden, D.A. et al., 1996, EMBO J 15:3197-3206; each of which is incorporated herein in its entirety by reference). Indeed, many reports point to a highly biased and variable usage of gene segments, in particular, D_{H} segments, among individuals. Unless otherwise indicated or unless it would be evident to one of ordinary skill in the art that a contradiction or inconsistency would arise, an unrearranged gene segment without reference to an RSS is presumed to comprise the two RSS with which the gene segment is naturally associated, e.g., flanked by, operably linked to, etc. In some embodiments, an unrearranged gene segment herein may comprise a gene segment in its germline (e.g., wildtype) configuration, e.g., a germline V_{H} gene segment and a germline J_{H} gene segment are each flanked on both sides by 23-mer RSS. In contrast, a germline D_{H} gene segment, e.g., an unrearranged D_{H} gene segment, is flanked on each side by a 12-mer RSS.

As such, an unrearranged gene segment may also refer to a gene segment in its germline configuration, including any RSS associated with such germline configuration. Moreover, a plurality of gene segments in their germline configuration generally refers to the not only each individual gene segment being in its germline (e.g., unrearranged) configuration, but also the order and/or location of the functional gene segments. See, e.g., Lefranc, M.-P., Exp. Clin. Immunogenet., 18, 100-116 (2001); incorporated herein in its entirety by reference, for the germline configuration of human V, D and J gene segments.

The assembly of gene segments to form heavy and light chain variable regions results in the formation of antigen-binding regions (or sites) of immunoglobulins. Such antigen-binding regions are characterized, in part, by the presence of hypervariable regions, which are commonly referred to as complementary determining regions (CDRs). There are three CDRs for both heavy and light chains (i.e., for a total of six CDRs) with both CDR1 and CDR2 being entirely encoded by the V gene segment. CDR3, however, is encoded by the sequence resulting from the joining of the V and J segments for light chains, and the V, D and J segments for heavy chains. Thus, the additional gene segment employed during recombination to form a heavy chain variable region coding sequence significantly increases the diversity of the antigen-binding sites of heavy chains. Thus, provided non-human animals containing an engineered D_{H} region as described herein generate CDR3 diversity characterized by D_{H}-to-D_{H} recombination and have an increased amino acid length as compared to CDR3 regions of heavy chain variable regions generated by traditional VDJ recombination.

Without being bound by theory, it is thought that further diversity in heavy chain CDR3 repertoire may possible through increasing the number of junctions forming the rearranged heavy chain variable region gene sequence and/or increasing the length of the CDR3 region. One mechanism to increase the number of junctions and/or increase the length of the CDR3 region may be through the joining of a first D_{H} segment (which may be generically referred to D_{H}"A") to another D_{H} segment (which may be generically referred to as D_{H}"B") in a D_{H}-D_{H} recombination event prior to subsequent D_{H}-J_{H} and V-D_{H}J_{H} recombination events, leading to V_{H}(D_{H}A-D_{H}B)J_{H} gene sequence. In nature D_{H}-D_{H} recombination events were long thought to be prohibited by the 12/23 rule (Alt, F.W. et al., 1984, EMBO J. 3(6):1209-19; herein incorporated by reference in its entirety). However, it appears that D_{H}-D_{H} recombination events do occur at very low frequency in humans in contravention of the 12/23 rule, (1 in 800 or ∼0.125% naive B cells; see, e.g., Ollier, P.J. et al. 1985, EMBO J. 4(13B):3681-88; Milner, E C B. et al. 1986, Immunol. Today 7 :36-40; Liu, Z. et al., 1987, Nucleic Acids Res. 15(11):4688; Liu, Z. et al., 1987, Nucleic Acids Res. 15(15):6296; Meek, K.D. et al., 1989, J. Exp. Med. 169(2):519-33; Meek, K.D. et al., 1989, J. Exp. Med. 170:39-57; Baskin, B. et al. 1998, Clin. Exp. Immunol. 112:44-7; Briney, B.S. et al., 2012 Immunol. 137: 56-64; each of which are incorporated herein in its entirety by reference) and are thought to be the primary mechanism for generating the unusually long CDR3s observed in some heavy chains (Janeway's Immunobiology. 9th Edition. Kenneth Murphy, Casey Weaver. Chapter 5, 2017; incorporated in its entirety by reference). However, some potential therapeutic targets (e.g. but not limited to, viruses, cell surface receptors, type IV transmembrane proteins like GPCRs, ion channels) have masked or recessed epitopes that are inaccessible to normal antibodies but may be recognized by antibodies having long HCDR3 sequences. For example, antibodies with very long HCDR3s are often found in patients with chronic viral infections and in some cases have broadly neutralizing activity (e.g. broadly neutralizing antibodies to HIV-1 or influenza). To select antibodies capable of reaching these hidden epitopes, it would be useful to increase the frequency of heavy chains with very long HCDR3s. Without wishing to be bound by theory, one way to achieve this is to increase the frequency of heavy chain V_{H}(D_{H}A-D_{H}B)J_{H} rearrangements by engineering a D_{H} segments to comprise a 23-mer RSS and/or to increase the frequency of recombination of a D_{H} gene segment to the longer J_{H}6 gene segment. In mouse, D_{H}-J_{H} joining is thought to occur in two ordered steps: (1) primary rearrangement of the proximal DQ52 segment (referred to as D_{H}7-27 in humans) to one of the nearest J_{H} segments (J_{H}1 or J_{H}2). The strong µ0 promoter upstream of DQ52 then allows for the remaining J_{H} segments (J_{H}3 and J_{H}4) to be more accessible to recombination activating genes (RAGs); (2) secondary rearrangement of a distal D_{H} segment to one of the remaining J_{H} segments (J_{H}3 or J_{H}4). *See,* *Figure 1**, top panel.* This is consistent with the more frequent usage of downstream J_{H} segments observed in both mice (J_{H}3-J_{H}4) and humans (J_{H}4-J_{H}6) (Nitschke et al. 2001 J. Immunol. 166:2540-52, incorporated herein by reference in its entirety). Without wishing to be bound by theory, it is hypothesized that replacing D_{H}7-27 and J_{H}1-J_{H}3 (or J_{H}1-J_{H}5) with a synthetic D_{H} gene (e.g., a synthetic D_{H}3-3 segment) having a 5' 23-mer RSS and 3' 12-mer RSS would result in a high frequency of V_{H}(D_{H}-D_{H})J_{H} recombination and may occur by a three-step mechanism: (1) 23-D_{H}-12 rearrangement to J_{H}4, J_{H}5, or J_{H}6 to yield a 23(D_{H})J_{H}, (2) rearrangement of a distal D_{H} (D_{H}1-1 to D_{H}1-26) to the 23(D_{H})J_{H} to yield a 12(D_{H}-D_{H})J_{H} (V_{H} rearrangement to the 23(D_{H})J_{H} would be prohibited by the 12/23 rule), (3) V_{H} to 12(D_{H}-D_{H})J_{H} rearrangement to yield a VDDJ coding sequence encoding an immunoglobulin heavy chain variable domain. *See, e.g.,* *Figure 1**, bottom panel.* It is also hypothesized that replacing D_{H}2-2, D_{H}2-8, and D_{H}2-15 gene segments, which are long D_{H} gene segments (comprising more than 31 nucleotides encoding two cysteines that form can form a disulfide bond thought to stabilize long HCDR3 regions [see, e.g., Wang et al. 2013 Cell 153:1379-93, incorporated herein in its entirety by reference], respectively with those gene segments operably linked to a 5'end 12-mer RSS and 3'end 23-mer RSS would also result in a high frequency of V_{H}(D_{H}-D_{H})J_{H} recombination that may occur by a three-step mechanism: (1) 12:D_{H}-12 rearrangement to J_{H}4, J_{H}5, or J_{H}6 to yield a 12(D_{H})J_{H}, (2) rearrangement of a distal 12:D_{H}2-2:23, 12:D_{H}2-8:23, or 12:D_{H}2-15-23 to the 12(D_{H})J_{H} to yield a 12(D_{H}-D_{H})J_{H}, and (3) V_{H} to 12(D_{H}-D_{H})J_{H} rearrangement to yield a VDDJ coding sequence encoding an immunoglobulin heavy chain variable domain. As described herein, an engineered D_{H} region was constructed by placing a 23-mer spacer at either a 5' or 3' flanking position adjacent to one or more D_{H} segments, thereby enabling D_{H} to D_{H} recombination in a humanized immunoglobulin heavy chain variable region locus.

In some embodiments, non-human animals described herein comprise an immunoglobulin heavy chain variable region locus that demonstrates VDJ recombination that does not follow the 12/23 rule as compared to a reference non-human animal. In some embodiments, non-human animals described herein comprise one or more RSSs adjacent to or flanking one or more D_{H} segments that are modified as compared to wild-type D_{H} segments. In some embodiments, one or more D_{H} gene segments of an immunoglobulin heavy chain variable region of a non-human animal as described herein are each operably linked to either a 5' or 3' 23-mer RSS so that the frequency of D_{H}-D_{H} recombination is increased in the non-human animal as compared to a reference non-human animal. Recombination efficiency and/or frequency may be determined, in some embodiments, by usage frequencies of gene segments in a population of antibody sequences (e.g., from an individual or group of individuals; see e.g., Arnaout, R. et al., 2011, PLoS One 6(8):e22365; Glanville, .1. et al., 2011, Proc. Natl. Acad. Sci. U.S.A. 108(50):20066-71; each of which is incorporated herein in its entirety by reference). Thus, non-human animals described herein may, in some embodiments, comprise one or more D_{H} segments that are each operably linked to or flanked by a 23-mer RSS so that D_{H}-D_{H} recombination occurs at an increased frequency as compared to D_{H}-D_{H} recombination in a reference non-human animal. In some embodiments, a rodent comprising an engineered D_{H} region as described herein exhibits at least a 3-fold increased frequency of D_{H}-D_{H} recombination as compared to D_{H}-D_{H} recombination in a reference non-human animal. In some embodiments, a rodent comprising an engineered D_{H} region as described herein exhibits at least a 4-fold increased frequency of D_{H}-D_{H} recombination as compared to D_{H}-D_{H} recombination in a reference non-human animal. In some embodiments, a rodent comprising an engineered D_{H} region as described herein exhibits at least a 5-fold increased frequency of D_{H}-D_{H} recombination as compared to D_{H}-D_{H} recombination in a reference non-human animal. In some embodiments, a rodent comprising an engineered D_{H} region as described herein exhibits at least a 10-fold increased frequency of D_{H}-D_{H} recombination as compared to D_{H}-D_{H} recombination in a reference non-human animal. In some embodiments, a rodent comprising an engineered D_{H} region as described herein exhibits at least a 20-fold increased frequency of D_{H}-D_{H} recombination as compared to D_{H}-D_{H} recombination in a reference non-human animal. In some embodiments, a rodent comprising an engineered D_{H} region as described herein exhibits at least a 30-fold increased frequency of D_{H}-D_{H} recombination as compared to D_{H}-D_{H} recombination in a reference non-human animal. In some embodiments, a rodent comprising an engineered D_{H} region as described herein exhibits at least a 40-fold increased frequency of D_{H}-D_{H} recombination as compared to D_{H}-D_{H} recombination in a reference non-human animal. In some embodiments, a rodent comprising an engineered D_{H} region as described herein exhibits at least a 50-fold increased frequency of D_{H}-D_{H} recombination as compared to D_{H}-D_{H} recombination in a reference non-human animal.

### Provided in vivo systems

The present invention is based on the recognition that particular antigens are associated with low and/or poor immunogenicity and, therefore, are poor targets for antibody-based therapeutics. Indeed, many disease targets (e.g., viruses, channel proteins) have been characterized as intractable or undruggable. Thus, the present invention is based on the creation of an *in vivo* system for the development of antibodies and antibody-based therapeutics that overcome deficiencies associated with established drug discovery technologies and/or approaches. In some embodiments, the present invention provides an *in vivo* system characterized by the presence of immunoglobulin loci, in particular, humanized immunoglobulin heavy chain variable region loci, that include an engineered D_{H} region in which one or more D_{H} RSS are altered, modified or engineered from a 12-mer-D_{H}-12-mer format to a 12-mer-D_{H}-23-mer or 23-mer-D_{H}-12-mer format, thereby enabling D_{H}-to-D_{H} recombination at an increased frequency as compared D_{H}-to-D_{H} recombination observed in a reference *in vivo* system. The present disclosure specifically demonstrates the construction of a transgenic rodent whose genome comprises an immunoglobulin heavy chain variable region that includes an engineered D_{H} region, which engineered D_{H} region includes one or more D_{H} segments that are each operably linked to a 23-mer RSS positioned relative to each of the one or more D_{H} segment to allow for an increased frequency of D_{H}-D_{H} recombination. The methods described herein can be adapted to achieve any number of D_{H} segments (e.g., traditional or synthetic) that each are operably linked to a 5' or 3' 23-mer RSS for creating an engineered D_{H} region as described herein. The engineered D_{H} region, once integrated into an immunoglobulin heavy chain variable region (i.e., placed in operable linkage with V_{H} and J_{H} gene segments and/or one or more constant regions), provides for recombination of V_{H} and J_{H} gene segments with more than one D_{H} segment, e.g., to generate antibodies characterized by heavy chains having added diversity (i.e., long CDR3s, e.g., wherein at least 95% of the heavy chain CDR3 sequences are at least 14 amino acids in length) to direct binding to particular antigens. In some embodiments, such heavy chain variable regions have the capability to access difficult-to-reach epitopes in viruses, channel proteins, GPCRs, etc.

Without wishing to be bound by any particular theory, we note that data provided herein demonstrate that, in some embodiments, rodents whose genome comprises an immunoglobulin heavy chain variable locus that includes an engineered D_{H} region characterized by the inclusion of a D_{H} segment operably linked to a 5' 23-mer RSS effectively generate antibodies produced by V(DD)J recombination. We also note that data provided herein demonstrate that, in some embodiments, rodents whose genome comprises an immunoglobulin heavy chain variable locus that includes an engineered D_{H} region characterized by the inclusion of three D_{H} segments each operably linked to a 3' 23-mer RSS effectively generate antibodies produced by V(DD)J recombination. Also noted herein is that deletion of some or all five J_{H} gene segments upstream of the J_{H}6 gene segment results in preferential recombination to the J_{H}6 gene segment. Notably, the J_{H}6 gene segment comprises 63 nucleotides, e.g., 10 more nucleotides than the other J_{H}1, J_{H}2, J_{H}3, J_{H}4, and J_{H}5 gene segments, which respectively comprise 52, 53, 50, 40, and 51 nucleotides. Thus, the present disclosure, in at least some embodiments, embraces the development of an *in vivo* system for generating antibodies and/or antibody-based therapeutics to intractable disease targets, e.g., by providing rodents that generate rearranged immunoglobulin heavy chain variable region gene sequences, e.g., V_{H}D_{H}J_{H} and V_{H}(D_{H}A-D_{H}B)J_{H}, e.g., V_{H}(D_{H}A-D_{H}B)J_{H}6, sequences encoding heavy chain variable domains with CDR3 lengths of at least 20 amino acids, e.g., CDR3 lengths between 20-30 amino acids in length. In some embodiments, at least 8-10% of the rearranged immunoglobulin heavy chain variable region gene sequences, e.g., V_{H}D_{H}J_{H} and V_{H}(D_{H}A-D_{H}B)J_{H} gene sequences of a non-human animal described herein encode CDR3 regions that are at least 21 amino acids in length.

In some embodiments, described herein is a non-human animal, e.g., a rodent, e.g., a rat or a mouse, that comprises (1) in its germline genome, e.g., in a germ cell, an immunoglobulin heavy chain locus comprising an engineered D_{H} region comprising a D_{H} gene segment operably linked to a 23-mer RSS, and (2) in its somatic genome, e.g., in a B cell, a rearranged heavy chain V_{H}(D_{H}A-D_{H}B)J_{H} coding sequence, wherein the first or second D_{H} gene segment (i.e., D_{H}A or D_{H}B of the V_{H}(D_{H}A-D_{H}B)J_{H} coding sequence, respectively) comprises the D_{H} gene segment operably linked to a 23-mer RSS, or a portion thereof, e.g., wherein the first or second D_{H} gene segment has at least 9 consecutive nucleotides aligning with a D_{H} gene segment operably linked to a 23-mer RSS, and wherein each of the first and second D_{H} gene segments comprises at least 5 consecutive nucleotides aligning with a corresponding germline D_{H} gene segment irrespective of any overlap.

In some embodiments, provided non-human animals comprise an immunoglobulin heavy chain locus characterized by the presence of a plurality of human V_{H}, D_{H} and J_{H} gene segments arranged in germline configuration and operably linked to non-human immunoglobulin heavy chain constant region genes, enhancers and regulatory regions. In some embodiments, provided non-human animals comprise one or more human V_{H} gene segments, one or more human D_{H} gene segments and one or more human J_{H} gene segments operably linked to a non-human immunoglobulin heavy chain constant region.

In some embodiments, provided non-human animals comprise at least human V_{H} gene segments V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2 and V_{H}6-1.

In some embodiments, provided non-human animals comprise human D_{H} gene segments D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}5-18, D_{H}6-19, D_{H}1-20, D_{H}2-21, D_{H}3-22, D_{H}6-25 and D_{H}1-26. In some embodiments, provided non-human animals further comprise D_{H}1-14, D_{H}4-11, D_{H}4-23, D_{H}5-24, or a combination thereof. In some embodiments, provided non-human animals further comprise human D_{H}7-27.

In some embodiments, described herein is a non-human animal, e.g., a rodent, e.g., a rat or a mouse, that comprises (1) in its germline genome, e.g., in a germ cell, an immunoglobulin heavy chain locus comprising an engineered D_{H} region comprising a D_{H} gene segment operably linked to a 23-mer RSS, and (2) in its somatic genome, e.g., in a B cell, a rearranged heavy chain V_{H}(D_{H}A-D_{H}B)J_{H} coding sequence, wherein the first or second D_{H} gene segment (i.e., D_{H}A or D_{H}B of the V_{H}(D_{H}A-D_{H}B)J_{H} coding sequence, respectively) comprises the D_{H} gene segment operably linked to a 23-mer RSS, or a portion thereof, e.g., wherein the first or second D_{H} gene segment has at least 9 consecutive nucleotides aligning with a D_{H} gene segment operably linked to a 23-mer RSS, and wherein each of the first and second D_{H} gene segments comprises at least 5 consecutive nucleotides aligning with a corresponding germline D_{H} gene segment, irrespective of any overlap. In some embodiments, provided non-human animals comprise a human D_{H} gene segments operably linked to a 23-mer RSS. In some embodiments, the human D_{H} gene segment operably linked to a 23-mer RSS comprises two cysteine codons. In some embodiments, the human D_{H} gene segment operably linked to a 23-mer RSS contains at least 37 nucleotides. In some embodiments, the human D_{H} gene segment operably linked to a 23-mer RSS contains at least 19 nucleotides. In some embodiments, the human D_{H} gene segment operably linked to a 23-mer RSS contains at least 20 nucleotides. In some embodiments, the human D_{H} gene segment operably linked to a 23-mer RSS contains at least 23 nucleotides. In some embodiments, the human D_{H} gene segment operably linked to a 23-mer RSS contains at least 28 nucleotides. In some embodiments, the human D_{H} gene segment operably linked to a 23-mer RSS contains at least 31 nucleotides. In some embodiments, the human D_{H} gene segment operably linked to a 23-mer RSS contains at least 37 nucleotides.

In some embodiments, the human D_{H} gene segment operably linked to a 23-mer RSS comprises the human D_{H}1 gene segment. In some embodiments, the human D_{H} gene segment operably linked to a 23-mer RSS comprises the human D_{H}2 gene segment. In some embodiments, the human D_{H} gene segment operably linked to a 23-mer RSS comprises the human D_{H}3 gene segment. In some embodiments, the human D_{H} gene segment operably linked to a 23-mer RSS comprises the human D_{H}4 gene segment. In some embodiments, the human D_{H} gene segment operably linked to a 23-mer RSS comprises the human D_{H}5 gene segment. In some embodiments, the human D_{H} gene segment operably linked to a 23-mer RSS comprises the human D_{H}6 gene segment. In some embodiments, the human D_{H} gene segment operably linked to a 23-mer RSS comprises the human D_{H}7gene segment.

In some embodiments, the human D_{H} gene segment operably linked to a 23-mer RSS comprises the human D_{H}1-1 gene segment. In some embodiments, the human D_{H} gene segment operably linked to a 23-mer RSS comprises the human D_{H}2-2 gene segment. In some embodiments, the human D_{H} gene segment operably linked to a 23-mer RSS comprises the human D_{H}3-3 gene segment. In some embodiments, the human D_{H} gene segment operably linked to a 23-mer RSS comprises the human D_{H}4-4 gene segment. In some embodiments, the human D_{H} gene segment operably linked to a 23-mer RSS comprises the human D_{H}5-5 gene segment. In some embodiments, the human D_{H} gene segment operably linked to a 23-mer RSS comprises the human D_{H}6-6 gene segment. In some embodiments, the human D_{H} gene segment operably linked to a 23-mer RSS comprises the human D_{H}1-7 gene segment. In some embodiments, the human D_{H} gene segment operably linked to a 23-mer RSS comprises the human D_{H}2-8 gene segment. In some embodiments, the human D_{H} gene segment operably linked to a 23-mer RSS comprises the human D_{H}3-9 gene segment. In some embodiments, the human D_{H} gene segment operably linked to a 23-mer RSS comprises the human D_{H}3-10 gene segment. In some embodiments, the human D_{H} gene segment operably linked to a 23-mer RSS comprises the human D_{H}5-12 gene segment. In some embodiments, the human D_{H} gene segment operably linked to a 23-mer RSS comprises the human D_{H}6-13 gene segment. In some embodiments, the human D_{H} gene segment operably linked to a 23-mer RSS comprises the human D_{H}2-15 gene segment. In some embodiments, the human D_{H} gene segment operably linked to a 23-mer RSS comprises the human D_{H}3-16 gene segment. In some embodiments, the human D_{H} gene segment operably linked to a 23-mer RSS comprises the human D_{H}4-17 gene segment. In some embodiments, the human D_{H} gene segment operably linked to a 23-mer RSS comprises the human D_{H}5-18 gene segment. In some embodiments, the human D_{H} gene segment operably linked to a 23-mer RSS comprises the human D_{H}6-19 gene segment. In some embodiments, the human D_{H} gene segment operably linked to a 23-mer RSS comprises the human D_{H}1-20 gene segment. In some embodiments, the human D_{H} gene segment operably linked to a 23-mer RSS comprises the human D_{H}2-21 gene segment. In some embodiments, the human D_{H} gene segment operably linked to a 23-mer RSS comprises the human D_{H}3-22 gene segment. In some embodiments, the human D_{H} gene segment operably linked to a 23-mer RSS comprises the human D_{H}6-25 gene segment. In some embodiments, the human D_{H} gene segment operably linked to a 23-mer RSS comprises the human D_{H}1-26 gene segment. In some embodiments, the human D_{H} gene segment operably linked to a 23-mer RSS comprises the human D_{H}1-14 gene segment. In some embodiments, the human D_{H} gene segment operably linked to a 23-mer RSS comprises the human D_{H}4-11 gene segment. In some embodiments, the human D_{H} gene segment operably linked to a 23-mer RSS comprises the human D_{H}4-23 gene segment. In some embodiments, the human D_{H} gene segment operably linked to a 23-mer RSS comprises the human D_{H}5-24 gene segment. In some embodiments, the human D_{H} gene segment operably linked to a 23-mer RSS comprises the human D_{H}7-27 gene segment.

In some embodiments, provided non-human animals comprise the full or substantially full repertoire of human D_{H} gene segments generally arranged in the order found in an unrearranged human genomic variable locus, wherein one of the human D_{H} gene segments of the full or substantially full repertoire is replaced with a D_{H} gene segment engineered to be operably linked to a 23-mer RSS. In some embodiments one of the human D_{H} gene segments of the full or substantially full repertoire is replaced with corresponding a D_{H} gene segment engineered to be operably linked to a 23-mer RSS, e.g., a wildtype D_{H}2-2 gene segment is replaced with a D_{H}2-2 gene segment engineered to be operably linked to a 23-mer RSS. In some embodiments, one of the human D_{H} gene segments of the full or substantially full repertoire is replaced with another D_{H} gene segment engineered to be operably linked to a 23-mer RSS, e.g., a wildtype D_{H}7-27 gene segment is replaced with a D_{H}3-3 gene segment engineered to be operably linked to a 23-mer RSS. In some embodiments, the D_{R}1-1 gene segment of a full or substantially full repertoire human D_{H} gene segments generally arranged in the order found in an unrearranged human genomic variable locus is replaced with a corresponding or another D_{H} gene segment engineered to be operably linked to a 23-mer RSS, e.g., a 3' 23-mer RSS. In some embodiments, the D_{H}1-1, D_{H}2-2, D_{H}2-8, D_{H}2-15, D_{H}2-21 gene segments) or any combination thereof of a full or substantially full repertoire human D_{H} gene segments generally arranged in the order found in an unrearranged human genomic variable locus is replaced with a corresponding or another D_{H} gene segment engineered to be operably linked to a 23-mer RSS, e.g., a 3' 23-mer RSS. In some embodiments, each of the D_{H}2-2, D_{H}2-8, and D_{H}2-15 gene segments of a full or substantially full repertoire human D_{H} gene segments generally arranged in the order found in an unrearranged human genomic variable locus is replaced with a corresponding or another D_{H} gene segment engineered to be operably linked to a 23-mer RSS, e.g., a 3' 23-mer RSS.

In some embodiments, provided non-human animals comprise at least human J_{H} gene segment J_{H}6. In some embodiments, provided non-human animals comprise at least human J_{H} gene segments J_{H}4, J_{H}5 and J_{H}6. In some embodiments, provided non-human animals comprise human J_{H} gene segments J_{H}1, J_{H}2, J_{H}3, J_{H}4, J_{H}5 and J_{H}6.

In some embodiments, a non-human immunoglobulin heavy chain constant region includes one or more non-human immunoglobulin heavy chain constant region genes such as, for example, immunoglobulin M (IgM), immunoglobulin D (IgD), immunoglobulin G (IgG), immunoglobulin E (IgE) and immunoglobulin A (IgA). In some embodiments, a non-human immunoglobulin heavy chain constant region includes a rodent IgM, rodent IgD, rodent IgG3, rodent IgG1, rodent IgG2b, rodent IgG2a, rodent IgE and rodent Ig4 constant region genes. In some embodiments, said human V_{H}, D_{H} and J_{H} gene segments are operably linked to one or more non-human immunoglobulin heavy chain enhancers (i.e., enhancer sequences or enhancer regions). In some embodiments, said human V_{H}, D_{H} and J_{H} gene segments are operably linked to one or more non-human immunoglobulin heavy chain regulatory regions (or regulatory sequences). In some embodiments, said human V_{H}, D_{H} and J_{H} gene segments are operably linked to one or more non-human immunoglobulin heavy chain enhancers (or enhancer sequence) and one or more non-human immunoglobulin heavy chain regulatory regions (or regulatory sequence).

In some embodiments, provided non-human animals do not contain (or lack) an endogenous Adam6 gene. In some embodiments, provided non-human animals do not contain or lack an endogenous Adam6 gene (or Adam6-encoding sequence) in the same germline genomic position as found in a germline genome of a wild-type non-human animal of the same species. In some embodiments, provided non-human animals do not contain or lack a human Adam6 pseudogene. In some embodiments, provided non-human animals comprise insertion of at least one nucleotide sequence that encodes one or more non-human (e.g., rodent) Adam6 polypeptides. Said insertion may be outside of an engineered immunoglobulin heavy chain locus as described herein (e.g., upstream of a 5' most V_{H} gene segment), within an engineered immunoglobulin heavy chain locus or elsewhere in the germline genome of a non-human animal (e.g., a randomly introduced non-human Adam6-encoding sequence), cell or ti ssue. In some embodiments, provided non-human animals do not contain or lack a functional endogenous Adam6 pseudogene.

In some embodiments, a provided non-human animal, non-human cell or non-human tissue as described herein does not detectably express, in whole or in part, an endogenous non-human V_{H} region in an antibody molecule. In some embodiments, a provided non-human animal, non-human cell or non-human tissue as described herein does not contain (or lacks or contains a deletion of) one or more nucleotide sequences that encode, in whole or in part, an endogenous non-human V_{H} region (e.g., V_{H}, D_{H} and/or J_{H}) in an antibody molecule. In some embodiments, a provided non-human animal, non-human cell or non-human tissue as described herein has a germline genome that includes a deletion of endogenous non-human V_{H}, D_{H} and J_{H} gene segments, in whole or in part. In some embodiments, a provided non-human animal is fertile.

In some embodiments, provided non-human animals further comprise an engineered immunoglobulin κ light chain locus characterized by the presence of a plurality of human Vκ and Jκ gene segments arranged in germline configuration and inserted upstream of, and operably linked to, a non-human Cκ gene. In some embodiments, an engineered immunoglobulin κ light chain locus comprises at least human Vκ gene segments that appear in the proximal variable cluster (or proximal arm, or proximal duplication) of a human immunoglobulin κ light chain locus. In some embodiments, an engineered immunoglobulin κ light chain locus comprises at least human Vκ gene segments Vκ2-40, Vκ1-39, Vκ1-33, Vκ2-30, Vκ2-8, Vκ1-27, Vκ2-24, Vκ6-21, Vκ3-20, Vκ1-17, Vκ1-16, Vκ3-15, Vκ1-12, Vκ3-11, Vκ1-9, Vκ1-8, Vκ1-6, Vκ1-5, Vκ5-2 and Vκ4-1. In some embodiments, an engineered immunoglobulin κ light chain locus comprises at least 1, 2, 3, 4, 5, 10, or 15 human Vκ gene segments selected from Vκ2-40, Vκ1-39, Vκ1-33, Vκ2-30, Vκ2-8, Vκ1-27, Vκ2-24, Vκ6-21, Vκ3-20, Vκ1-17, VK1-16, Vκ3-15, Vκ1-12, Vκ3-11, Vκ1-9, Vκ1-8, Vκ1-6, Vκ1-5, Vκ5-2 and Vκ4-1. In some embodiments, an engineered immunoglobulin κ light chain locus comprises human Jκ gene segments Jκ1, Jκ2, Jκ3, Jκ4 and Jκ5. In some embodiments, an engineered immunoglobulin κ light chain locus comprises at least 1, 2, 3, or 4 human Jκ gene segments selected from Jκ1, Jκ2, Jκ3, Jκ4 and Jκ5.

In many embodiments, said human Vκ and Jκ gene segments are operably linked to one or more non-human immunoglobulin κ light chain enhancers (i.e., enhancer sequences or enhancer regions). In some embodiments, said human Vκ and Jκ gene segments are operably linked to a murine Igκ light chain intronic enhancer region (Igκ Ei or Eiκ). In many embodiments, said human Vκ and Jκ gene segments are operably linked to one or more non-human immunoglobulin κ light chain regulatory regions (or regulatory sequences). In some embodiments, said human Vκ and Jκ gene segments are operably linked to a murine Igκ light chain 3' enhancer region (Igκ 3'E or 3'Eκ). In some embodiments, said human Vκ and Jκ gene segments are operably linked to a murine Eiκ and operably linked to a murine 3'Eκ. In some embodiments, said human Vκ and Jκ gene segments are operably linked to one or more non-human immunoglobulin κ light chain enhancers (or enhancer sequences or enhancer regions) and one or more non-human immunoglobulin κ light chain regulatory regions (or regulatory sequences). In some embodiments, an engineered immunoglobulin κ light chain locus contains the same non-human immunoglobulin κ light chain enhancer regions (or enhancer sequences) that appear in a wild-type immunoglobulin κ light chain locus. In some embodiments, an engineered immunoglobulin κ light chain locus contains non-human Igκ light chain enhancer regions (or enhancer sequences) that appear in a wild-type immunoglobulin κ light chain locus of a different species (e.g., a different rodent species).

In some embodiments, an engineered immunoglobulin κ light chain locus as described herein does not contain (i.e., lacks) a human VpreB gene (or human VpreB gene-encoding sequence).

In some embodiments, a non-human Cκ gene of an engineered immunoglobulin κ light chain locus includes a rodent Cκ gene such as, for example, a mouse Cκ gene or a rat Cκ gene. In some embodiments, a non-human Cκ gene of an engineered immunoglobulin κ light chain locus is or comprises a mouse Cκ gene from a genetic background that includes a 129 strain, a BALB/c strain, a C57BL/6 strain, a mixed 129xC57BL/6 strain or combinations thereof.

In some embodiments, a provided non-human animal, non-human cell or non-human tissue as described herein does not detectably express, in whole or in part, an endogenous non-human Vκ region in an antibody molecule. In some embodiments, a provided non-human animal, non-human cell or non-human tissue as described herein does not contain (or lacks or contains a deletion of) one or more nucleotide sequences that encode, in whole or in part, an endogenous non-human Vκ region in an antibody molecule. In some embodiments, a provided non-human animal, non-human cell or non-human tissue as described herein has a germline genome that includes a deletion of endogenous non-human Vκ and Jκ gene segments, in whole or in part.

In some embodiments, provided non-human animals further comprise a wild-type or inactivated (e.g., by gene targeting) immunoglobulin λ light chain locus.

In some embodiments, a provided non-human animal, non-human cell or non-human tissue as described herein does not detectably express, in whole or in part, an endogenous non-human Vλ region in an antibody molecule. In some embodiments, a provided non-human animal, non-human cell or non-human tissue as described herein does not contain (or lacks, or contains a deletion of) one or more nucleotide sequences that encode, in whole or in part, an endogenous non-human Vλ region in an antibody molecule. In some embodiments, a provided non-human animal, non-human cell or non-human tissue as described herein has a germline genome that includes a deletion of endogenous non-human Vλ and Jλ gene segments, in whole or in part. In some embodiments, a provided non-human animal, non-human cell or non-human tissue as described herein as a germline genome that includes a deletion of endogenous non-human Vλ, Jλ and Cλ gene segments, in whole or in part.

Guidance for the creation of targeting vectors, non-human cells and animals harboring such engineered immunoglobulin loci can be found in U.S. Patent Nos. 8,642,835, 8,697,940, 9,006,511, 9,012,717, 9,029,628, 9,035,128, 9,066,502, 9,150,662 and 9,163,092, which are incorporated by reference in their entireties herein. Persons skilled in the art are aware of a variety of technologies, known in the art, for accomplishing such genetic engineering and/or manipulation of non-human (e.g., mammalian) genomes or for otherwise preparing, providing, or manufacturing such sequences for introducing into the germline genome of non-human animals.

### DNA constructs

Typically, a polynucleotide molecule containing immunoglobulin gene segments as described herein, in particular, one or more D_{H} segments that each are operably linked to a 5' or 3' 23-mer RSS is inserted into a vector, preferably a DNA vector, in order to replicate the polynucleotide molecule in a suitable host cell.

Due to their size, D_{H} segments can be cloned directly from genomic sources available from commercial suppliers or designed *in silico* based on published sequences available from GenBank. Alternatively, bacterial artificial chromosome (BAC) libraries can provide immunoglobulin sequences. BAC libraries contain an average insert size of 100-150kb and are capable of harboring inserts as large as 300kb (Shizuya, et al., 1992, Proc. Natl. Acad. Sci., USA 89:8794-8797; Swiatek, et al., 1993, Genes and Development 7:2071-2084; Kim, et al., 1996, Genomics 34 213-218; herein incorporated by reference in their entireties). For example, human and mouse genomic BAC libraries have been constructed and are commercially available (e.g., Invitrogen, Carlsbad Calif.). Genomic BAC libraries can also serve as a source of immunoglobulin sequences as well as transcriptional control regions.

Alternatively, immunoglobulin sequences may be isolated, cloned and/or transferred from yeast artificial chromosomes (YACs). An entire immunoglobulin locus, or substantial portion thereof, can be cloned and contained within one or a few YACs. If multiple YACs are employed and contain regions of overlapping homology, they can be recombined within yeast host strains to produce a single construct representing an entire locus. YAC arms can be additionally modified with mammalian selection cassettes by retrofitting to assist in introducing the constructs into embryonic stems cells or embryos by methods known in the art and/or described herein.

DNA constructs can be prepared using methods known in the art. For example, a DNA construct can be prepared as part of a larger plasmid. Such preparation allows the cloning and selection of the correct constructions in an efficient manner as is known in the art. DNA fragments containing one or more D_{H} segments that are each operably linked to a 5' or 3' 23-mer RSS as described herein can be located between convenient restriction sites on the plasmid so that they can be easily isolated from the remaining plasmid sequences for incorporation into the desired animal.

In some embodiments, methods employed in preparation of plasmids and transformation of host organisms are known in the art. For other suitable expression systems for both prokaryotic and eukaryotic cells, as well as general recombinant procedures, see Molecular Cloning: A Laboratory Manual, 2nd Ed., ed. by Sambrook, J. et al., Cold Spring Harbor Laboratory Press: 1989; incorporated herein in its entirety by reference.

### Production of Non-Human Animals

Non-human animals are provided that express antibodies having heavy chain CDR3 diversity characterized by long amino acid length resulting from integration of 23-mer RSSs adjacent to one or more D_{H} segments that allow for D_{H}-to-D_{H} recombination within an immunoglobulin heavy chain variable region in the genome of the non-human animal. Suitable examples described herein include rodents, in particular, mice. One or more D_{H} segments, in many embodiments, include heterologous D_{H} segments (e.g., human D_{H} segments). Non-human animals, embryos, cells and targeting constructs for making non-human animals, non-human embryos, and cells containing said D_{H} segments that are capable of D_{H}-D_{H} recombination at an increased frequency as compared to wild-type or reference non-human animals are also provided.

In some embodiments, one or more D_{H} segments are modified to be adjacent to (or operably linked to) a 5' or 3' 23-mer RSS within a diversity cluster (i.e., a D_{H} region) of an immunoglobulin heavy chain variable region in the genome of a non-human animal. In some embodiments, a D_{H} region (or portion thereof) of an immunoglobulin heavy chain variable region is not deleted (i.e., intact). In some embodiments, a D_{H} region (or portion thereof) of an immunoglobulin heavy chain variable region is altered, disrupted, deleted, engineered or replaced with one or more D_{H} segments that are each operably linked to a 5' or 3' 23-mer RSS. In some embodiments, all or substantially all of a D_{H} region is replaced with one or more synthetic D_{H} segments that are each operably linked to a 5' or 3' 23-mer RSS; in some embodiments, one or more traditional D_{H} gene segments are not deleted or replaced in a D_{H} region of an immunoglobulin heavy chain variable region. In some embodiments, a D_{H} region contains both traditional D_{H} segments (i.e., one or more D_{H} segments each operably linked to a 5' 12-mer RSS and a 3' 12-mer RSS) and engineered D_{H} gene segments (i.e., one or more D_{H} segments each operably linked to a 5' or 3' 23-mer RSS. In some embodiments, a D_{H} region as described herein is a synthetic D_{H} region. In some embodiments, a D_{H} region is a human D_{H} region. In some embodiments, a D_{H} region is a murine D_{H} region. In some embodiments, an engineered D_{H} region (or portion thereof) as described herein is inserted into an immunoglobulin heavy chain variable region so that said engineered D_{H} region (or portion thereof) is operably linked with one or more V_{H} gene segments and/or one or more J_{H} gene segments. In some embodiments, said engineered D_{H} region is inserted into one of the two copies of an immunoglobulin heavy chain variable region, giving rise to a non-human animal that is heterozygous with respect to the said engineered D_{H} region. In some embodiments, a non-human animal is provided that is homozygous for an engineered D_{H} region. In some embodiments, a non-human animal is provided that is heterozygous for an engineered D_{H} region.

In some embodiments, a non-human animal as described herein contains a randomly integrated human immunoglobulin heavy chain variable region that includes a D_{H} region that contains one or more D_{H} segments that are each operably linked to a 5' or 3' 23-mer RSS within its genome. Thus, such non-human animals can be described as having a human immunoglobulin heavy chain transgene containing an engineered D_{H} region. An engineered D_{H} region can be detected using a variety of methods including, for example, PCR, Western blot, Southern blot, restriction fragment length polymorphism (RFLP), or a gain of allele (GOA) or loss of allele (LOA) assay. In some such embodiments, a non-human animal as described herein is heterozygous with respect to an engineered D_{H} region as described herein. In some such embodiments, a non-human animal as described herein is homozygous with respect to an engineered D_{H} region as described herein. In some such embodiments, a non-human animal as described herein is hemizygous with respect to an engineered D_{H} region as described herein. In some such embodiments, a non-human animal as described herein contains one or more copies of an engineered D_{H} region as described herein.

In some embodiments, an engineered D_{H} region of a non-human animal as described herein includes at least one D_{H} segment that is associated with (or operably linked to) a 5' 23-mer RSS. In some embodiments, an engineered D_{H} region of a non-human animal as described herein includes at least one D_{H} segment that is associated with (or operably linked to) a 3' 23-mer RSS. In some embodiments, an engineered D_{H} region of a non-human animal as described herein includes at least a human D_{H}3-3 segment that is associated with (or operably linked to) a 5' 23-mer RSS. In some embodiments, an engineered D_{H} region of a non-human animal as described herein includes at least a human D_{H}2 segment that is associated with (or operably linked to) a 3' 23-mer RSS, wherein the human D_{H}2 segment is selected from the group consisting of human D_{H}2-2, human D_{H}2-8, human D_{H}2-15 and human D_{H}2-21.

In some embodiments, an engineered D_{H} region of a non-human animal as described herein includes more than one D_{H} segment that are each associated with (or operably linked to) a 5' 23-mer RSS. In some embodiments, an engineered D_{H} region of a non-human animal as described herein includes more than one D_{H} segment that are each associated with (or operably linked to) a 3' 23-mer RSS. In some embodiments, an engineered D_{H} region of a non-human animal as described herein includes human D_{H}2-2, human D_{H}2-8, and human D_{H}2-15 segments that are each associated with (or operably linked to) a 3' 23-mer RSS.

Compositions and methods for making non-human animals whose genome comprises an immunoglobulin heavy chain variable region that includes an engineered D_{H} region, wherein the engineered D_{H} region includes one or more D_{H} segments that are each associated with (or operably linked to) a 5' or 3' 23-mer RSS, are provided, including compositions and methods for making non-human animals that express antibodies comprising a heavy chain variable region that includes a CDR3 region having an amino acid sequence encoded by more than one D_{H} segment from an immunoglobulin heavy chain locus that contains human V_{H} and J_{H} gene segments operably linked to one or more non-human heavy chain constant region genes. In some embodiments, compositions and methods for making non-human animals that express such antibodies under the control of an endogenous enhancer(s) and/or an endogenous regulatory sequence(s) are also provided. In some embodiments, compositions and methods for making non-human animals that express such antibodies under the control of a heterologous enhancer(s) and/or a heterologous regulatory sequence(s) are also provided. Methods include inserting one or more D_{H} segments and other sequences that that allow for D_{H}-D_{H} recombination at an increased frequency as compared to wild-type D_{H} segments, in the genome of a non-human animal so that an antibody is expressed which includes immunoglobulin heavy chains resulting from V(DD)J recombination.

In some embodiments, methods include inserting DNA that includes a D_{H} gene segment with a 5' 23-mer RSS and a 3' 12-mer RSS operably linked to one or more J_{H} gene segments. As described herein, the D_{H} gene segment is positioned downstream of a µ0 promoter sequence. In some embodiments, methods include inserting a D_{H} segment with a 5' 23-mer RSS and a 3' 12-mer RSS in a position relative to a µ0 promoter sequence so that said J_{H} gene segment is accessible to RAG genes (e.g., RAG-1 and/or RAG-2) during recombination. Genetic material that includes the D_{H} segment and flanking RSSs described above may be inserted into the genome of a non-human animal, thereby creating a non-human animal having an engineered D_{H} region that contains said D_{H} segment and necessary RSSs to allow for recombination with an adjacent D_{H} gene segment and V_{H} and J_{H} gene segments.

In some embodiments, methods include inserting DNA that includes three D_{H} gene segments each associated with a 5' 12-mer RSS and a 3' 23-mer RSS operably linked to six J_{H} gene segments. As described herein, the D_{H} gene segments are positioned amongst a plurality of D_{H} gene segments that are each associated with 5' and 3' 12-mer RSS. In some embodiments, methods include inserting D_{H}2-2, D_{H}2-8 and D_{H}2-15 gene segments each associated with a 5' 12-mer RSS and a 3' 23-mer RSS in a diversity cluster with a plurality of other D_{H} gene segments each associated with traditional or wild-type RSS. Genetic material that includes the D_{H} gene segments and flanking RSSs described above may be inserted into the genome of a non-human animal, thereby creating a non-human animal having an engineered D_{H} region that contains said D_{H} segments and necessary RSSs to allow for recombination with adjacent D_{H} gene segments and V_{H} and J_{H} gene segments.

Where appropriate, sequences corresponding to (or encoding) D_{H} segments may be modified to include codons that are optimized for expression in the non-human animal (e.g., see U.S. Patent Nos. 5,670,356 and 5,874,304; each of which is incorporated by reference in its entirety). Codon optimized sequences are synthetic sequences, and preferably encode the identical polypeptide (or a biologically active fragment of a full-length polypeptide which has substantially the same activity as the full-length polypeptide) encoded by the non-codon optimized parent polynucleotide. In some embodiments, sequences corresponding to (or encoding) D_{H} segments may include an altered sequence to optimize codon usage for a particular cell type (e.g., a rodent cell). For example, the codons of sequences corresponding to D_{H} segments to be inserted into the genome of a non-human animal (e.g., a rodent) may be optimized for expression in a cell of the non-human animal. Such a sequence may be described as a codon-optimized sequence.

Insertion of D_{H} segments operably linked to a 5' or 3' 23-mer RSS into a D_{H} region so that said D_{H} segments are operably linked to V_{H} and J_{H} gene segments (e.g., a plurality of V_{H} and J_{H} gene segments) employs a relatively minimal modification of the genome and results in expression of antibodies comprising heavy chains characterized by CDR3s having longer amino acid lengths.

Methods for generating transgenic non-human animals, including knockouts and knock-ins, are well known in the art (see, e.g., Gene Targeting: A Practical Approach, Joyner, ed., Oxford University Press, Inc. (2000); incorporated herein in its entirety by reference). For example, generation of transgenic rodents may optionally involve disruption of the genetic loci of one or more endogenous rodent genes (or gene segments) and introduction of one or more D_{H} segments each operably linked to a 23-mer RSS into the rodent genome, in some embodiments, at the same location as an endogenous rodent gene (or gene segments). In some embodiments, one or more D_{H} segments each operably linked to a 23-mer RSS are introduced into a D_{H} region of a randomly inserted immunoglobulin heavy chain locus in the genome of a rodent. In some embodiments, one or more D_{H} segments each operably linked to a 23-mer RSS are introduced into a D_{H} region of an endogenous immunoglobulin heavy chain locus in the genome of a rodent; in some embodiments, an endogenous immunoglobulin heavy chain locus is altered, modified, or engineered to contain human gene segments (e.g., V and/or J) operably linked to one or more constant region genes (e.g., human or murine).

A schematic illustration (not to scale) of representative targeting vectors for constructing an engineered D_{H} region and integration into rodent embryonic stem (ES) cells to create a rodent whose genome comprises an immunoglobulin heavy chain variable region that includes an engineered diversity cluster (i.e., D_{H} region), which diversity cluster includes one or more D_{H} segments each operably linked to a 5' or 3' 23-mer RSS is provided in Figure 2. Exemplary strategies and methods for inserting such vectors into immunoglobulin heavy chain variable regions in the genome of rodent ES cells are provided in Figures 3-8. In each of Figures 2-8, NotI restriction enzyme recognition sites are indicated were appropriate, names and approximate locations (small dash) of various primer/probe sets (see Table 4) are indicated for various alleles shown (not to scale), and unless otherwise noted, open symbols and lines represent human sequence, while closed symbols and dark lines represent mouse sequence. The following abbreviations are used for each of the figures, spec: spectinomycin resistance gene; neo: neomycin resistance gene; hyg: hygromycin resistance gene; lp: *lox*P site-specific recombination recognition site; Ei: murine heavy chain intronic enhancer; IgM: murine immunoglobulin M constant region gene; L: *loxP* site sequence; Frt: Flippase recognition target sequence; µ0 pro: µ0 promoter sequence.

As illustrated in Figure 2, DNA fragments containing a plurality of D_{H} segments with one D_{H} segments operably linked to a 5' 23-mer RSS (Figure 2, top and middle) or with three D_{H} segments each operably linked to a 3' 23-mer RSS (Figure 2, bottom) are made using VELOCIGENE^{®} technology (see, e.g., U.S. Patent No. 6,586,251 and Valenzuela et al., 2003, Nature Biotech. 21(6):652-659; herein incorporated by reference in their entireties) and molecular biology techniques known in the art. In ***Figure 2****,* unless otherwise noted, open symbols and lines represent human sequence, while closed symbols and dark lines represent mouse sequence. The not-to-scale relative locations of the human V_{H}6-1, D_{H}2-2, D_{H}2-8, D_{H}2-15, D_{H}3-3, and J_{H}1, J_{H}2, J_{H}3, J_{H}4, J_{H}5 and J_{H}6 gene segments are depicted, when present and any gene segment remaining unnamed is a D_{H} gene segment. Generally, the D_{H} region as depicted with the hash marks comprises the full repertoire of unrearranged human D_{H} gene segments (see, e.g., www.imgt.org/IMGTrepertoire/index.php?section=LocusGenes&repertoire=locus&species=human &group=IGH; incorporated herein in its entirety by reference) with the following exceptions: the top two targeting vectors lacks the last D_{H}7-27 gene, which is replaced by a sequence comprising the D_{H}3-3 gene segment flanked by a 5'-end 23-mer RSS and a 3'-end 12-mer RSS (depicted as an open arrow); the bottom targeting vector lacks the unrearranged human D_{H}2-2 gene segment, the unrearranged human D_{H}2-8 gene segment, and D_{H}2-15 gene segments, which are respectively replaced by the D_{H}2-2 gene segment flanked by a 5'-end 12-mer RSS and a 3'-end 23-mer RSS, the D_{H}2-8 gene segment flanked by a 5'-end 12-mer RSS and a 3'-end 23-mer RSS, and the D_{H}2-15 gene segment flanked by a 5'-end 12-mer RSS and a 3'-end 23-mer RSS (each of which engineered D_{H}2-2, D_{H}2-8, and D_{H}2-15 gene segment is depicted as an open arrow). Unfilled vertical rectangles represent locations of unique artificial 40-mers homologous to sequence primers and probes placed in the 12:D_{H}2-2:23|12:D_{H}2-8:23|12:D_{H}2-15:23 targeting vector to help insure correct modification of the targeting vector/ES cell. Sequences for the unique artificial 40-mers denoted as "1," "2," "10," "16," "8," and "18" are set forth as SEQ ID NO:73, SEQ ID NO:74, SEQ ID NO:75, SEQ ID NO:76, SEQ ID NO:77, and SEQ ID NO:78, respectively.

DNA fragments are assembled with homology arms for precise targeted insertion into a humanized immunoglobulin heavy chain variable region locus (Figures 3, 5, 7). Selection cassette (e.g., neomycin) flanked by site-specific recombination recognition sites (e.g., *loxP*) are included in the targeting vectors for facilitate screening of proper insertion in ES cells clones and may be removed by transient expression of a recombinase (e.g., Cre) in positive ES cell clones (Figures 4, 6, 8). The DNA fragments include the necessary sequences for proper assembly (i.e., recombination), transcription and expression of heavy chain variable regions once integrated into an immunoglobulin heavy chain locus. The targeting vectors are designed so that the engineered D_{H} region is flanked 5' by human V_{H} genomic DNA and 3' by human J_{H} genomic DNA and non-human (e.g., rodent) genomic heavy chain constant region DNA (e.g., intronic enhancer and a IgM constant region gene). The final targeting vectors for incorporation into the genome of a non-human cell (e.g., a rodent embryonic stem cell) contain V_{H} genomic DNA (e.g., containing one or more V_{H} gene segments), an engineered D_{H} region, 3' J_{H} genomic DNA, and non-human (e.g., rodent) genomic heavy chain constant region DNA, all of which are operably linked to allow for recombination between V_{H} gene segments, the engineered D_{H} region and a J_{H} gene segment once integrated into the genome of a non-human animal. Once assembled, the targeting vectors are linearized and electroporated into rodent ES cells.

The targeting vectors are introduced into rodent (e.g., mouse) embryonic stem cells so that the sequence contained in the targeting vector (i.e., an engineered D_{H} region) results in the capacity of a non-human cell or non-human animal (e.g., a mouse) that expresses antibodies that contain CDR3s having amino acids encoded by more than one D_{H} segment.

As described herein, transgenic rodents are generated where an engineered D_{H} region has been introduced into an immunoglobulin heavy chain locus of the rodent genome (e.g., an immunoglobulin heavy chain locus engineered to contain human variable region gene segments, which may be an endogenous immunoglobulin heavy chain locus so engineered).

Immunoglobulin loci comprising human variable region gene segments are known in the art and can be found, for example, in U. S. Pat. Nos. 5,633,425; 5,770,429; 5,814,318; 6,075,181; 6,114,598; 6,150,584; 6,998,514; 7,795,494; 7,910,798; 8,232,449; 8,502,018; 8,697,940; 8,703,485; 8,754,287; 8,791,323; 8,809,051; 8,907,157; 9,035,128; 9,145,588;9,206,263; 9,447,177; 9,551,124; 9,580,491 and 9,475,559, each of which is hereby incorporated by reference in its entirety, as well as in U.S. Pat. Pub. Nos. 20100146647, 20110195454, 20130167256, 20130219535, 20130326647, 20130096287, and 2015/0113668, each of which is hereby incorporated by reference in its entirety, and in PCT Pub. Nos. WO2007117410, WO2008151081, WO2009157771, WO2010039900, WO2011004192, WO2011123708 and WO2014093908, each of which are hereby incorporated by reference in its entirety.

In some embodiments, non-human animals as disclosed herein comprise exogenous fully human immunoglobulin transgenes comprising an engineered D_{H} region, which are able to rearrange in precursor B cells in mice (Alt et al., 1985, Immunoglobulin genes in transgenic mice, Trends Genet 1:231-236; incorporated herein in its entirety by reference). In these embodiments, fully human immunoglobulin transgenes comprising an engineered D_{H} region may be (randomly) inserted and endogenous immunoglobulin genes may also be knocked-out (Green et al., 1994, Antigen-specific human monoclonal antibodies from mice engineered with human Ig heavy and light chain YACs, Nat Genet 7:13-21; Lonberg et al., 1994, Antigen-specific human antibodies from mice comprising four distinct genetic modifications, Nature 368:856-859; Jakobovits et al., 2007, From XenoMouse technology to panitumumab, the first fully human antibody product from transgenic mice, Nat Biotechnol 25:1134-1143; each of which publications is incorporated by reference in its entirety) e.g., wherein endogenous immunoglobulin heavy chain and κ light chain loci are inactivated, e.g., by targeted deletion of small but critical portions of each endogenous locus, followed by introduction of human immunoglobulin gene loci as randomly integrated large transgenes, or minichromosomes (Tomizuka et al., 2000, Double trans-chromosomic mice: maintenance of two individual human chromosome fragments containing Ig heavy and kappa loci and expression of fully human antibodies, PNAS USA 97:722-727; incorporated by reference in its entirety).

In some embodiments, human or humanized immunoglobulin heavy and light chain loci comprising an engineered D_{H} region are at endogenous immunoglobulin heavy and light chain loci, respectively. A method for a large in situ genetic replacement of the mouse germline immunoglobulin variable gene loci with human germline immunoglobulin variable gene loci while maintaining the ability of the mice to generate offspring has been previously described. *See, e.g.,* U.S. Patent Nos. 6,596,541 and 8,697,940, each of which is incorporated in its entirety by reference. Specifically, the precise replacement of six megabases of both the mouse heavy chain and κ light chain immunoglobulin variable gene loci with their human counterparts while leaving the mouse constant regions intact is described. As a result, mice have been created that have a precise replacement of their entire germline immunoglobulin variable repertoire with equivalent human germline immunoglobulin variable sequences, while maintaining mouse constant regions. The human variable regions are linked to mouse constant regions to form chimeric human-mouse immunoglobulin loci that rearrange and express at physiologically appropriate levels. The antibodies expressed are "reverse chimeras," i.e., they comprise human variable region sequences and mouse constant region sequences. These mice having humanized immunoglobulin variable regions that express antibodies having human or humanized variable regions and mouse constant regions are called VELOCIMMUNE^{®} mice.

VELOCIMMUNE^{®} humanized mice exhibit a fully functional humoral immune system that is essentially indistinguishable from that of wild-type mice. They display normal cell populations at all stages of B cell development. They exhibit normal lymphoid organ morphology. Antibody sequences of VELOCIMMUNE^{®} mice exhibit normal V(D)J rearrangement and normal somatic hypermutation frequencies. Antibody populations in these mice reflect isotype distributions that result from normal class switching (e.g., normal isotype cis-switching). Immunizing VELOCIMMUNE^{®} mice results in robust humoral immune responses that generate large, diverse antibody repertoires having human immunoglobulin variable domains suitable for use as therapeutic candidates. This platform provides a plentiful source of naturally affinity-matured human immunoglobulin variable region sequences for making pharmaceutically acceptable antibodies and other antigen-binding proteins. It has also been shown that replacement of even a single endogenous V_{H} gene segment with a human V_{H} gene segment can result in an immune response comprising humanized immunoglobulin variable domain. *See, e.g.,* Tien et al. (2016) Cell 166:1471-84; incorporated herein in its entirety by reference. It is the precise replacement of mouse immunoglobulin variable sequences with human immunoglobulin variable sequences such that the human immunoglobulin variable sequences are operably linked with endogenous non-human constant region gene sequence(s) in a reverse chimeric manner that allows for making VELOCIMMUNE^{®} mice.

Mice modified in a reverse chimeric manner include mice modified to comprise at an endogenous immunoglobulin locus a human(ized) variable region (e.g., comprising (D), J, and one or more human V gene segments) operably linked to an endogenous constant region, e.g.,
(a) at an endogenous heavy chain locus:
   (i) an unrearranged human(ized) immunoglobulin heavy chain variable region in operable linkage to an endogenous heavy chain constant region, wherein the unrearranged human(ized) immunoglobulin heavy chain variable region comprises a plurality of unrearranged human heavy chain variable region V_{H} gene segments (e.g., all functional human unrearranged human V_{H} gene segments), one or more unrearranged immunoglobulin heavy chain D_{H} gene segments and one or more unrearranged immunoglobulin heavy chain J_{H} gene segments,
      optionally wherein the one or more unrearranged immunoglobulin heavy chain D_{H} gene segments and one or more unrearranged immunoglobulin heavy chain J_{H} gene segments are one or more unrearranged human immunoglobulin heavy chain D_{H} gene segments (e.g., all functional human D_{H} gene segments) and/or one or more unrearranged human immunoglobulin heavy chain J_{H} gene segments (e.g., all functional human J_{H} gene segments);
   (ii) a restricted unrearranged human(ized) heavy chain variable region in operable linkage to an endogenous heavy chain constant region, wherein the restricted unrearranged human(ized) heavy chain variable region consists essentially of a single unrearranged human heavy chain variable region V_{H} gene segment operably linked with one or more unrearranged immunoglobulin heavy chain D_{H} gene segments and one or more unrearranged immunoglobulin heavy chain J_{H} gene segments, optionally wherein the one or more unrearranged immunoglobulin heavy chain D_{H} gene segments and one or more unrearranged immunoglobulin heavy chain J_{H} gene segments are one or more unrearranged human immunoglobulin heavy chain D_{H} gene segments and/or one or more unrearranged human immunoglobulin heavy chain J_{H} gene segments, respectively;
   (iii) a histidine modified unrearranged human(ized) heavy chain variable region in operable linkage to an endogenous heavy chain constant region, wherein the histidine modified unrearranged human(ized) heavy chain variable region comprises an unrearranged immunoglobulin heavy chain variable gene sequence comprising in a complementarity determining region 3 (CDR3) encoding sequence a substitution of at least one non-histidine codon with a histidine codon or an insertion of at least one histidine codon; or
   (iv) a heavy chain only immunoglobulin encoding sequence comprising an unrearranged human(ized) heavy chain variable region in operable linkage to an endogenous heavy chain constant region, wherein the endogenous heavy chain constant region comprises (1) an intact endogenous IgM gene that encodes an IgM isotype that associates with light chain and (2) a non-IgM gene, e.g., an IgG gene, lacking a sequence that encodes a functional CH1 domain, wherein the non-IgM gene encodes a non-IgM isotype lacking a CH1 domain capable of covalently associating with a light chain constant domain;
   and/or
(b) at an endogenous light chain locus:
   (i) an unrearranged human(ized) immunoglobulin light chain variable region in operable linkage to an endogenous light chain constant region, wherein the unrearranged human(ized) immunoglobulin light chain variable region comprises a plurality of unrearranged human light chain variable region V_{L} gene segments (e.g., all functional human unrearranged human V_{L} gene segments) and one or more unrearranged immunoglobulin light chain J_{L} gene segments,
      optionally wherein the one or more unrearranged immunoglobulin light chain J_{L} gene segments are one or more unrearranged human immunoglobulin light chain J_{L} gene segments (e.g., all functional human J_{H}L gene segments),
      optionally wherein the endogenous immunoglobulin light chain locus is an endogenous immunoglobulin light chain kappa (κ) locus, the unrearranged human(ized) immunoglobulin light chain variable region comprises human variable κ (V_{κ}) and joining κ (J_{κ}) gene segments, and wherein the endogenous light chain constant region is an endogenous κ chain constant region sequence and/or wherein the endogenous immunoglobulin light chain locus is an endogenous immunoglobulin light chain lambda (λ), the unrearranged human(ized) immunoglobulin light chain variable region comprises human variable λ (V_{λ}) and joining λ (J_{λ}) gene segments, and the endogenous light chain constant region is an endogenous λ chain constant region sequence, optionally wherein the endogenous immunoglobulin light chain λ locus comprises (a) one or more human V_{λ} gene segments, (b) one or more human J_{λ} gene segments, and (c) one or more human C_{λ} gene segments, wherein (a) and (b) are operably linked to (c) and a rodent immunoglobulin light chain constant (C_{λ}) gene segment, and wherein the endogenous immunoglobulin λ light chain locus further comprises: one or more rodent immunoglobulin λ light chain enhancers (Eλ), and one or more human immunoglobulin λ light chain enhancers (Eλ), optionally comprising three human Eλs;
   (ii) a common light chain encoding sequence comprising a rearranged human(ized) light chain variable region sequence in operable linkage to an endogenous light chain constant region, wherein the rearranged human(ized) light chain variable region sequence comprises a human light chain variable region V_{L} gene segment rearranged with an immunoglobulin light chain J_{L} gene segment;
   (iii) a restricted unrearranged human(ized) light chain variable region in operable linkage to an endogenous light chain constant region, wherein the restricted unrearranged human(ized) light chain variable region comprises no more than two unrearranged human immunoglobulin light chain variable (V_{L}) gene segments operably linked to one or more unrearranged human immunoglobulin light chain joining (J_{L}) gene segments;
   (iv) a histidine modified unrearranged human(ized) light chain variable region in operable linkage to an endogenous light chain constant region, wherein the histidine modified unrearranged human(ized) light chain variable region comprises an unrearranged human(ized) immunoglobulin light chain variable gene sequence comprising in a complementarity determining region 3 (CDR3) encoding sequence a substitution of at least one non-histidine codon with a histidine codon or an insertion of at least one histidine codon; or
   (v) a histidine modified rearranged human(ized) light chain variable region in operable linkage to an endogenous light chain constant region, wherein the histidine modified rearranged human(ized) light chain variable region comprises a rearranged human(ized) immunoglobulin light chain variable gene sequence comprising in a complementarity determining region 3 (CDR3) encoding sequence a substitution of at least one non-histidine codon with a histidine codon or an insertion of at least one histidine codon,

optionally wherein the mouse further comprises
   (i) a human(ized) immunoglobulin heavy chain locus comprising a functional ADAM6 gene such that the mouse exhibits wildtype fertility of the non-human animal;
      and/or
   (ii) an exogenous terminal deoxynucleotidyl transferase (TdT) gene for increased antigen receptor diversity, optionally such that at least 10% of the rearranged variable region genes comprise non-template additions,
which mice have been previously described. *See, e.g.,* U.S. Patent Nos. 8,697,940; 8,754,287; 9,204,624; 9,334,334; 9,801,362; 9,332,742; and 9,516,868; U.S. Patent Publications 20110195454, 20120021409, 20120192300, 20130045402; 20150289489; 20180125043; 20180244804; PCT Publication No. WO2019/113065, WO2017210586, and WO2011163314; Lee et al. (2014) Nature Biotechnology 32:356, each of which is incorporated herein in its entirety by reference.

In some embodiments, the present invention includes a genetically modified non-human animal whose genome, e.g., germline genome, comprises:
an endogenous immunoglobulin locus comprising an immunoglobulin heavy chain variable region comprising a human V_{H} gene segment, a human engineered D_{H} gene region of the invention, and a human J_{H} gene segment, wherein the immunoglobulin heavy chain variable region is operably linked to a constant region, and/or
an endogenous chain locus comprising an immunoglobulin light chain variable region comprising a human VL gene segment and a human JL gene segments, wherein the immunoglobulin light chain variable region is operably linked to a constant region.

In some embodiments, a non-human animal, e.g., a rodent, e.g., a rat or a mouse, comprises in its genome a replacement of one or more endogenous V_{H}, D_{H}, and J_{H} segments at an endogenous immunoglobulin heavy chain locus with one or more human V_{H}, D_{H}, and J_{H} segments, wherein the one or more human V_{H}, D_{H}, and J_{H} segments comprises a human D_{H} gene segment operably linked to a 23-mer RSS and are operably linked to an endogenous immunoglobulin heavy chain gene; and optionally an unrearranged or rearranged human V_{L} and human J_{L} segment operably linked to a non-human, e.g., rodent, e.g., a mouse or rat, or human immunoglobulin light chain constant (C_{L}) region gene, e.g., at an endogenous non-human light chain locus.

In certain embodiments, the genetically modified non-human animals comprise in their genome, e.g., germline genome, an immunoglobulin locus (exogenous or endogenous) containing an immunoglobulin variable region comprising one or more unrearranged human immunoglobulin variable region gene segments including an engineered D_{H} region and an immunoglobulin constant region comprising an immunoglobulin constant region gene and in which the one or more unrearranged human immunoglobulin variable region gene segments are operably linked to the immunoglobulin constant region gene.

Generally, a genetically modified immunoglobulin locus comprises an immunoglobulin variable region (comprising immunoglobulin variable region gene segments) operably linked to an immunoglobulin constant region. In some embodiments, the genetically modified immunoglobulin locus comprises one or more human unrearranged immunoglobulin heavy chain variable region gene segments, including an engineered D_{H} region, operably linked to a heavy chain constant region gene. In some embodiments, the genetically modified immunoglobulin locus comprises human unrearranged immunoglobulin variable region κ gene segments operably linked to a κ chain constant region gene. In some embodiments, the genetically modified immunoglobulin locus comprises human unrearranged immunoglobulin variable region λ gene segments operably linked to a κ chain constant region gene. In some embodiments, the genetically modified immunoglobulin locus comprises human unrearranged immunoglobulin variable region λ gene segments operably linked to a λ chain constant region gene.

In certain embodiments, the non-human animal comprises at an endogenous heavy chain locus an unrearranged human(ized) immunoglobulin heavy chain variable region comprising an engineered D_{H} region in operable linkage to an endogenous heavy chain constant region, wherein immunoglobulin variable region contains one or more unrearranged human Ig heavy chain variable region gene segments. In some embodiments, the one or more unrearranged human Ig variable region gene segments comprises at least one human immunoglobulin heavy chain variable (V_{H}) segment, one or more immunoglobulin heavy chain diversity (D_{H}) segments (e.g., one or more unrearranged human D_{H} segments operably linked to a 23-mer RSS), and one or more immunoglobulin heavy chain joining (J_{H}) segments (optionally one or more unrearranged human J_{H} segments). In some embodiments, the unrearranged human Ig variable region gene segments comprise a plurality of unrearranged human V_{H} segments, one or more unrearranged (human) D_{H} segments (e.g., one or more unrearranged (human) D_{H} segments operably linked to a 23-mer RSS) and one or more unrearranged (human) J_{H} segments. In some embodiments, the unrearranged human Ig variable region gene segments comprise at least 3 V_{H} gene segments, at least 18 V_{H} gene segments, at least 20 V_{H} gene segments, at least 30 V_{H} gene segments, at least 40 V_{H} gene segments, at least 50 V_{H} gene segments, at least 60 V_{H} gene segments, at least 70 V_{H} gene segments, or at least 80 V_{H} gene segments. In some embodiments, the unrearranged human Ig gene segments include all of the functional human D_{H} gene segments, wherein at least one of the functional human D_{H} gene segments is modified to be operably linked to a 23-mer RSS. In some embodiments, the unrearranged human Ig gene segments include all of the functional human J_{H} gene segments. Exemplary variable regions comprising Ig heavy chain gene segments are provided, for example, in Macdonald et al, Proc. Natl. Acad. Sci. USA 111 :5147-52 and supplemental information, which is hereby incorporated by reference in its entirety.

In some embodiments, the non-human animals provided herein comprise at an endogenous heavy chain locus a restricted unrearranged human(ized) heavy chain variable region in operable linkage to an endogenous heavy chain constant region comprising at least a non-human IgM gene, wherein the restricted unrearranged human(ized) heavy chain variable region is characterized by a single human V_{H} gene segment, a plurality of D_{H} gene segments (e.g., human D_{H} gene segments, including one or more unrearranged (human) D_{H} segments operably linked to a 23-mer RSS) and a plurality of J_{H} gene segments (e.g. human J_{H} gene segments), wherein the restricted immunoglobulin heavy chain locus is capable of rearranging and forming a plurality of distinct rearrangements, wherein each rearrangement is derived from the single human V_{H} gene segment, one of the D_{H} segments, and one of the J_{H} segments, and wherein each rearrangement encodes a different heavy chain variable domain (e.g., as described in U.S. Pat. Pub. No. 20130096287, which is hereby incorporated by reference herein in its entirety). In some embodiments the single human V_{H} gene segment is V_{H}1-2 or V_{H}1-69.

In certain embodiments, a non-human animal comprises at an endogenous light chain locus an unrearranged human(ized) immunoglobulin light chain variable region in operable linkage to an endogenous light chain constant region. In some embodiments the unrearranged human(ized) immunoglobulin light chain variable region contains unrearranged human Ig κ variable region gene segments. In some embodiments, the unrearranged human(ized) immunoglobulin variable region comprises a plurality of unrearranged human Vκ segments and one or more unrearranged human J_{K} segments. In some embodiments, the unrearranged human immunoglobulin variable region gene segments comprise all of the human Jκ segments. In some embodiments, the immunoglobulin variable region gene segments comprise four functional Vκ segments and all human Jκ segments. In some embodiments, the immunoglobulin variable region gene segments comprise 16 functional Vκ segments and all human Jκ segments (e.g., all functional human Vκ segments and Jκ segments). In some embodiments, the unrearranged human immunoglobulin variable region gene segments comprise all of the human Vκ segments and all human Jκ segments. Exemplary variable regions comprising Ig κ gene segments are provided, for example, in Macdonald et al, Proc. Natl. Acad. Sci. USA 111 :5147-52 and supplemental information, which is hereby incorporated by reference in its entirety.

In some embodiments, a restricted unrearranged human(ized) light chain variable region in operable linkage to an endogenous light chain constant region is characterized in that the unrearranged human(ized) light chain variable region comprises no more than two human V_{L} gene segments and a plurality of J_{L} gene segments (e.g., dual light chain mice, or DLC, as described in U. S. Pat. No. 9,796,788, which is hereby incorporated by reference herein in its entirety). In some embodiments the V_{L} gene segments are Vκ gene segments. In some embodiments the V_{L} gene segments are Vλ gene segments. In some embodiments the Vκ gene segments are IGKV3-20 and IGKV1 -39. In some embodiments, a non-human animal comprises exactly two unrearranged human Vκ gene segments and five unrearranged human Jκ gene segments operably linked to a mouse light chain constant region at the endogenous κ light chain loci of the mouse, optionally wherein the exactly two unrearranged human Vκ gene segments are a human Vκ1-39 gene segment and a human Vκ3-20 gene segment, wherein the five unrearranged human Jκ gene segments are a human Jκ1 gene segment, a human Jκ2 gene segment, a human Jκ3 gene segment, a human Jκ4 gene segment, and a human Jκ5 gene segment, wherein the unrearranged human kappa light chain gene segments are capable of rearranging and encoding human variable domains of an antibody, and optionally further wherein the non-human animal does not comprise an endogenous Vκ gene segment that is capable of rearranging to form an immunoglobulin light chain variable region.

In certain embodiments, the unrearranged human(ized) immunoglobulin light chain variable region in operable linkage to an endogenous light chain constant region contains unrearranged human Igλ variable region gene segments. In some embodiments, the unrearranged human immunoglobulin variable region gene segments comprise a plurality of human Vλ segments and one or more human Jλ segments. In some embodiment, the unrearranged human immunoglobulin variable region gene segments comprise one or more human Vλ segments, one or more human JX segments, and one or more human Cλ constant region sequences. In some embodiments, the unrearranged human immunoglobulin variable region gene segments comprise all of the human Vλ segments. In some embodiments, the unrearranged human immunoglobulin variable region gene segments comprise all of the human Jλ segments. Exemplary variable regions comprising Ig λ gene segments are provided, for example, U. S. Pat. Nos. 9,035,128 and 6,998,514, each of which is hereby incorporated by reference herein in its entirety. In some embodiments, the unrearranged human(ized) immunoglobulin light chain variable region in operable linkage to an endogenous light chain constant region comprises (a) one or more human Vλ gene segments, (b) one or more human Jλ gene segments, and (c) one or more human Cλ gene segments, wherein (a) and (b) are operably linked to (c) and an endogenous (e.g., rodent) Cλ gene segment, and wherein the endogenous immunoglobulin λ light chain locus further comprises: one or more rodent immunoglobulin λ light chain enhancers (Eλ), and one or more human immunoglobulin λ light chain enhancers (Eλ), optionally comprising three human Eλ.

In certain embodiments, the unrearranged human(ized) immunoglobulin light chain variable region in operable linkage to an endogenous light chain constant region comprises an unrearranged human Igλ variable region gene segments operably linked to an endogenous (e.g., rodent, e.g., rat or mouse) Cκ gene such that the non-human animal expresses an immunoglobulin light chain that comprises a human λ variable domain sequence derived from the Vλ and Jλ gene segments fused with an endogenous κ constant domain, see, e.g., US Patent No. 9,226,484, incorporated herein in its entirety by reference.

In some embodiments, the immunoglobulin variable region comprising unrearranged human immunoglobulin variable region gene segments also includes human immunoglobulin variable region intergenic sequences. In some embodiments, the immunoglobulin variable region includes non-human (e.g., rodent, rat, mouse) Ig variable region intergenic sequences. In some embodiments, the intergenic sequence is of endogenous species origin.

In some embodiments, the immunoglobulin variable region is a rearranged light variable region (a universal light chain variable region). In some embodiments, the rearranged Ig light chain variable region gene is a human rearranged Ig light chain variable region gene. Exemplary rearranged Ig light chain variable regions are provided in, e.g., U.S. Patent Nos.: 9,969,814; 10,130,181, and 10,143,186 and U.S. Patent Pub. Nos. 20120021409, 20120192300, 20130045492, 20130185821, 20130302836, and 20150313193, each of which are hereby incorporated by reference herein in its entirety. In some embodiments, the non-human organism ("universal light chain" organism) comprising a universal light chain variable region is used to produce bispecific antibodies. In some embodiments, a common light chain encoding sequence comprises a single rearranged human immunoglobulin light chain Vκ/Jκ sequence operably linked to an endogenous light chain constant region, wherein the single rearranged human immunoglobulin light chain Vκ/Jκ sequence is either (i) a human Vκ1-39/Jκ5 sequence comprising a human Vκ1-39 gene segment fused to a human Jκ5 gene segment, or (ii) a human Vκ3-20/Jκ1 sequence comprising a human Vκ3-20 gene segment fused to a human Jκ1 gene segment.

In some embodiments, the immunoglobulin variable region is a light chain and/or a heavy chain immunoglobulin variable region that includes insertions and/or replacements of histidine codons designed to introduce pH- dependent binding properties to the antibodies generated in such non-human organism. In some of such embodiments, the histidine codons are inserted and/or replaced in the nucleic acid sequences encoding CDR3. Various such light and/or heavy immunoglobulin loci are provided in U.S. Patent Nos. 9,301,510; 9,334,334; and 9,801,362 and U.S. Patent Application Publication No. 20140013456, each of which is incorporated herein by reference in its entirety. In some embodiments, the histidine modified rearranged human(ized) light chain variable region in operable linkage to an endogenous light chain constant region comprises a single rearranged human immunoglobulin light chain variable region gene sequence comprising human Vκ and Jκ segment sequences, optionally wherein the Vκ segment sequence is derived from a human Vκ1-39 or Vκ3-20 gene segment, and wherein the single rearranged human immunoglobulin light chain variable region gene sequence comprises a substitution of at least one non-histidine codon of the Vκ segment sequence with a histidine codon that is expressed at a position selected from the group consisting of 105, 106, 107, 108, 109, 111 and a combination thereof (according to IMGT numbering). In some embodiments, the histidine modified unrearranged human(ized) heavy chain variable region in operable linkage to an endogenous heavy chain constant region comprises an unrearranged human(ized) immunoglobulin heavy chain variable gene sequence comprising in a complementarity determining region 3 (CDR3) encoding sequence (e.g., in a (human) D_{H} gene segment modified to be operably linked to a 23-mer RSS) a substitution of at least one non-histidine codon with a histidine codon or an insertion of at least one histidine codon. In some embodiments, the unrearranged human(ized) immunoglobulin heavy chain variable gene sequence comprises unrearranged human V_{H}, unrearranged human D_{H} or synthetic D_{H} including a D_{H} gene segment operably linked to a 23-mer RSS, and unrearranged human J_{H} gene segments, optionally wherein the unrearranged human D_{H} or synthetic D_{H} gene segment or D_{H} gene segment operably linked to a 23-mer RSS comprises the substitution of at least one non-histidine codon with a histidine codon or an insertion of at least one histidine codon. In some embodiments, the histidine modified unrearranged human(ized) light chain variable region in operable linkage to an endogenous heavy chain constant region comprises unrearranged V_{L} and unrearranged J_{L} gene segments. In some embodiments, the histidine modified unrearranged human(ized) light chain variable region comprises no more than two unrearranged human V_{L} (e.g., no more than two Vκ gene segments) and one or more unrearranged human J_{L} (e.g., Jκ) gene segment(s), wherein each of the no more than two human V_{L} gene segments comprises in a CDR3 encoding sequence a substitution of at least one non-histidine codon with a histidine codon or an insertion of at least one histidine codon. In some embodiments, the no more than two unrearranged human Vκ gene segments are human Vκ1-39 and Vκ3-20 gene segments each comprising one or more substitutions of a non-histidine codon with a histidine codon, and wherein the human Vκ and Jκ gene segments are capable of rearranging and the human Vκ and Jκ gene segments encode a human light chain variable domain comprising one or more histidines at a position selected from the group consisting of 105, 106, 107, 108, 109, 111 (according to IGMT numbering), and a combination thereof, wherein the one or more histidines are derived from the one or more substitutions.

In some embodiments, the immunoglobulin constant region comprises a heavy chain constant region gene. In some embodiments, the heavy chain constant region gene is a human heavy chain constant region gene. In some embodiments, the heavy chain constant region gene is of endogenous species origin. In some embodiments, the heavy chain constant region gene is a mouse constant region gene or a rat constant region gene. In some embodiments, the constant region gene is a mixture of human and non-human sequence. For example, in some embodiments, the constant region gene encodes a human CH1 region and a non-human (e.g., endogenous species origin, mouse, rat) CH2 and/or CH3 region. In some embodiments, the heavy chain constant region gene is an Cµ, Cδ, Cγ (Cγ1, Cy2, Cy3, Cy4), Cα or Cε constant region gene. In some embodiments, the constant region gene is an endogenous constant region gene. In some embodiments, the constant region gene encodes a mutated CH1 region so that the non-human animal expresses heavy chain only antibodies (see., e.g., U.S. Patent No. 8,754,287, U.S. Patent Application Publication No. 2015/0289489, each of which is incorporated herein by reference in its entirety). In some embodiments, e.g., where the goal is to generate heavy chains to make bispecific antibodies (e.g., in universal or dual light chain organisms), the Fc domains of the heavy chains comprise modifications to facilitate heavy chain heterodimer formation and/or to inhibit heavy chain homodimer formation. Such modifications are provided, for example, in U.S. Pat. Nos. 5,731,168; 5,807,706; 5,821,333; 7,642,228 and 8,679,785 and in U.S. Pat. Pub. No. 2013/0195849, each of which is hereby incorporated by reference herein in its entirety.

In some embodiments, the immunoglobulin constant region comprises a light chain constant region gene. In some embodiments, the light chain constant region gene is a κ constant region gene. In some embodiments, the light chain constant region gene is a λ constant region gene. In some embodiments, the light chain constant region gene is of endogenous species origin. In some embodiments, the light chain constant region gene is a mouse constant region gene or a rat constant region gene. In some embodiments, the light chain constant region gene is a mixture of human and non-human sequence.

In some embodiments, the immunoglobulin variable region comprising human variable region gene segments and the immunoglobulin constant region gene to which the variable region gene segments are operably linked are located at an endogenous immunoglobulin locus. In some embodiments, the endogenous immunoglobulin locus is an endogenous heavy chain locus. In some embodiments, the endogenous immunoglobulin locus is an endogenous κ locus. In some embodiments, the endogenous immunoglobulin locus is an endogenous λ locus. In some embodiments, the constant region gene to which the human variable region gene segments are operably linked is an endogenous constant region gene.

In some embodiments, one or more of the endogenous immunoglobulin loci or a portion of the one or more endogenous loci (e.g., a variable region and/or a constant region) in the genome of the non-human animal provided herein is inactivated. Endogenous immunoglobulin variable region gene loci and portions thereof can be inactivated using any method known in the art, including, but not limited to, the deletion of the locus or a portion thereof from the genome of the organism, the replacement of a locus or a portion thereof with a different nucleic acid sequence, the inversion of a portion of the locus and/or the displacement of a portion of the locus to another position in the genome of the non-human organism. In some embodiments the inactivation of the locus is only a partial inactivation. In some embodiments, the variable region of the locus is inactivated but the constant region remains functional (e.g., because it is operably linked to non-endogenous variable region gene segments).

In some embodiments, the genetically modified non-human animal includes an inactivated endogenous immunoglobulin heavy chain locus. In some embodiments, the endogenous immunoglobulin heavy chain locus or a portion thereof is inactivated by deletion, replacement, displacement and/or inversion of at least part of the endogenous variable region of the endogenous heavy chain locus. In some embodiments, the at least part of the variable region of the endogenous heavy chain locus that is deleted, replaced, displaced, and/or inverted comprises the J segments of the variable region. In some embodiments, the endogenous immunoglobulin heavy chain locus or portion thereof is inactivated by deletion, replacement, displacement and/or inversion of at least part of the endogenous constant region of the endogenous heavy chain locus. In some embodiments, the at least part of the constant region of the endogenous heavy chain locus that is deleted, replaced, displaced, and/or inverted comprises the Oµ gene of the endogenous constant region.

In some embodiments, the genetically modified non-human animal includes an inactivated endogenous immunoglobulin κ chain locus. In some embodiments, the endogenous immunoglobulin κ chain locus or a portion thereof is inactivated by deletion, replacement, displacement and/or inversion of at least part of the endogenous variable region of the endogenous κ chain locus. In some embodiments, the at least part of the variable region of the endogenous κ chain locus that is deleted, replaced, displaced, and/or inverted comprises the J segments of the variable region. In some embodiments, the endogenous immunoglobulin κ chain locus or portion thereof is inactivated by deletion, replacement, displacement and/or inversion of at least part of the endogenous constant region of the endogenous κ chain locus. In some embodiments, the at least part of the constant region of the endogenous κ chain locus that is deleted, replaced, displaced, and/or inverted comprises the Cκ gene of the endogenous constant region.

In some embodiments, the genetically modified non-human animal includes an inactivated endogenous immunoglobulin λ chain locus. In some embodiments, the endogenous immunoglobulin λ chain locus or a portion thereof is inactivated by deletion, replacement, displacement and/or inversion of at least part of an endogenous variable region of the endogenous λ chain locus. In some embodiments, the at least part of at least one V-J-C gene cluster in the endogenous λ chain locus is deleted, replaced, displaced, and/or inverted. In some embodiments, the endogenous immunoglobulin λ chain locus or portion thereof is inactivated by deletion, replacement, displacement and/or inversion of at least part of an endogenous constant region of the endogenous λ chain locus. In some embodiments, the at least part of the constant region of the endogenous λ chain locus that is deleted, replaced, displaced, and/or inverted comprises a C gene of the endogenous constant region.

In various embodiments, the immunoglobulin locus modifications do not affect fertility of the non-human animal. In some embodiments, the heavy chain locus comprises a functional, e.g., endogenous ADAM6a gene, ADAM6b gene, or both, and the genetic modification does not affect the expression and/or function of the endogenous ADAM6a gene, ADAM6b gene, or both. In some embodiments, the genome of the genetically modified non-human animal further comprises an ectopically located functional, e.g., endogenous ADAM6a gene, ADAM6b gene, or both. Exemplary non-human animals expressing exogenous ADAM6a and/or ADAM6b are described in U.S. Pat. Nos. 8,642,835 and 8,697,940, each of which is hereby incorporated by reference in its entirety.

In some embodiments, the genetically modified non-human animal further comprises and expresses an exogenous terminal deoxynucleotidyl transferase (TdT) for increased antigen receptor diversity. Exemplary non-human animals expressing exogenous TdT are described in PCT Publication WO 2017210586, which is hereby incorporated by reference in its entirety.

In some embodiments, the genome of a provided non-human animal further comprises one or more human immunoglobulin heavy and/or light chain genes (see, e.g., U.S. Patent No. 8,502,018; U.S. Patent No. 8,642,835; U.S. Patent No. 8,697,940; U.S. Patent No: 8,791,323; and U.S. Patent Application Publication Nos. 2013/0096287 A1 and 2018/0125043 A1; and PCT Publication No. WO2019/113065, each of which are herein incorporated by reference in their entireties). Alternatively, an engineered D_{H} region can be introduced into an embryonic stem cell of a different modified strain such as, e.g., a VELOCIMMUNE^{®} strain (see, e.g., U.S. Patent No. 8,502,018 or U.S. Patent No. 8,642,835; herein incorporated by reference in their entireties). In some embodiments, non-human animals as described herein may be prepared by introducing a targeting vector as described herein, into a cell from a modified strain. To give but one example, a targeting vector as described herein, may be introduced into a non-human animal as described in U.S. Patent Nos. 8,642,835 and 8,697,940; herein incorporated by reference in their entireties, which non-human animal expresses antibodies that have fully human variable regions and mouse constant regions. In some embodiments, non-human animals as described herein are prepared to further comprise human immunoglobulin genes (variable and/or constant region genes). In some embodiments, non-human animals as described herein comprise an engineered D_{H} region as described herein, and genetic material from a heterologous species (e.g., humans), wherein the genetic material encodes, in whole or in part, one or more human heavy and/or light chain variable regions.

The non-human animals as described herein may be prepared as described above, or using methods known in the art, to comprise additional human or humanized genes, oftentimes depending on the intended use of the non-human animal. Genetic material of such additional human or humanized genes may be introduced through the further alteration of the genome of cells (e.g., embryonic stem cells) having the genetic modifications as described above or through breeding techniques known in the art with other genetically modified strains as desired.

For example, as described herein, non-human animals comprising an engineered D_{H} region may further comprise (e.g., via cross-breeding or multiple gene targeting strategies) one or more modifications as described U.S. Patent Application Publication Nos. 2011-0195454 A1, 2012-0021409 A1, 2012-0192300 A1, 2013-0045492 A1, 2013-0185821 A1, 2013-0198880 A1, 2013-0302836 A1, 2015-0059009 A1; International Patent Application Publication Nos. WO 2011/097603, WO 2012/148873, WO 2013/134263, WO 2013/184761, WO 2014/160179, WO 2014/160202; all of which are hereby incorporated by reference in their entireties.

A transgenic founder non-human animal can be identified based upon the presence of an engineered D_{H} region in its genome and/or expression of antibodies that include CDR3 regions containing amino acids resulting from D_{H}-D_{H} recombination in tissues or cells of the non-human animal. A transgenic founder non-human animal can then be used to breed additional non-human animals carrying the engineered D_{H} region thereby creating a series of non-human animals each carrying one or more copies of an engineered D_{H} region. Moreover, transgenic non-human animals carrying an engineered D_{H} region can further be bred to other transgenic non-human animals carrying other transgenes (e.g., human immunoglobulin genes) as desired.

Transgenic non-human animals may also be produced to contain selected systems that allow for regulated or directed expression of the transgene. Exemplary systems include the Cre/*lox*P recombinase system of bacteriophage P1 (see, e.g., Lakso, M. et al., 1992, Proc. Natl. Acad. Sci. USA 89:6232-6236, which is incorporated by reference in its entirety) and the FLP/Frt recombinase system of S. cerevisiae (O'Gorman, S. et al, 1991, Science 251:1351-1355; each of which is incorporated by reference in its entirety). Such animals can be provided through the construction of "*double*" transgenic animals, e.g., by mating two transgenic animals, one containing a transgene comprising a selected modification (e.g., an engineered D_{H} region) and the other containing a transgene encoding a recombinase (e.g., a Cre recombinase).

Although embodiments employing an engineered D_{H} region in a mouse (i.e., a mouse with an engineered D_{H} region operably linked with human V_{H} and J_{H} gene segments, all of which are operably linked with one or more murine heavy chain constant region genes) are extensively discussed herein, other non-human animals that comprise an engineered D_{H} region are also provided. In some embodiments, such non-human animals comprise an engineered D_{H} region operably linked to endogenous V_{H} and J_{H} gene segments. In some embodiments, such non-human animals comprise an engineered D_{H} region operably linked to humanized V_{H} and J_{H} gene segments. Such non-human animals include any of those which can be genetically modified to express antibodies having CDR3s that include amino acids resulting from D_{H}-D_{H} recombination at an increased frequency as compared to a wild-type immunoglobulin heavy chain locus as disclosed herein, including, e.g., mammals, e.g., mouse, rat, rabbit, pig, bovine (e.g., cow, bull, buffalo), deer, sheep, goat, chicken, cat, dog, ferret, primate (e.g., marmoset, rhesus monkey), etc. For example, for those non-human animals for which suitable genetically modifiable ES cells are not readily available, other methods are employed to make a non-human animal comprising the genetic modification. Such methods include, e.g., modifying a non-ES cell genome (e.g., a fibroblast or an induced pluripotent cell) and employing somatic cell nuclear transfer (SCNT) to transfer the genetically modified genome to a suitable cell, e.g., an enucleated oocyte, and gestating the modified cell (e.g., the modified oocyte) in a non-human animal under suitable conditions to form an embryo.

Methods for modifying a non-human animal genome (e.g., a pig, cow, rodent, chicken, etc.) include, e.g., employing a zinc finger nuclease (ZFN), a transcription activator-like effector nuclease (TALEN) or a Cas protein (i.e., a CRISPR/Cas system) to modify a genome to include an engineered D_{H} region as described herein. Guidance for methods for modifying the germline genome of a non-human animal can be found in, e.g., U.S. Patent Application Publication Nos. 2015-0376628 A1, US 2016-0145646 A1 and US 2016-0177339 A1; incorporated herein by reference in their entireties.

In some embodiments, a non-human animal as described herein is a mammal. In some embodiments, a non-human animal as described herein is a small mammal, e.g., of the superfamily Dipodoidea or Muroidea. In some embodiments, a genetically modified animal as described herein is a rodent. In some embodiments, a rodent as described herein is selected from a mouse, a rat, and a hamster. In some embodiments, a rodent as described herein is selected from the superfamily Muroidea. In some embodiments, a genetically modified animal as described herein is from a family selected from Calomyscidae (e.g., mouse-like hamsters), Cricetidae (e.g., hamster, New World rats and mice, voles), Muridae (true mice and rats, gerbils, spiny mice, crested rats), Nesomyidae (climbing mice, rock mice, with-tailed rats, Malagasy rats and mice), Platacanthomyidae (e.g., spiny dormice), and Spalacidae (e.g., mole rates, bamboo rats, and zokors). In some embodiments, a genetically modified rodent as described herein is selected from a true mouse or rat (family Muridae), a gerbil, a spiny mouse, and a crested rat. In some embodiments, a genetically modified mouse as described herein is from a member of the family Muridae. In some embodiment, a non-human animal as described herein is a rodent. In some embodiments, a rodent as described herein is selected from a mouse and a rat. In some embodiments, a non-human animal as described herein is a mouse.

In some embodiments, a non-human animal as described herein is a rodent that is a mouse of a C57BL strain selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, and C57BL/O1a. In some embodiments, a mouse of the present invention is a 129-strain selected from the group consisting of a strain that is 129P1, 129P2, 129P3, 129X1, 129S1 (e.g., 129S1/SV, 129S1/SvIm), 129S2, 129S4, 129S5, 129S9/SvEvH, 129/SvJae, 129S6 (129/SvEvTac), 129S7, 129S8, 129T1, 129T2 (see, e.g., Festing et al., 1999, Mammalian Genome 10:836; Auerbach, W. et al., 2000, Biotechniques 29(5):1024-1028, 1030, 1032; herein incorporated by reference in its entirety). In some embodiments, a genetically modified mouse as described herein is a mix of an aforementioned 129 strain and an aforementioned C57BL/6 strain. In some embodiments, a mouse as described herein is a mix of aforementioned 129 strains, or a mix of aforementioned BL/6 strains. In some embodiments, a 129 strain of the mix as described herein is a 129S6 (129/SvEvTac) strain. In some embodiments, a mouse as described herein is a BALB strain, e.g., BALB/c strain. In some embodiments, a mouse as described herein is a mix of a BALB strain and another aforementioned strain.

In some embodiments, a non-human animal as described herein is a rat. In some embodiments, a rat as described herein is selected from a Wistar rat, an LEA strain, a Sprague Dawley strain, a Fischer strain, F344, F6, and Dark Agouti. In some embodiments, a rat strain as described herein is a mix of two or more strains selected from the group consisting of Wistar, LEA, Sprague Dawley, Fischer, F344, F6, and Dark Agouti.

### Methods

Several *in vitro* and *in vivo* technologies have been developed for the production of antibody-based therapeutics. In particular, *in vivo* technologies have featured the production of transgenic animals (i.e., rodents) containing human immunoglobulin genes either randomly incorporated into the genome of the animal (e.g., see U.S. Patent No. 5,569,825, incorporated by reference in its entirety) or precisely placed at an endogenous immunoglobulin locus in operable linkage with endogenous immunoglobulin constant regions of the animal (e.g., see U.S. Patent Nos. 8,502,018; 8,642,835; 8,697,940; and 8,791,323; each of which is incorporated by reference in its entirety). Both approaches have been productive in producing promising antibody therapeutic candidates for use in humans. Further, both approaches have the advantage over *in vitro* approaches in that antibody candidates are chosen from antibody repertoires generated *in vivo,* which includes selection for affinity and specificity for antigen within the internal milieu of the host's immune system. In this way, antibodies bind to naturally presented antigen (within relevant biological epitopes and surfaces) rather than artificial environments or *in silico* predictions that can accompany *in vitro* technologies. Despite the robust antibody repertoires produced from *in vivo* technologies, antibodies to complex (e.g., viruses, channel polypeptides) or cytoplasmic antigens remains difficult. Further, generating antibodies to polypeptides that share a high degree of sequence identity between species (e.g., human and mouse) remains a challenge due to immune tolerance.

Thus, the present invention is, among other things, based on the recognition that the construction of an *in vivo* system characterized by the production of antibodies having added diversity in CDRs, in particular, CDR3s, generated from non-traditional gene segment rearrangement (i.e., D_{H} to D_{H} recombination) can be made using one or more D_{H} segments that are each operably linked to a 5' or 3' 23-mer RSS. By having the 5' or 3' 23-mer RSS, recombination between D_{H} segments is increased as compared to an immunoglobulin heavy chain locus that lacks such engineered D_{H} gene segments. Such added diversity can direct binding to particular antigens (e.g., viruses, channel polypeptides). Upon recombination of a V_{H} gene segment, at least two D_{H} gene segments, and a J_{H} gene segment, a heavy chain variable region coding sequence is formed that contains a CDR3 region having an amino acid sequence resulting from D_{H}-to-D_{H} recombination. Furthermore, this CDR3 resulting from D_{H} to D_{H} recombination contains an added diversity due to increased amino acid length. Furthermore, such a CDR3 region has the capability to direct binding to a particular antigen (or epitope) that is otherwise unable to be bound by an antibody generated by traditional VDJ recombination.

Provided non-human animals may be employed for making a human antibody, where the human antibody comprises variable domains derived from one or more variable region nucleic acid sequences encoded by genetic material of a cell of a non-human animal as described herein. For example, a provided non-human animal is immunized with an antigen of interest (e.g., virus or channel polypeptide, in whole or in part) under conditions and for a time sufficient that the non-human animal develops an immune response to said antigen of interest. Antibodies are isolated from the non-human animal (or one or more cells, for example, one or more B cells) and characterized using various assays measuring, for example, affinity, specificity, epitope mapping, ability for blocking ligand-receptor interaction, inhibition receptor activation, etc. In some embodiments, antibodies produced by provided non-human animals comprise one or more human variable domains that are derived from one or more human variable region nucleotide sequences isolated from the non-human animal. In some embodiments, anti-drug antibodies (e.g., anti-idiotype antibody) may be raised in provided non-human animals.

Non-human animals as described herein provide an improved *in vivo* system and source of biological materials (e.g., cells) for producing human antibodies that are useful for a variety of assays. In some embodiments, provided non-human animals are used to develop therapeutics that target one or more viruses and/or modulate viral activity and/or modulate viral interactions with other binding partners (e.g., a cell surface receptor). In some embodiments, provided non-human animals are used to develop therapeutics that target one or more channel proteins and/or modulate channel protein activity and/or modulate channel protein interactions with other binding partners. In some embodiments, provided non-human animals are used to identify, screen and/or develop candidate therapeutics (e.g., antibodies, siRNA, etc.) that bind one or more virus, channel or G-protein-coupled receptor (GPCR) polypeptides. In some embodiments, provided non-human animals are used to screen and develop candidate therapeutics (e.g., antibodies, siRNA, etc.) that block activity of one or more virus polypeptides, one or more human channel polypeptides or one or more human GPCR polypeptides. In some embodiments, provided non-human animals are used to determine the binding profile of antagonists and/or agonists of one or more human GPCR polypeptides or of one or more human channel polypeptides. In some embodiments, provided non-human animals are used to determine the epitope or epitopes of one or more candidate therapeutic antibodies that bind one or more human GPCR polypeptides or that bind one or more human channel polypeptides.

In some embodiments, provided non-human animals are used to determine the pharmacokinetic profiles of antibodies. In some embodiments, one or more provided non-human animals and one or more control or reference non-human animals are each exposed to one or more candidate therapeutic antibodies at various doses (e.g., 0.1 mg/kg, 0.2 mg/kg, 0.3 mg/kg, 0.4 mg/kg, 0.5 mg/kg, 1 mg/kg, 2 mg/kg, 3 mg/kg, 4 mg/kg, 5 mg/mg, 7.5 mg/kg, 10 mg/kg, 15 mg/kg, 20 mg/kg, 25 mg/kg, 30 mg/kg, 40 mg/kg, or 50 mg/kg or more). Candidate therapeutic antibodies may be dosed via any desired route of administration including parenteral and non-parenteral routes of administration. Parenteral routes include, e.g., intravenous, intraarterial, intraportal, intramuscular, subcutaneous, intraperitoneal, intraspinal, intrathecal, intracerebroventricular, intracranial, intrapleural or other routes of injection. Non-parenteral routes include, e.g., oral, nasal, transdermal, pulmonary, rectal, buccal, vaginal, ocular. Administration may also be by continuous infusion, local administration, sustained release from implants (gels, membranes or the like), and/or intravenous injection, e.g., using an intravenous fluid bag. Blood is isolated from non-human animals (humanized and control) at various time points (e.g., 0 hr, 6 hr, 1 day, 2 days, 3 days, 4 days, 5 days, 6 days, 7 days, 8 days, 9 days, 10 days, 11 days, or up to 30 or more days). Various assays may be performed to determine the pharmacokinetic profiles of administered candidate therapeutic antibodies using samples obtained from non-human animals as described herein including, but not limited to, total IgG, anti-therapeutic antibody response, agglutination, etc.

In some embodiments, provided non-human animals as are used to measure the therapeutic effect of blocking or modulating activity of a target antigen and the effect on gene expression as a result of cellular changes or cell surface density of the target antigen (in the case of a cell surface receptor) of cells of non-human animals as described herein. In some embodiments, a provided non-human animal or cells isolated therefrom are exposed to a candidate therapeutic that binds an antigen of interest and, after a subsequent period of time, analyzed for effects on target antigen-dependent processes (or interactions), for example, ligand-receptor interactions or antigen related signaling.

In some embodiments, provided non-human animals are used to measure the therapeutic effect of blocking or modulating channel activity (or channel signaling, or channel-mediated interactions, or channel action potentials) and the effect on gene expression as a result of cellular changes or the channel density of cells of non-human animals as described herein. In some embodiments, a provided non-human animal or cells isolated therefrom are exposed to a candidate therapeutic that binds a human channel polypeptide (or a portion thereof) and, after a subsequent period of time, analyzed for effects on channel-dependent processes (or interactions), for example, ligand-receptor interactions or channel action potentials.

In some embodiments, provided non-human animals express antibodies, thus cells, cell lines, and cell cultures can be generated to serve as a source of antibodies for use in binding and functional assays, e.g., to assay for binding or function of an antagonist or agonist, particularly where the antagonist or agonist is specific for a human polypeptide sequence or epitope or, alternatively, specific for a human polypeptide sequence or epitope that functions in ligand-receptor interaction (binding). In some embodiments, epitopes bound by candidate therapeutic antibodies or siRNAs can be determined using cells isolated from provided non-human animals.

In some embodiments, cells from provided non-human animals can be isolated and used on an ad hoc basis or can be maintained in culture for many generations. In some embodiments, cells from a provided non-human animal are immortalized (e.g., via use of a virus) and maintained in culture indefinitely (e.g., in serial cultures).

In some embodiments, non-human animals as described herein provide an *in vivo* system for the generation of antibody variants that binds a human target antigen. Such variants include antibodies having a desired functionality, specificity, low cross-reactivity to a common epitope shared by two or more human target antigens. In some embodiments, provided non-human animals are employed to generate panels of antibodies to generate a series of antibody variants that are screened for a desired or improved functionality.

In some embodiments, non-human animals as described herein provide an *in vivo* system for generating antibody libraries. Such libraries provide a source for heavy and light chain variable region sequences that may be grafted onto different Fc regions based on a desired effector function and/or used as a source for affinity maturation of the variable region sequence using techniques known in the art (e.g., site-directed mutagenesis, error-prone PCR, etc.).

In some embodiments, non-human animals as described herein provide an *in vivo* system for the analysis and testing of a drug or vaccine. In some embodiments, a candidate drug or vaccine may be delivered to one or more provided non-human animals, followed by monitoring of the non-human animals to determine one or more of the immune responses to the drug or vaccine, the safety profile of the drug or vaccine, or the effect on a disease or condition and/or one or more symptoms of a disease or condition. Exemplary methods used to determine the safety profile include measurements of toxicity, optimal dose concentration, antibody (i.e., anti-drug) response, efficacy of the drug or vaccine, and possible risk factors. Such drugs or vaccines may be improved and/or developed in such non-human animals.

Vaccine efficacy may be determined in a number of ways. Briefly, non-human animals described herein are vaccinated using methods known in the art and then challenged with a vaccine or a vaccine is administered to already-infected non-human animals. The response of a non-human animal(s) to a vaccine may be measured by monitoring of, and/or performing one or more assays on, the non-human animal(s) (or cells isolated therefrom) to determine the efficacy of the vaccine. The response of a non-human animal(s) to the vaccine is then compared with control animals, using one or more measures known in the art and/or described herein.

Vaccine efficacy may further be determined by viral neutralization assays. Briefly, non-human animals as described herein are immunized and serum is collected on various days post-immunization. Serial dilutions of serum are pre-incubated with a virus during which time antibodies in the serum that are specific for the virus will bind to it. The virus/serum mixture is then added to permissive cells to determine infectivity by a plaque assay or microneutralization assay. If antibodies in the serum neutralize the virus, there are fewer plaques or lower relative luciferase units compared to a control group.

Non-human animals as described herein provide an improved *in vivo* system for development and characterization of antibody-based therapeutics for use in cancer and/or inflammatory diseases. Inflammation has long been associated with cancer (reviewed in, e.g., Grivennikov, S.I. et al., 2010, Cell 140:883-99; Rakoff-Nahoum, S., 2006, Yale J. Biol. Med. 79: 123-30, each of which is herein incorporated by reference in their entireties). Indeed, a developing tumor environment is characterized, in part, by infiltration of various inflammatory mediators. Also, persistent inflammation can lead to a higher probability of developing cancer. Thus, in some embodiments, a non-human animal as described herein provides for an *in vivo* system for the development and/or identification of anti-cancer and/or anti-inflammatory therapeutics. In some embodiments, provided non-human animals or control non-human animals (e.g., having a genetic modification different than described herein or no genetic modification, i.e., wild-type) may be implanted with a tumor (or tumor cells), followed by administration of one or more candidate therapeutics. In some embodiments, candidate therapeutics may include a multi-specific antibody (e.g., a bi-specific antibody) or an antibody cocktail. In some embodiments, candidate therapeutics include combination therapy such as, for example, administration of two or more mono-specific antibodies dosed sequentially or simultaneously. The tumor may be allowed sufficient time to be established in one or more locations within the non-human animal prior to administration of one or more candidate therapeutics. Tumor cell proliferation, growth, survival, etc. may be measured both before and after administration with the candidate therapeutic(s). Cytotoxicity of candidate therapeutics may also be measured in the non-human animal as desired.

### Kits

The present invention further provides a pack or kit comprising one or more containers filled with at least one non-human animal, non-human cell, DNA fragment, and/or targeting vector as described herein. Kits may be used in any applicable method (e.g., a research method). Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both, or a contract that governs the transfer of materials and/or biological products (e.g., a non-human animal or non-human cell as described herein) between two or more entities.

Other features of the invention will become apparent in the course of the following descriptions of exemplary embodiments, which are given for illustration and are not intended to be limiting thereof.

### EXAMPLES

The following examples are provided so as to describe to those of ordinary skill in the art how to make and use methods and compositions of the invention, and are not intended to limit the scope of what the inventors regard as their invention. Unless indicated otherwise, temperature is indicated in Celsius, and pressure is at or near atmospheric.

### Example 1. Targeting vector design and construction

This example illustrates the construction of targeting vectors for inserting into the genome of a non-human animal such as a rodent (e.g., a mouse). In particular, the methods described in this example demonstrate the production of targeting vectors for inserting into the genome of rodent (e.g., a mouse) embryonic stem (ES) cells to produce a rodent whose genome comprises an immunoglobulin heavy chain variable region that includes a engineered heavy chain diversity (D_{H}) region, which engineered D_{H} region includes one or more D_{H} segments that are each operably linked to a 5' or 3' 23-mer RSS. In this example, three targeting vectors are described: a first targeting vector containing an engineered D_{H} region that includes a proximal (or 3') D_{H} segment operably linked to a 5' 23-mer RSS and operably linked to one human J_{H} segment, a second targeting vector containing an engineered D_{H} region that includes a proximal (or 3') D_{H} segment operably linked to a 5' 23-mer RSS and operably linked to three human J_{H} segments, and a third targeting vector containing an engineered D_{H} region that includes three D_{H} segments each associated with a 3' 23-mer RSS and operably linked to six human J_{H} segments (Figure 2). Each of these targeting vectors were generated and separately inserted into a humanized immunoglobulin heavy chain variable region (Figures 3, 5, 7). As described below, the engineered D_{H} region was placed in operable linkage with heavy chain variable (V_{H}) and heavy chain joining (J_{H}) segments so that, upon VDJ recombination, antibodies having CDR3s resulting from D_{H}-D_{H} recombination are expressed.

Targeting vectors containing one or more human D_{H} segments each associated with a 5' or 3' 23-mer RSS for insertion into an immunoglobulin heavy chain variable region were created using VELOCIGENE^{®} technology (see, e.g., U.S. Patent No. 6,586,251 and Valenzuela et al., 2003, Nature Biotech. 21(6):652-659; each of which are herein incorporated by reference in their entireties) and molecular biology techniques known in the art. The methods described in this example can be employed to utilize any D_{H} segment, set of D_{H} segments, or combination of D_{H} segments as desired.

### A. 23:D_{H}3-3:12/J_{H}6 targeting vector (Figure 2)

Briefly, a first targeting vector was constructed using a genomic DNA fragment containing a plurality of human D_{H} segments and a synthetic human D_{H}3-3 segment positioned in the place of human D_{H}7-27. A donor for *in vitro* Cas9/GA modification was made by *de novo* synthesis (Blue Heron Bio) and comprised from 5' to 3: (a) a 100 bp homology arm starting 350 bp upstream of the 5' 12-mer RSS of D_{H}7-27 (b) AgeI and XhoI sites for insertion of a neomycin-resistance cassette, (c) the 250bp region upstream of the 5'-end 12 RSS of D_{H}7-27, (d) a synthetic human D_{H}3-3 segment engineered with a 5'end 23-mer RSS (from J_{H}4) and a 3'end 12-mer RSS (from D_{H}3-3), and (3) a 100bp homology box starting 3bp downstream of J_{H}5. A loxp-UbC-Em7-Neo-loxp cassette, e.g., a neomycin resistance gene flanked by *lox*P site-specific recombination recognition sites, was positioned ~250bp upstream of the synthetic D_{H} segment and downstream by ligation into the AgzI and XhoI sites to allow selection in E. coli and mouse ES cells. This 23:D_{H}3-3:12/J_{H}6 donor (SEQ ID NO:61) including the neomycin cassette was used to modify a BAC by *in vitro* Cas9/GA using two Cas9:gRNA complexes. Prior to the modification, the BAC was identical in sequence to the chimeric IgH locus of a VELOCIMMUNE^{®} mouse (see, e.g., Figures 3 and 4, showing an exemplary non-limiting endogenous immunoglobulin heavy chain locus of a VELOCIMMUNE^{®} mouse that is heterozygous for a 6394 allele comprising a humanized variable region that lacks J_{H} gene segments and is operably linked to and endogenous mouse immunoglobulin heavy chain constant region sequence and a 1460 allele comprising a humanized variable region operably linked to an endogenous mouse immunoglobulin heavy chain constant region sequence.). Specifically, the BAC was identical to the 1460 allele starting from the most proximal human V_{H} gene (V_{H}6-1) and including all 27 human D_{H} genes, all 6 human J_{H} genes, the mouse IgH intronic enhancer (Eµ), the mouse IgM switch region (Sµ), and the first 4 exons of the mouse IgM gene. The BAC also included a spectinomycin (spec)-resistance cassette and ~29kb of human intergenic sequence upstream from the V_{H}6-1 gene. The human-mouse junction is 222bp 3' of the human J_{H}6 gene and 490 bp 5' of the mouse Eµ enhancer. Insertion of the 23:D3-3-12/J_{H}6 donor into the BAC via GA resulted in the final 23:D3-3:12/J_{H}6 targeting vector comprising a replacement of the D_{H}7-7 gene segment with the engineered 23:D3-3:12 segment and deletion of J_{H}1, J_{H}2, J_{H}3, J_{H}4, and J_{H}5 gene segments(Figure 2). Table 1 provides the sequence of the gRNA, primers and probes used to determine accurate construction of the 23:D_{H}3-3:12/J_{H}6 targeting vector.

**Table 1: Primer and gRNA sequences for construction of 23:D_{H}3-3:12/J_{H}6 targeting vector**

| ***Cas9 sites and gRNAs*** | ***Sequence (protospacer adjacent motif)*** |
|---|---|
| DNA recognition site | TCAGAAAGCAAGTGGATGAG(AGG) ***SEQ ID NO:62*** |
| 5' D7.27 crRNA | UCAGAAAGCAAGUGGAUGAG GUUUUAGAGCUAUGCUGUUUUG; SEQ ID NO:63 |
| | |
| DNA recognition site | TTAGGGAGACTCAGCTTGCC(AGG); ***SEQ ID NO:64*** |
| 3' JH1-5 del crRNA | UUAGGGAGACUCAGCUUGCC GUUUUAGAGCUAUGCUGUUUUG; SEQ ID NO:65 |
| | |

| ***PCR*/*Sequencing Primers*** | ***Sequence*** |
|---|---|
| 5' up detect D7-27 | GTGAACAGGTGGAACCAAC; ***SEQ ID NO:66*** |
| 3' ub pro-200 | CCAGTGCCCTAGAGTCACCCA; ***SEQ ID NO:67*** |
| | |
| 5' neo detect | CTCCCACTCATGATCTATAGA; ***SEQ ID NO:68*** |
| 3' down detect JH1-5 del | ACGCAATCATCACGACAGC; ***SEQ ID NO:69*** |

The 23 :D3-3: 12/JH6 targeting vector was linearized with NotI and electroporated into mouse embryonic stem cells having a genome that was heterozygous for a humanized immunoglobulin heavy chain variable region (i.e., a first heavy chain allele (1460het) containing a plurality of human V_{H}, D_{H}, and J_{H} segments operably linked to a rodent immunoglobulin heavy chain constant region including rodent heavy chain enhancers and regulatory regions, and containing an inserted nucleotide sequence encoding one or more murine Adam6 genes (e.g., U.S. Patent Nos. 8,642,835 and 8,697,940, each of which is incorporated by reference herein in its entirety); and a second heavy chain allele (6394het) containing a plurality of human V_{H} and D_{H} segments, a J_{H} region deletion, and a rodent immunoglobulin heavy chain constant region including rodent heavy chain enhancers and regulatory regions, and containing an inserted nucleotide sequence encoding one or more murine Adam6 genes (e.g., U.S. Patent Nos. 8,642,835 and 8,697,940, each of which is incorporated by reference herein in its entirety)) and that was homozygous (HO) for a humanized endogenous κ locus comprising the full repertoire of human immunoglobulin light chain Vκ and Jκ gene segments operably linked to an endogenous mouse immunoglobulin heavy chain Cκ region (1293) (see Figure 3). The 6799 allele is created upon electroporation and proper homologous recombination of the 1460 allele with the 23:D3-3:12/J_{H}6 targeting vector comprising the neomycin cassette. (Figure 3). These engineered mouse ES cells heterozygous for the 6394 and 679 alleles were employed to facilitate efficient screening of positive ES clones (see below) and subsequent cre-mediated removal of the drug resistance cassettes, after which deletion the 6394 and 6799 alleles are respectively referred to as 6643 and 6800 (Figure 4).

### B. 23:D_{H}3-3:12/J_{H}4-6 targeting vector (Figure 2)

In a similar manner, another targeting vector (23:D_{H}3-3:12/J_{H}4-6 ) was constructed using the same a genomic DNA fragment containing a plurality of human D_{H} segments and a synthetic human D_{H}3-3 segment positioned in the place of human D_{H}7-27. However, this second targeting vector included three of the six human J_{H} segments (i.e., J_{H}4, J_{H}5, J_{H}6). To create the final 23:D_{R}3-3: 12/J_{H}4-6 targeting vector, a donor was used to modify the BAC identical in sequence to the chimeric IgH locus of a VELOCIMMUNE^{®} mouse (see, e.g., Figures 3 and 4, showing an exemplary non-limiting endogenous immunoglobulin heavy chain locus of a VELOCIMMUNE^{®} mouse that is heterozygous for a 6394 allele comprising a humanized variable region that lacks J_{H} gene segments and is operably linked to and endogenous mouse immunoglobulin heavy chain constant region sequence and a 1460 allele comprising a humanized variable region operably linked to an endogenous mouse immunoglobulin heavy chain constant region sequence). The donor comprised, from 5' to 3': (a) a 100bp homology box starting 350bp upstream of the 5'-end 12 RSS of D7-27, (b) AgeI and Xhol sites for inserting a neomycin-resistance cassette, (c) the 250bp region upstream of the 5'-end 12 RSS of D7-27, (d) a synthetic human D_{H}3-3 segment flanked 5' by a 23-mer RSS (from J_{H}4) and 3' by a 12-mer RSS (from D_{H}3-3), and (e) a 100bp homology box starting 3bp downstream of J_{H}3. A loxp-UbC-Em7-Neo-loxp cassette, e.g., a neomycin resistance gene flanked by *lox*P site-specific recombination recognition sites, was positioned ~250bp upstream of the synthetic D_{H} segment by ligation into the AgeI and XhoI sites to allow selection in E. coli and mouse ES cells. This 23:D3-3:12/J_{H}4-6 donor comprising a nucleotide sequence set forth as SEQ ID NO:52, including the neomycin cassette, was used to modify the BAC identical in sequence to the 1460 allele (Figures 3 and 4). Specifically, the BAC was identical to the 1460 allele starting from the most proximal human V_{H} gene (V_{H}6-1) and including all 27 human D_{H} genes, all 6 human J_{H} genes, the mouse IgH intronic enhancer (Eµ), the mouse IgM switch region (Sµ), and the first 4 exons of the mouse IgM gene. The BAC also included a spectinomycin (spec)-resistance cassette and ~29kb of human intergenic sequence upstream from the V_{H}6-1 gene. The human-mouse junction is 222bp 3' of the human J_{H}6 gene and 490 bp 5' of the mouse Eµ enhancer. Insertion of the 23:D3-3:12/J_{H}4-6 donor into the BAC via *in vitro* Cas9/GA using two Cas9:gRNA complexes resulted in the final 23:D3-3:12/J_{H}4-6 targeting vector (Figure 2) comprising a replacement of the D_{H}7-7 gene segment with the engineered 23:D3-3:12 segment and deletion of J_{H}1, J_{H}2, and J_{H}3 gene segments. Table 2 provides the sequence of the gRNA, primers and probes used to determine accurate construction of the 23:D_{H}3-3:12/J_{H}6 targeting vector.

**Table 2: Primer and gRNA sequences for construction of 23:D_{H}3-3:12/JH4-6 targeting vector**

| ***Cas9 sites and gRNAs*** | ***Sequence (protospacer adjacent motif)*** |
|---|---|
| DNA recognition site | TCAGAAAGCAAGTGGATGAG(AGG); ***SEQ ID NO:53*** |
| 5' D7.27 gRNA | UCAGAAAGCAAGUGGAUGAG GUUUUAGAGCUAUGCUGUUUUG; SEQ ID NO:54 |
| | |
| DNA recognition site | GCTCCAGGACAGAGGACGCT(GGG); ***SEQ ID NO:55*** |
| 3' J_{H}1-3 del gRNA | GCUCCAGGACAGAGGACGCU GUUUUAGAGCUAUGCUGUUUUG; SEQ ID NO:56 |
| | |

| ***PCR*/*Sequencing Primers*** | ***Sequence*** |
|---|---|
| 5' up detect D7-27 | GTGAACAGGTGGAACCAAC; ***SEQ ID NO:57*** |
| 3' ub pro-200 | CCAGTGCCCTAGAGTCACCCA; ***SEQ ID NO:58*** |
| | |
| 5' neo detect | CTCCCACTCATGATCTATAGA; ***SEQ ID NO:59*** |
| 3' down detect J_{H}1-3 del | GTCCCAGTTCCCAAAGAAAG; ***SEQ ID NO:60*** |

The 23 :D3-3: 12/J_{H}4-6 targeting vector was also linearized with NotI and electroporated into mouse embryonic stem cells having a genome that was heterozygous for a humanized immunoglobulin heavy chain variable region (i.e., a first heavy chain allele (1460het) containing a plurality of human V_{H}, D_{H}, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940, each of which is incorporated by reference in its entirety]; and a second heavy chain allele (6394het) containing a plurality of human V_{H} and D_{H} segments, a J_{H} region deletion, and a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940, each of which is incorporated by reference herein]) and that was homozygous (HO) for a humanized endogenous κ locus comprising the full repertoire of human immunoglobulin light chain Vκ and Jκ gene segments operably linked to an endogenous mouse immunoglobulin heavy chain Cκ region (1293) (see, Figure 5). The 6797 allele is created upon electroporation and proper homologous recombination of the 1460 allele with the 23:D3-3:12/J_{H}4-6 targeting vector comprising the neomycin cassette. (Figure 5). These engineered mouse ES cells heterozygous for the 6394 and 6797 alleles were employed to facilitate efficient screening of positive ES clones (see below) and subsequent cre-mediated removal of the drug resistance cassettes, after which deletion the 6394 and 6797 alleles are respectively referred to as 6643 and 6798 (Figure 6).

### C. 12:D_{H}2-2:23|12:D_{H}2-8:23|12:D_{H}2-15:23 /J_{H}1-6 targeting vector (Figure 2)

Briefly, a third targeting vector was constructed using a DNA fragment containing a plurality of human D_{H} segments that included three D_{H}2 family gene segments (D_{H}2-2, D_{H}2-8 and D_{H}2-15) each having a 12-mer 5' RSS and a 23-mer 3' RSS. Firstly, the BAC identical in sequence to the chimeric IgH locus of a VELOCIMMUNE^{®} mouse (see, e.g., Figures 3 and 4, showing an exemplary non-limiting endogenous immunoglobulin heavy chain locus of a VELOCIMMUNE^{®} mouse that is heterozygous for a 6394 allele comprising a humanized variable region that lacks J_{H} gene segments and is operably linked to and endogenous mouse immunoglobulin heavy chain constant region sequence and a 1460 allele comprising a humanized variable region operably linked to an endogenous mouse immunoglobulin heavy chain constant region sequence). Specifically, the BAC was identical to the 1460 allele starting from the most proximal human V_{H} gene (V_{H}6-1) and including all 27 human D_{H} genes, all 6 human J_{H} genes, the mouse IgH intronic enhancer (Eµ), the mouse IgM switch region (Sµ), and the first 4 exons of the mouse IgM gene was modified using bacterial homologous recombination (BHR) to insert a loxp-UbC-Em7-Neo-loxp cassette, e.g., a neomycin resistance gene flanked by *lox*P site-specific recombination recognition sites, 512 bp upstream of the first human D_{H} segment (D_{H}1-1) for selection in *E. coli* and mouse ES cells. This resulting BAC was subsequently modified with three donors using *in vitro* Cas9/GA.
1. The first donor for modification of D_{H}2-2 contained, from 5' to 3', a 50bp homology box starting 1419bp upstream of the 5'-end 12 RSS of D_{H}2-2, a multiple cloning site (MreI-NsiI-EcoRI-KpnI-MreI), a modified D2-2 gene with the 3'-end 12-mer RSS replaced by an optimized 3'-end 23-mer RSS derived from human V_{H}1-69 (CACAGTGTGA AAACCCACAT CCTGAGAGTG ACACAAACC; T->A, G->C; SEQ ID NO:151), and a 50bp homology box ending 863bp downstream of the 3'-end 23-mer RSS of D2-2. The nucleotide sequence of this first donor for modification of D_{H}2-2 is set forth as SEQ ID NO:70. Since the D_{H} region consists of four direct repeats of ~10kb, designing unique primers and probes for screening is extremely difficult. To overcome this problem, two unique 40bp sequences were inserted: one 74bp downstream of the 5' homology box and one 40bp upstream of the 3' homology box. These unique 40-mers were used as binding sites for PCR/sequencing primers and Taqman probes, are denoted by unfilled vertical rectangles "1" and "2" of Figure 2, and comprise a nucleotide sequence set forth as SEQ ID NO:73 and SEQ ID NO:74, respectively
2. The second donor for modification of D_{H}2-8 contained, from 5' to 3', a 50bp homology box starting 552bp upstream of the 5'-end 12 RSS of D_{H}2-8, a multiple cloning site (MreI-NsiI-EcoRI-KpnI-MreI), a modified D2-8 gene with the 3'-end 12-mer RSS replaced by an optimized 3'-end 23-mer RSS derived from human VH1-69 (CACAGTGTGA AAACCCACAT CCTGAGAGTG ACACAAACC; T->A, G->C SEQ ID NO:151), and a 50bp homology box ending 867bp downstream of the 3'-end 23-mer RSS of D2-8. The nucleotide sequence of this second donor for modification of D_{H}2-8 is set forth as SEQ ID NO:71. Since the D_{H} region consists of four direct repeats of ~10kb, designing unique primers and probes for screening is extremely difficult. To overcome this problem, two unique 40bp sequences were inserted: one 74bp downstream of the 5' homology box and one 40bp upstream of the 3' homology box. These unique 40-mers were used as binding sites for PCR/sequencing primers and Taqman probes, are denoted by unfilled vertical rectangles "10" and "16" of Figure 2 and comprise a nucleotide sequence set forth as SEQ ID NO:75 and SEQ ID NO:76, respectively.
3. The third donor for modification of D_{H}2-15 contained, from 5' to 3', a 50bp homology box starting 391bp upstream of the 5'-end 12 RSS of D_{H}2-15, a multiple cloning site (MreI-NsiI-EcoRI-KpnI-MreI), a modified D2-15 gene with the 3'-end 12-mer RSS replaced by an optimized 3'-end 23-mer RSS derived from human V_{H}1-69 (CACAGTGTGA AAACCCACAT CCTGAGAGTG ACACAAACC; T->A, G->C SEQ ID NO:151), and a 50bp homology box ending 867bp downstream of the 3'-end 23-mer RSS of D2-15. The nucleotide sequence of this third donor for modification of D_{H}2-2 is set forth as SEQ ID NO:72. Since the D_{H} region consists of four direct repeats of ~10kb, designing unique primers and probes for screening is extremely difficult. To overcome this problem, two unique 40bp sequences were inserted: one 74bp downstream of the 5' homology box and one 40bp upstream of the 3' homology box. These unique 40-mers were used as binding sites for PCR/sequencing primers and Taqman probes, are denoted by unfilled vertical rectangles "8" and "18" of Figure 2, and comprise a nucleotide sequence set forth as SEQ ID NO:77 and SEQ ID NO:78, respectively.

After *in vitro* Cas9/GA modification with the three donors, the final targeting vector contained, from 5' to 3', a 49kb 5' homology arm containing human variable region DNA, a Neomycin selection cassette, an engineered human D_{H} region including three human DH segments each flanked 5' by a 12-mer RSS and 3' by a 23-mer RSS, six human J_{H} segments, and a 24kb 3' homology arm containing a mouse heavy chain intronic enhancer (Ei) and a mouse IgM constant region gene. (Figure 2). Table 3 provides the sequence of the gRNA, primers and probes used to determine accurate construction of the 12:D_{H}2-2:23|12:D_{H}2-8:23|12:D_{H}2-15:23 /J_{H}1-6 targeting vector.

**Table 3: Primer and gRNA sequences for construction of 12:DH2-2:23|12:DH2-8:23|12:DH2-15:23 /JH1-6**

| ***Step*** | ***Name*** | ***Sequence (protospacer adjacent motif)*** |
|---|---|---|
| 1. BHR: VI433-pLMa0298= VI826 | 5' hIgHD HU2(h268) | GCAGTAACCCTCAGGAAGCA; ***SEQ ID NO:79*** |
| | 3' hIgHD HU2 AgeI(h269) | CTTCC**ACCGGT**ACAGCCACACCAAGGTCATC; ***SEQ ID NO:80*** |
| | | |
| | 5' hIgHD HD2 XhoI(h271) | CTTCC**CTCGAG**GAACACTGTCAGCTCCCACA; ***SEQ ID NO:81*** |
| | 3' hIgHD HD2(h272) | AGATCCTCC ATGCGTGCTG; ***SEQ ID NO:82*** |
| | | |
| Jxn PCR | 5' hIgHD HU2 detect(h270) | TCTCCTCTCGTCGCCTCTAC; ***SEQ ID NO:83*** |
| | 3' ub pro-200 | CCAGTGCCCTAGAGTCACCCA; ***SEQ ID NO:84*** |
| | | |
| Jxn PCR | 5' neo detect | CTCCCACTCATGATCTATAGA; ***SEQ ID NO:85*** |
| | 3' hIgHD HD2 detect(h273) | GGGTGCTTGGGTCCTGTTAG; ***SEQ ID NO:86*** |
| 2. Cas9/GA: VI826-p51816= VI835 | | |
| gRNAs | 5' D2-2 Cas9 DNA target | AGGTCTGGAGCTACAAGCGG(TGG); ***SEQ ID NO:87*** |
| | 5' D2-2 gRNA | AGGUCUGGAGCUACAAGCGG GUUUUAGAGCUAUGCUGUUUUG; SEQ ID NO:88 |
| | | |
| | 3' D2-2 Cas9 DNA target | AGGACAACAGTGAGGGTTAC(AGG); ***SEQ ID NO:89*** |
| | 3' D2-2 gRNA | AGGACAACAGUGAGGGUUAC GUUUUAGAGCUAUGCUGUUUUG; SEQ ID NO:90 |
| | | |
| Jxn PCR | 5' up detect D2-2(h274) | GTCAAAGGTGGAGGCAGTG; ***SEQ ID NO:91*** |
| | 3' cm up detect | TCCAGCTGAACGGTCTGGTTA; ***SEQ ID NO:92*** |
| | | |
| Jxn PCR | D2-2seqF2 | AGCGATTCAACAGCTAACC; ***SEQ ID NO:93*** |
| | 3' down detect D2-2(h275) | CCAGTAGAAGTAACGACCAC; ***SEQ ID NO:94*** |
| 3. MreI/JO-1 GA: VI8352-delCM= VI841 | | |
| GA | JO-1 | CTCCAGACCCCCAAGACTGGGACCTGCCTTCC TGCCACCGCTTGTAGCTCCAGACCTCCGTGCC TCCCCCGACCACTTAC; SEQ ID NO:95 |
| | | |
| Jxn PCR | 5' up detect D2-2(h274) | GTCAAAGGTGGAGGCAGTG; ***SEQ ID NO:96*** |
| | D2-2seqR1 | CCCGTGCCTAGATCAATGC; ***SEQ ID NO:97*** |
| | | |
| Additional D2-2 sequencing primers | D2-2seqF1 | AGGCATTGATCTAGGCACG; ***SEQ ID NO:98*** |
| | D2-2seqR2 | CTGTTGAATCGCTGCAAGC; ***SEQ ID NO:99*** |
| 4. Cas9/GA: VI841- | | |
| p53419= VI853 | | |
| crRNAs | 5' D2-8 Cas9 DNA target | ACCTGAAGACGCCCAGTCAA(CGG) ***SEQ ID NO:100*** |
| | 5' D2-8 crRNA | ACCUGAAGACGCCCAGUCAA GUUUUAGAGCUAUGCUGUUUUG; SEQ ID NO:101 |
| | | |
| | 3' D2-8 Cas9 DNA target | GCCACAAGCACAAAAGTACA(GGG); ***SEQ ID NO:102*** |
| | 3' D2-8 crRNA | GCCACAAGCACAAAAGUACA GUUUUAGAGCUAUGCUGUUUUG; SEQ ID NO:103 |
| | | |
| Jxn PCR | 5' up detect D2-8(h276) | GCCTTACCCAAGTCTTTCC; ***SEQ ID NO:104*** |
| | 3' cm up detect | TCCAGCTGAACGGTCTGGTTA; ***SEQ ID NO:105*** |
| | | |
| Jxn PCR | D2-8seqF2 | TCCAATAAGTCGGTTCGGC; ***SEQ ID NO:106*** |
| | 3' down detect D2-8(h277) | GCAGAAGTAACCACCACTG; ***SEQ ID NO:107*** |
| 5. MreI/JO-2 GA= VI853-delCM= VI872 | | |
| GA | JO-2 | TCACAGGCCTCTCCTGGGAAGAGACCTGAAG ACGCCCAGTCAACGGAGTCTAACACCAAACC TCCCTGGAGGCCGATGGG; SEQ ID NO:108 |
| | | |
| Jxn PCR | 5' up detect D2-8(h276) | GCCTTACCCAAGTCTTTCC; ***SEQ ID NO:109*** |
| | D2-8seqR1 | GCCCTACTTGTAGTTACGTG; ***SEQ ID NO:110*** |
| | | |
| Additional D2-8 sequencing primers | D2-8seqF1 | GCAAA ATCCGACACGTAAC; ***SEQ ID NO:111*** |
| | D2-8seqR2 | CGAACCGACTTATTGGATGC; ***SEQ ID NO:112*** |
| 6. Cas9/GA: VI872-p51818= VI886 | | |
| crRNAs | 5' D2-8 Cas9 DNA target | GGTGGCCCCATAACACACCT(AGG); ***SEQ ID NO:113*** |
| | 5' D2-8 crRNA | GGUGGCCCCAUAACACACCU GUUUUAGAGCUAUGCUGUUUUG; SEQ ID NO:114 |
| | | |
| | 3' D2-8 Cas9 DNA target | GGACAACAGTAGAGGGCTAC(AGG); ***SEQ ID NO:115*** |
| | 3' D2-8 crRNA | GGACAACAGUAGAGGGCUAC GUUUUAGAGCUAUGCUGUUUUG; SEQ ID NO:116 |
| | | |
| Jxn PCR | 5' up detect D2-15(h278) | GCACTCCCTCTAAAGACAAG; ***SEQ ID NO:117*** |
| | 3' cm up detect | TCCAGCTGAACGGTCTGGTTA; ***SEQ ID NO:118*** |
| | | |
| Jxn PCR | D2-15seqF2 | GTAAAAATACCGCCGCTGG; ***SEQ ID NO:119*** |
| | 3' down detect D2-15(h279) | GCATTTTCCTGTTCTTGCG; ***SEQ ID NO:120*** |
| 7. MreI/JO-2 GA= VI886-delCM = VI896/MAI D20187 | | |
| GA | JO-3 | GCTGACATCCCGAGCTCCTCAATGGTGGCCCC ATAACACACCTAGGAAACATAACACACCCAC AGCCCCACCTGGAACAG; SEQ ID NO:121 |
| | | |
| Jxn PCR | 5' up detect D2-15(h278)* | GCACTCCCTCTAAAGACAAG; ***SEQ ID NO:122*** |
| | D2-15seqR1 | CTGGGAGATATTGGTGCATC; ***SEQ ID NO:123*** |
| | | |
| Additional D2-15 sequencing primers | D2-15seqF1 | TGCACCAATATCTCCCAGT; ***SEQ ID NO:124*** |
| | D2-15seqR2 | CGGCGGTATTTTTACTGACC; ***SEQ ID NO:125*** |

| Name | Forward Primer; Probe; Revere Primer | Type of Probe | Type of Assay | Specificity |
|---|---|---|---|---|
| 23:D3-3:12/1 | Fwd -TTTTTGTGCACCCCTTAATGG; ***SEQ ID NO:126*** | Taqman MGB | GOA | 23:D3-3:12 segment |
| | ProbeATGTGGTATTACGATTTTTGGA; ***SEQ ID NO:127*** | | | |
| | TCTGTGACACTGTGGGTATAATAAC C; SEQ ID NO:128 Reverse- | | | |
| | Fwd - CCCACAGTGTCACAGAGTCCAT; ***SEQ ID NO:129*** | | | |
| | Probe - CAAGATGGCTTTCCTTCTGCCTCC; ***SEQ ID NO:130*** | | | |
| 23:D3-3:12/2 | Reverse - TCCCAGCTCCAGGACAGAG; ***SEQ ID NO:131*** | BHQ1 | GOA | MAID6797 3' jxn |
| | FwdTGTCACAGAGTCCATCAAAAACCT; ***SEQ ID NO:132*** | | | |
| | Probe - CACCACCCCCTCTC; ***SEQ ID NO:133*** | | | |
| 23:D3-3:12/3 | ReverseCCTGAGACCCTGGCAAGCT; ***SEQ ID NO:134*** | Taqman MGB | GOA | MAFD6799 3' jxn |

| Name | Forward Primer, Probe, Reverse Primer | Type of Probe | Type of Assay | Specificity |
|---|---|---|---|---|
| | Fwd -ATGGCGCTTGCAGCGATTC; ***SEQ ID NO:135*** | | | |
| | Probe - CCAATACTAACGAGGAAATGCAAACCCA; ***SEQ ID NO:136*** | | | |
| Jxn D_{H}2-2 | Reverse - ACCCTCACTGTTGTCCTTCTG; ***SEQ ID NO:137*** | BHQ1 | GOA | 40bp-2 jxn of D2-2 |
| | Fwd -GCAAACGTCCATCTGAAGGAGAA; ***SEQ ID NO:138*** | | | |
| | Probe - CAAATAAACGATGGCAGGCTACACCCG; ***SEQ ID NO:139*** | | | |
| Jxn D_{H}2-8 | Reverse - GAGTTGCCGAACCGACTTATTG; ***SEQ ID NO:140*** | BHQ1 | GOA | 40bp-16 jxn of D2-8 |
| | Fwd -GCAATGGTAGGTTCATGTCCATC; ***SEQ ID NO:141*** | | | |
| Jxn D_{H}2-15 | Probe- ACCGCCGCTGGTACTCCAAT; ***SEQ ID NO:142*** | BHQ1 | GOA | 40bp-18 jxn of D2-15 |
| | Reverse- CCCTGGCTGTCTGGGTTTG; ***SEQ ID NO:143*** | | | |

The 12:D_{H}2-2:23:|12:D_{H}2-8:23|12:D_{H}2-15:23x3/J_{H}1-6 targeting vector was also linearized with NotI and electroporated into mouse embryonic stem cells having a genome that was homozygous for a humanized immunoglobulin heavy chain variable region that contained the full repertoire of functional human V_{H} gene segments, a deletion of the D_{H} region except for D_{H}7-27, and a full repertoire of functional human J_{H} gene segments (6011) and that was homozygous (HO) for a humanized endogenous κ locus comprising the full repertoire of human immunoglobulin light chain Vκ and Jκ gene segments operably linked to an endogenous mouse immunoglobulin heavy chain Cκ region (1293) (see, Figure 7). The 20187 allele is created upon electroporation and proper homologous recombination of a 6011 allele with the 12:D_{H}2-2:23|12:D_{H}2-8:23|12:D_{H}2-15:23 /J_{H}1-6 targeting vector comprising the neomycin cassette. (Figure 7). These engineered mouse ES cells were employed to facilitate efficient screening of positive ES clones (see below) and subsequent cre-mediated removal of the neomycin drug resistance cassette, after which removal the 20187 allele is to as 20188 (Figure 8).

### Example 2. ES cell screening

This example demonstrates the production of non-human animals (e.g., rodents) whose genome comprises an immunoglobulin heavy chain variable region that includes an engineered heavy chain diversity (D_{H}) region, wherein the engineered D_{H} region includes one or more D_{H} segments that are each operably linked to a 23-mer RSS.

Correct assembly of the targeting vectors described in Example 1 and targeted insertion of the DNA fragments into the diversity cluster of a humanized immunoglobulin heavy chain locus contained with BAC DNA clones was confirmed by sequencing and polymerase chain reaction during the construction each targeting vector. Targeted BAC DNA, confirmed by polymerase chain reaction, was then introduced into mouse embryonic stem (ES) cells via electroporation followed by culturing in selection medium. The genome of the ES cells used for electroporation of each targeting vector is depicted in Figures 3, 5 and 7, respectively. Drug-resistant colonies were picked 10 days after electroporation and screened by TAQMAN^{™} and karyotyping for correct targeting as previously described (Valenzuela et al., *supra*; Frendewey, D. et al., 2010, Methods Enzymol. 476:295-307, which are incorporated herein by reference in its entirety) using primer/probe sets that detected proper integration of the engineered D_{H} gene segments (Table 4; F: forward primer, P: probe, R: reverse primer).

The VELOCIMOUSE^{®} method (DeChiara, T.M. et al., 2010, Methods Enzymol. 476:285-294; DeChiara, T.M., 2009, Methods Mol. Biol. 530:311-324; Poueymirou et al., 2007, Nat. Biotechnol. 25:91-99; which are incorporated by reference in their entireties herein) was used, in which targeted ES cells were injected into uncompacted 8-cell stage Swiss Webster embryos, to produce healthy fully ES cell-derived F0 generation mice heterozygous for the engineered D_{H} region and that express antibodies containing heavy chain variable regions that include CDR3 regions generated from D_{H}-D_{H} recombination. F0 generation heterozygous male were crossed with C57Bl6/NTac females to generate F1 heterozygotes that were intercrossed to produce F2 generation homozygotes and wild-type mice.

The drug selection cassette may optionally be removed by the subsequent addition of a recombinase (e.g., by Cre treatment) or by breeding to a Cre deleter mouse strain (see, e.g., International Patent Application Publication No. WO 2009/114400, incorporated herein in its entirety by reference) in order to remove any loxed selected cassette introduced by the targeting construct that is not removed, e.g., at the ES cell stage or in the embryo. Optionally, the selection cassette is retained in the mice. Selection cassettes engineered into targeting vectors described herein were removed in positive ES cells by transient expression of Cre recombinase (see Figures 4, 6 and 8, respectively).

**Table 4. Primer/probe sets for ES cell screening**

| Name | | Sequence (5'-3') | SEQ ID NO |
|---|---|---|---|
| hIgHJ2 | F | GAGGCTGTGCTACTGGTACTTC | 1 |
| | P | CCGTGGCACCCTGGTCACTGT | 2 |
| | R | TGGTGCCTGGACAGAGAAG | 3 |
| | | | |
| VI741-42h1 | F | TTTTTGTGCACCCCTTAATGG | 4 |
| | P | ATGTGGTATTACGATTTTTGGA | 5 |
| | R | CTCTGTGACACTGTGGGTATAATAACC | 6 |
| | | | |
| VI742h2 | F | TGTCACAGAGTCCATCAAAAACCT | 7 |
| | P | CACCACCCCCTCTC | 8 |
| | R | CCTGAGACCCTGGCAAGCT | 9 |
| | | | |
| Neo | F | GGTGGAGAGGCTATTCGGC | 10 |
| | P | TGGGCACAACAGACAATCGGCTG | 11 |
| | R | GAACACGGCGGCATCAG | 12 |
| | | | |
| Jxn D_{H}2-2 | F | ATGGCGCTTGCAGCGATTC | 13 |
| | P | CCAATACTAACGAGGAAATGCAAACCCA | 14 |
| | R | ACCCTCACTGTTGTCCTTCTG | 15 |
| | | | |
| Jxn D_{H}2-8 | F | GCAAACGTCCATCTGAAGGAGAA | 16 |
| | P | CAAATAAACGATGGCAGGCTACACCCG | 17 |
| | R | GAGTTGCCGAACCGACTTATTG | 18 |
| | | | |
| Jxn D_{H}2-15 | F | GCAATGGTAGGTTCATGTCCATC | 19 |
| | P | ACCGCCGCTGGTACTCCAAT | 20 |
| | R | CCCTGGCTGTCTGGGTTTG | 21 |
| | | | |
| hIgHJ6 | F | CAGCAGAGGGTTCCATGAGAA | 22 |
| | P | CAGGACAGGGCCACGGACAGTC | 23 |
| | R | GCCACCCAGAGACCTTCTGT | 24 |
| | | | |
| hIgH31 | F | ATCACACTCATCCCATCCCC | 25 |
| | P | CCCTTCCCTAAGTACCACAGAGTGGGCTC | 26 |
| | R | CACAGGGAAGCAGGAACTGC | 27 |
| | | | |
| mIgHp2 | F | GCCATGCAAGGCCAAGC | 28 |
| | P | CCAGGAAAATGCTGCCAGAGCCTG | 29 |
| | R | AGTTCTTGAGCCTTAGGGTGCTAG | 30 |
| | | | |
| mADAM6-1 | F | CTGAGCATGGTGTCCCATCT | 31 |
| | P | TGCAGATAAGTGTCATCTGGGCAA | 32 |
| | R | CCAGAGTACTGTAGTGGCTCTAAG | 33 |
| | | | |
| hIgk5 | F | CCCCGTCCTCCTCCTTTTTC | 34 |
| | P | TCATGTCCATTAACCCATTTACCTTTTGCCCA | 35 |
| | R | TGCAAGTGCTGCCAGCAAG | 36 |
| | | | |
| hIgK21 | F | CATTTGGCTACATATCAAAGCCG | 37 |
| | P | CCTGAGCCAGGGAACAGCCCACTGATA | 38 |
| | R | ACATGGCTGAGGCAGACACC | 39 |
| | | | |
| mIgKd2 | F | GCAAACAAAAACCACTGGCC | 40 |
| | P | CTGTTCCTCTAAAACTGGACTCCACAGTAAATGGAAA | 41 |
| | R | GGCCACATTCCATGGGTTC | 42 |
| | | | |
| hyg | F | TGCGGCCGATCTTAGCC | 43 |
| | P | ACGAGCGGGTTCGGCCCATTC | 44 |
| | R | TTGACCGATTCCTTGCGG | 45 |
| | | | |
| hIgHD-J.1 | F | TGGCAGCTGTTCAACCATGT | 46 |
| | P | ATCCACTGTCCCAGACAGCACC | 47 |
| | R | GGCGGTTTGTGGAGTTTCCT | 48 |
| | | | |
| hIgHD-J.2 | F | TGTTGCCTCCCTGCTTCTAG | 49 |
| | P | CCCAGACCCTCCCTTGTTCCTGA | 50 |
| | R | GGCCCACGCATTGTTCAG | 51 |

### Example 3. Characterization of mice modified with the 23:D_{H}3-3:12/J_{H}6, 23:D_{H}3-3:12/J_{H}4-6, or 12:D_{H}2-2:23|12:D_{H}2-8:23|12:D_{H}2-15:23 /J_{H}1-6 targeting vectors

### Immunophenotyping

Immunophenotypic analysis of animals modified with 23:D_{H}3-3:12/J_{H}6 or 23:D_{H}3-3:12/J_{H}4-6 targeting vectors was performed for mice heterozygous for the respective 6800 or 6795 modified alleles (see Figures 4 and 6). Immunophenotypic analysis of animals modified with the 12:D_{H}2-2:23|12:D_{H}2-8:2-23| 12:D_{H}2-15:23/J_{H}1-6 targeting vector was performed for mice bred to homozygosity for the 20188 modified allele. B cell development was analyzed in these animals by fluorescence activated cell sorting (FACS). Briefly, spleens and leg bones (femur and tibia) were harvested from:
VELOCIMMUNE^{®} mice (n=3, 26% C57BL/6 23% 129S6/SvEvTac 51% Balb/cAnNTac; see, e.g., U.S. Patent Nos. 8,502,018 and 8,642,835),
6643het/6800het//1293ho mice (25% C57BL/6NTac 25% 129S6/SvEvTac 50% Balb/cAnNTac modified with the 23:D_{H}3-3:12/J_{H}6 targeting vector; see Figure 4; n=3;
6643het/6798het//1293ho mice (25% C57BL/6NTac 25% 129S6/SvEvTac 50% Balb/cAnNTac modified with the 23:D_{H}3-3:12/J_{H}4-6 targeting vector; see Figure 6; n=3); or
20188ho/1293ho mice (25% C57BL/6NTac 25% 129S6/SvFvTac 50% Balb/cAnNTac modified with the 12:D_{H}2-2:23|12:D_{H}2-8:23|12:D_{H}2.1523/J_{H}1-6 targeting vector; n= 3).
Bone marrow was collected from femurs by centrifugation. Red blood cells from spleen and bone marrow preparations were lysed with ACK lysis buffer (Gibco) followed by washing with 1xPBS with 2% FBS. Isolated cells (1×10⁶) were incubated with selected antibody cocktails for 30 min at +4°C: Stain 1: rat anti-mouse CD43-FITC (Biolegend 121206, clone 1B11), rat anti-mouse c-kit-PE (Biolegend 105808, clone 2B8), rat anti-mouse IgM-PeCy (eBiosciences 25-5790-82, clone II/41), rat anti-mouse IgD-PerCP-Cy5.5 (Biolegend 405710, clone 11-26c.2a), rat anti-mouse CD3-PB (Biolegend 100214, clone 17-A2), rat anti-mouse B220-APC (eBiosciences 17-0452-82, clone RA3-6B2), and rat anti-mouse CD19-APC-H7 (BD 560143 clone 1D3). Stain 2: rat anti-mouse kappa-FITC (BD 550003, clone 187.1), rat anti-mouse lambda-PE (Biolegend 407308, clone RML-42), rat anti-mouse IgM-PeCy (eBiosciences 25-5790-82, clone II/41), rat anti-mouse IgD-PerCP-Cy5.5 (Biolegend 405710, clone 11-26c.2a), rat anti-mouse CD3-PB (Biolegend 100214, clone 17-A2), rat anti-mouse B220-APC (eBiosciences 17-0452-82, clone RA3-6B2), and rat anti-mouse CD19-APC-H7 (BD 560143 clone 1D3). Following staining, cells were washed and then fixed in 2% formaldehyde. Data acquisition was performed on a BD LSRFORTESSA^{™} flow cytometer (BD Biosciences) and analyzed with FLOWJO^{™} software (BD Biosciences).

In mice heterozygous for the 6800 or 6798 alleles, total B cell numbers were decreased in both the spleen and bone marrow, and there was an increase in pro-B cells observed in the bone marrow when compared to control VELOCIMMUNE^{®} mice (data not shown). Overall, the B cells had similar κ and λ usage (data not shown). In mice homozygous for the 20188 allele, a slightly higher level of λ⁺ B cells in the spleen were seen (data not shown). None of the differences observed in the B cell populations in mice expressing the 6800, 6798, or 20188 alleles compared to control VELOLCIMMUNE^{®} mice were considered to affect B cell development of these animals (data not shown).

### Frequencies of D_{H}-D_{H} rearrangements and characteristics

Gene rearrangements in unimmunized naïve mice modified with the 23:D_{H}3-3:12/J_{H}6 targeting vector, the 23 :D_{H}3-3: 12/J_{H}4-6 targeting vector, or the 12:D_{H}2-2:23|12:D_{H}2-8:23|12:D_{H}2-15:23 targeting vector were analyzed by IgM repertoire sequencing. IgM repertoire library from spleen B cells was prepared using a SMARTer^{™} RACE (rapid amplification of cDNA ends) cDNA Amplification Kit (Clontech) with primers specific to the mouse constant IgM (Table 5, "nnnnnn" represents a 6bp index sequences to enable multiplexing samples for sequencing). Prepared repertoire library was then sequenced on a MiSeq sequencer (Illumina).

Illumina MiSeq paired-end sequences (2×300 cycles) were merged and filtered based on quality and perfect match to the heavy chain IgM constant region primer. Rearranged heavy chain sequences were aligned to germline V, D, and J reference databases (IMGT). Subsequent analyses only included productive rearrangements, defined as sequences with no stop codons within the open reading frame and an in-frame VDJ junction.

**Table 5: Primers used in library preparation for IgM repertoire sequencing**

| **RT primers** | **Oligo-dT (SEQ ID NO)** | **SEQUENCE** |
|---|---|---|
| **Template switching oligo** | **(SEQ ID NO:146)** | 5' -AGCAGTGGTATCAACGCAGAGTACATGGG-3' |
| **1^{st} round PCR primers** | **IgM Constant (SEQ ID NO: 147)** | 5′ - ACACTCTTTCCCTACACGACGCTCTTCCGATCTG GGAAGACATTTGGGAAGGAC - 3′ |
| | **PE2-PIIA (SEQ ID NO: 148)** | 5′ - GTGACTGGAGTTCAGACGTGTGCTCTTCCGATCT AAGCAGTGGTATCAACGCAGAGT - 3′ |
| **2^{nd} round PCR Primers** | **Forward (SEQ ID NO: 149)** | 5′ - AATGATACGGCGACCACCGAGATCTACACnnnnn nACACTCTTTCCCTACACGACGCTCTTCCGATCT- 3′ |
| | **Reverse (SEQ ID NO: 150)** | 5′ - CAAGCAGAAGACGGCATACGAGATnnnnnnGTGA CTGGAGTTCAGACGTGTGCTCTTCCGATCT- 3′ |

In unimmunized animals modified with the 23:D_{H}3-3:12/J_{H}6 targeting vector or the 23:D_{H}3-3:12/J_{H}4-6 targeting vector, no expression of D_{H}7-27 was detected, and J_{H} usage was respectively limited to J_{H}6, or J_{H}4, J_{H}5, and J_{H}6, as expected (data not shown). In unimmunized animals modified with the 12:D_{H}2-2:23|12:D_{H}2-8:23|12:D_{H}2-15:23 targeting vector, recombination with all J_{H}1-6 gene segments was detected, although recombination with J_{H}4 was seen at higher frequencies (about 50%) in both the modified and control animals (data not shown). In all modified animals, a variety of V_{H} gene segments were used (e.g., but not limited to V_{H}4-39, V_{H}3-23, etc.) and a variety of D_{H} gene segments (e.g., but not limited to D_{H}1-7, D_{H}3-10, and D_{H}5-12) recombined with the engineered D_{H} gene segments. Interestingly, usage of D_{H}2-2, D_{H}2-8, and D_{H}2-15 gene segments trended higher in animals modified with the 12:D_{H}2-2:23|12:D_{H}2-8:23|12:D_{H}2-15:23 targeting vector (2.41%; 6.34%; 6.91%; n=3) compared to control animals (2.57%, 3.44%; 3.58%; n=3). (Table 6).

Functional sequences with a minimum of two consecutive D_{H} segments identified within the junction region were further confirmed as resulting from a D_{H}-D_{H} recombination event using the following criteria. In sequences isolated from mice modified with the 23 :D_{H}3-3:12/J_{H}6 targeting vector or the 23 :D_{H}3-3:12/J_{H}4-6 targeting vector, a D_{H}-D_{H} recombination event was confirmed when (1) the identified "successive" or 3' D_{H} segment aligned with a minimum of 9 consecutive base pairs to a germline sequence of the IGHD3-3 D_{H} gene segment and (2) both the identified leading 5' and successive 3' D_{H} gene segments aligned with a minimum of 5 consecutive base pairs to a germline D_{H} gene segment, irrespective of any overlap. In sequences isolated from mice modified with the 12:D_{H}2-2:23|12:D_{H}2-8:23|12:D_{H}2-15:23 targeting vector, a D_{H}-D_{H} recombination event was confirmed when (1) the identified "leading" or 5' D_{H} segment aligned with a minimum of 9 consecutive base pairs to a germline sequence of the IGHD2-2 D_{H} gene segment, the IGHD2-8 D_{H} gene segment, or the IGHD2-15 D_{H} gene segment, and (2) both the identified leading 5' and successive 3' D_{H} gene segments aligned with a minimum of 5 consecutive nucleotides to a germline D_{H} gene segment, irrespective of any overlap.

Table 6 provides the number of functional Ig reads as an absolute amount and percent of total reads, the percentage of all functional reads derived from a D_{H}2-2 gene segment, a D_{H}2-8 gene segment, or a D_{H}2-15 gene segment, and the percentage of all reads derived from a D_{H}2-2 gene segment, a D_{H}2-8 gene segment, or a D_{H}2-15 gene segment that satisfy the criteria to be confirmed as the result of a D_{H}-D_{H} recombination event.

**Table 6**

| Mouse | % D2-2/D2-8/D2-15 usage | % D_{H}-D_{H} fusions |
|---|---|---|
| Control | 3.44 | 0.20* |
| Control | 3.58 | 0.10* |
| Control | 2.57 | 0.08* |
| 20188 S1 | 6.34 | 3.80 |
| 20188 S2 | 2.41 | 1.69 |
| 20188 S3 | 6.91 | 4.16 |

| | | |
|---|---|---|
| *D_{H}-D_{H} fusion in each of these control animals were confirmed using the same criteria set forth for those animals modified with the 12:D_{H}2-2:23\|12:D_{H}2-8:23\|12:D_{H}2-15:23 targeting vector. | | |

As shown in Table 6, all unimmunized modified animals showed an increase in D_{H}-D_{H} recombination events compared to the control animals. In animals modified with engineered 2-2, 2-8 and 2-15 gene segments, e.g., homozygous for the 20188 allele, D_{H}-D_{H} recombination events in 1.69% to 4.16% of all rearrangements comprising one of the three engineered 2-2, 2-8, 2-15 D_{H} gene segments. In contrast, D_{H}-D_{H} recombination was confirmed in less than 0.2% of all rearrangements derived from 2-2, 2-8, or 2-15 gene segment in control animals. In a separate experiment, the frequency of confirmed D_{H}-D_{H} recombination events involving an engineered D_{H}3-3 gene segment in mice heterozygous for the 6800 mice (Figure 4) ranged from 0.6 to 1.2% of all functional reads derived from a D_{H}3-3 gene segment.

In all modified animals, the rearranged V_{H}(D_{H}A-D_{H}B)J_{H} rearrangements encoded longer CDR3 lengths (e.g. but not limited to CDR3 lengths of about 21 amino acids) and incorporated more cysteine residues, (e.g., Figures 9 and 10).

In summary, mice modified with an engineered D_{H} gene segment exhibit a relatively normal B cell phenotype with an increased frequency of D_{H}-D_{H} recombination events due to the utilization of the engineered D_{H} gene segment. Additionally, the D_{H}-D_{H} recombined sequences encoded for CDR3 that were longer in length and had a higher rate of cysteine incorporation.

### Example 4. Production of antibodies

This example demonstrates production of antibodies in a rodent whose genome comprises an immunoglobulin heavy chain variable region that includes an engineered D_{H} region as described herein. The methods described in this example, and/or immunization methods well known in the art, can be used to immunize rodents containing an engineered D_{H} region as described herein with polypeptides or fragments thereof (e.g., peptides derived from a desired epitope), or a combination of polypeptides or fragments thereof, as desired.

Briefly, cohorts of mice that include an engineered D_{H} region as described herein are immunized with an antigen of interest using immunization methods known in the art. The antibody immune response (i.e., serum titer) is monitored by an ELISA immunoassay and/or on engineered cells by MSD. When a desired immune response is achieved, splenocytes (and/or other lymphatic tissue) are harvested and fused with mouse myeloma cells to preserve their viability and form immortal hybridoma cell lines. The hybridoma cell lines are screened (e.g., by an ELISA assay or MSD) and selected to identify hybridoma cell lines that produce antigen-specific antibodies. Hybridomas may be further characterized for relative binding affinity and isotype as desired. Using this technique, several antigen-specific chimeric antibodies (i.e., antibodies possessing human variable domains and rodent constant domains) are obtained.

DNA encoding the variable regions of heavy chain and light chains may be isolated and linked to desirable isotypes (constant regions) of the heavy chain and light chain for the preparation of fully-human antibodies. Such an antibody may be produced in a cell, such as a CHO cell. Fully human antibodies are then characterized for relative binding affinity and/or neutralizing activity of the antigen of interest.

DNA encoding the antigen-specific chimeric antibodies, or the variable domains of light and heavy chains, may be isolated directly from antigen-specific lymphocytes. Initially, high affinity chimeric antibodies are isolated having a human variable region and a rodent constant region and are characterized and selected for desirable characteristics, including, but not limited to, affinity, selectivity, epitope, etc. Rodent (e.g., but not limited to, rat, mouse, etc.) constant regions are replaced with a desired human constant region to generate fully-human antibodies. While the constant region selected may vary according to specific use, high affinity antigen-binding and target specificity characteristics reside in the variable region. Antigen-specific antibodies are also isolated directly from antigen-positive B cells (from immunized mice) without fusion to myeloma cells, as described in, e.g., U.S. Patent No. 7,582,298, incorporated herein by reference in its entirety. Using this method, several fully human antigen-specific antibodies (i.e., antibodies possessing human variable domains and human constant domains) are made.

In one experiment, control mice (e.g., VELOCIMMUNE^{®} mice with humanized immunoglobulin heavy and κ light chain variable region loci (n= 4-6; see, e.g., U.S. Patent Nos. 8,697,940 and 8,642,835; each of which are herein incorporated by reference in its entirety)) and test mice (e.g., mice modified to comprise an engineered 23:D_{H}3-3:12/J_{H}4-6 or 23:D_{H}3-3:12/J_{H}6 region as described herein (n= 4-6)) were immunized with different antigens and/or nucleic acids encoding same (a G protein-coupled receptor (GPCR), an ion channel, a chemokine receptor, or a pathogen).

Mice were able to generate responses to each of the tested antigens, with responses ranging from low titers to high specific titers (data not shown). Mice having an engineered 23:D_{H}3-3:12/J_{H}6 region as described herein demonstrated titers to a GPCR expressing cell line comparable to control mice having humanized immunoglobulin heavy and κ light chain variable region loci (Figure 11; filled circles represent an engineered cell line that expresses GPCR; unfilled circles are control cells that do not express GPCR).

Additionally, 100% of 23 tested antibodies specific to a G protein-coupled receptor (GPCR) that were generated in mice comprising an engineered D_{H} region had a heavy chain CDR3 length of 10 or more amino acids; 20% had a CDR3 length of 14 amino acids and 1 antibody had a CDR3 length of 18 amino acids (data not shown). In contrast, only about 60% of the 8 tested antibodies specific to the GPCR that were generated in mice having humanized immunoglobulin heavy and κ light chain variable region loci, but not an engineered D_{H} region, had CDR3 lengths of 10 amino acids or more, none of which had CDR3 lengths of 14 or more (data not shown).

A plurality of antiviral antibodies was isolated from a mouse having an engineered 23 :D_{H}3-3:12/J_{H}4-6 region, with at least one demonstrating a neutralization activity superior to that of the comparator molecule (data not shown).

Antibodies from a mouse having an engineered 23:D_{H}3-3:12/J_{H}6 and immunized with an ion channel comprised a population of antibodies having HCDR3 lengths of greater than 21 amino acids stemming from both V_{H}D_{H}J_{H} and V_{H}D_{H}A-D_{H}BJ_{H} rearrangement (as determined by stringent criteria described for this example), with the sequences encoding longer HCDR3 lengths being found more in the bone marrow compared to the spleen (Figure 12). Analysis of CDR3 lengths encoded by sequences determined to be the result of V_{H}D_{H}A-D_{H}BJ_{H} rearrangement according to the stringent criteria of this example (when using the stringent criteria of this example each D_{H} gene segment detected must display a minimum of 40% identity to a germline D gene segment, the 3' D_{H} gene segment must be derived from the D_{H}3-3 gene segment, and a maximum of 1 nucleotide mutation in each D_{H} gene segment) is shown in Figure 13. Table 7 below provides a comparison of the frequency of V_{H}D_{H}A-D_{H}BJ_{H} recombination in the bone marrow or spleen using the stringent criteria described in this example, or non-stringent criteria in this example (when using the non-stringent criteria of this example each D_{H} gene segment detected must display a minimum of 5 nucleotide identity to a germline D gene segment, the 3' D_{H} gene segment must be derived from the D_{H}3-3 gene segment).

**Table 7: V_{H}D_{H}A-D_{H}BJ_{H} recombination frequency**

| | Non-stringent Criteria | Stringent Criteria |
|---|---|---|
| Bone Marrow | 3.54 | 1.7 |
| Spleen | 0.38 | 0.04 |

### EQUIVALENTS

Having thus described several aspects of at least one embodiment of this invention, it is to be appreciated by those skilled in the art that various alterations, modifications, and improvements will readily occur to those skilled in the art. Such alterations, modifications, and improvements are intended to be part of this disclosure, and are intended to be within the spirit and scope of the invention. Accordingly, the foregoing description and drawing are by way of example only and the invention is described in detail by the claims that follow.

Those skilled in the art will appreciate typical standards of deviation or error attributable to values obtained in assays or other processes described herein. The publications, websites and other reference materials referenced herein to describe the background of the invention and to provide additional detail regarding its practice are hereby incorporated by reference in their entireties.

### FURTHER NUMBERED EMBODIMENTS

1. A nucleotide molecule comprising an engineered heavy chain diversity (D_{H}) gene segment that comprises at least one D_{H} gene segment operably linked to a 23-mer recombination signal sequence (RSS).
2. The nucleotide molecule of [1], wherein the nucleotide sequence comprises an engineered D_{H} region comprising (i) the at least one D_{H} gene segment operably linked to a 23-mer RSS and (ii) an unrearranged D_{H} gene segment flanked on one side by a 12-mer RSS and on the other side by another12-mer RSS, and
   wherein the (i) at least one D_{H} gene segment operably linked to a 23-mer RSS and the (ii) unrearranged D_{H} gene segment flanked on one side by a 12-mer RSS and on the other side by another12-mer RSS are operably linked such that (i) and (ii) are able to join in a D_{H}-D_{H} recombination event according to the 12/23 rule,
   optionally wherein the engineered D_{H} region comprises only human D_{H} gene segments.
3. The nucleotide molecule of [2], wherein the nucleotide molecule further comprises:
   (a) at least one unrearranged immunoglobulin heavy chain variable (V_{H}) gene segment that is upstream of and operably linked to the engineered D_{H} region,
   (b) at least one unrearranged immunoglobulin heavy chain joining (J_{H}) gene segment that is downstream of and operably linked to the engineered D_{H} region, or
   (c) a combination of (a) and (b).
4. The nucleotide molecule of [3], wherein the at least one V_{H} gene segment comprises an unrearranged human V_{H}6-1 gene segment, the at least one J_{H} gene segment comprises an unrearranged human J_{H}6 gene segment, or a combination thereof.
5. The nucleotide molecule of [3] or [4], wherein the at least one unrearranged J_{H} gene segment comprises an unrearranged human J_{H}4 gene segment, an unrearranged human J_{H}5 gene segment, and an unrearranged human J_{H}6 gene segment.
6. The nucleotide molecule of any one of [3-5], wherein the at least one unrearranged J_{H} gene segment comprises an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment, optionally wherein the unrearranged human J_{H}1, J_{H}2, J_{H}3, J_{H}4, J_{H}5, and J_{H}6 gene segments are in germline configuration.
7. The nucleotide molecule of any one of [3-6], wherein at least one unrearranged V_{H} gene segment comprises the full repertoire of functional unrearranged human V_{H} gene segments spanning between and including the unrearranged human V_{H}3-74 and unrearranged human V_{H}1-6 gene segments, optionally in germline configuration.
8. The nucleotide molecule of any one of the preceding [1-7], further comprising one or more functional rodent Adam6 genes, optionally wherein the one or more functional rodent Adam6 genes is located between a human V_{H}1-2 gene segment and a human V_{H}6-1 gene segment and/or replaces a human Adam6 gene.
9. The nucleotide molecule of any one of the preceding [1-8], wherein the 23-mer RSS is contiguous to the 5'-end of the at least one D_{H} gene segment.
10. The nucleotide molecule of any one of the preceding [1-9], wherein the 23-mer RSS is contiguous to the 3'-end of the at least one D_{H} gene segment.
11. The nucleotide molecule of any one of the preceding [1-10], wherein the at least one D_{H} gene segment operably linked to a 23-mer RSS comprises:
   (a) a human D_{H}3-3 gene segment operably linked to a 23-mer RSS, optionally wherein the 23-mer RSS is contiguous to the 5'-end of the D_{H}3-3 gene segment,
   (b) a human D_{H}2-2 gene segment operably linked to a 23-mer RSS, optionally wherein the 23-mer RSS is contiguous to the 3'-end of the D_{H}2-2 gene segment,
   (c) a human D_{H}2-8 gene segment operably linked to a 23-mer RSS, optionally wherein the 23-mer RSS is contiguous to the 3'-end of the D_{H}2-8 gene segment,
   (d) a human 2-15 gene segment operably linked to a 23-mer RSS, optionally wherein the 23-mer RSS is contiguous to the 3'-end of the D_{H}2-15 gene segment, or
   (e) any combination of (a)-(d).
12. The nucleotide molecule of any one of the preceding [1-11], comprising a nucleotide sequence comprising the sequence set forth as SEQ ID NO:52.
13. The nucleotide molecule of any one of [1-11], comprising a nucleotide sequence comprising the sequence set forth as SEQ ID NO:61.
14. The nucleotide molecule of any one of [1-13], comprising a nucleotide sequence comprising the nucleotide sequence set forth as SEQ ID NO:70.
15. The nucleotide molecule of any one of [1-14], comprising a nucleotide sequence comprising the nucleotide sequence set forth as SEQ ID NO:71.
16. The nucleotide molecule of any one of [1-15], comprising a nucleotide sequence comprising the nucleotide sequence set forth as SEQ ID NO:72.
17. A nucleotide molecule comprising in operable linkage from 5' to 3':
   (a) one or more unrearranged human V_{H} gene segments;
   (b) an engineered D_{H} region comprising from 5' to 3'
      (i) one or more unrearranged human D_{H} gene segments, wherein each of the one or more of human D_{H} gene segments is flanked on its 5' and 3' ends by a 12-mer RSS, and
      (ii) at least one D_{H} gene segment operably linked to a 23-mer RSS comprising a human D_{H}3-3 gene segment operably linked at its 5'-end to a 23-mer RSS; and
   (c) one or more unrearranged human J_{H} gene segments.
18. A nucleotide molecule comprising in operable linkage from 5' to 3':
   (a) one or more unrearranged human V_{H} gene segments;
   (b) an engineered D_{H} region, wherein the engineered D_{H} region comprises from 5' to 3'
      (i) at least one D_{H} gene segment operably linked to a 23-mer RSS comprising at least one human D_{H}2 gene segment operably linked at its 3' end to a 23-mer RSS, optionally wherein the at least one human D_{H}2 gene segment operably linked at its 3'end to a 23-mer RSS comprises a human D_{H}2-2 gene segment operably linked at its 3' end to a 23-mer RSS, a human D_{H}2-8 gene segment operably linked at its 3' end to a 23-mer RSS, a human D_{H}2-15 gene segment operably linked at its 3' end to a 23-mer RSS, or any combination thereof, and
      (ii) one or a plurality of human D_{H} gene segments, wherein each of the one or plurality of human D_{H} gene segments is flanked on its 5' and 3' ends by a 12-mer RSS; and
   (c) one or more unrearranged human J_{H} gene segments.
19. The nucleotide molecule of [17] or [18], wherein the one or more unrearranged human V_{H} gene segments comprises from 5' to 3' an unrearranged human V_{H}1-2 gene segment and an unrearranged human V_{H}6-1 gene segment, wherein the nucleotide further comprises one or more functional rodent Adam6 genes between the unrearranged human V_{H}1-2 gene segment and the unrearranged human V_{H}6-1 gene segment, and wherein the unrearranged human V_{H}1-2 gene segment, the one or more rodent Adam6 genes, and the unrearranged human V_{H}6-1 gene segment are contiguous.
20. The nucleotide molecule of any one of [17-19], wherein the one or more unrearranged human V_{H} gene segments comprises the full repertoire of functional unrearranged human V_{H} gene segments spanning between and including the unrearranged human V_{H}3-74 and unrearranged human V_{H}1-6 gene segments, optionally in germline configuration.
21. The nucleotide molecule of any one of [17-20], wherein the one or more unrearranged human J_{H} gene segments comprises an unrearranged human J_{H}6 gene segment.
22. The nucleotide molecule of [21], wherein the one or more unrearranged human J_{H} gene segments comprises an unrearranged human J_{H}4 gene segment, an unrearranged human J_{H}5 gene segment, and the unrearranged human J_{H}6 gene segment.
23. The nucleotide molecule of [22], wherein the one or more unrearranged human J_{H} gene segments comprises an unrearranged human J_{H}1 gene segment, an unrearranged human J_{H}2 gene segment, an unrearranged human J_{H}3 gene segment, the unrearranged human J_{H}4 gene segment, the unrearranged human J_{H}5 gene segment, and the unrearranged human J_{H}6 gene segment, optionally wherein the unrearranged human J_{H}1, J_{H}2, J_{H}3, J_{H}4, J_{H}5, and J_{H}6 gene segments are in germline configuration.
24. The nucleotide molecule of any one of [1-23], further comprising at its 3'-end a heavy chain immunoglobulin constant region (C_{H}) or a portion thereof.
25. The nucleotide molecule of [24], wherein the C_{H} is a rodent C_{H} region or a portion thereof.
26. The nucleotide molecule of [25], wherein the rodent C_{H} region or a portion thereof comprises a rodent intronic enhancer region and a rodent IgM gene.
27. The nucleotide molecule of any one of [1-26], further comprising a drug selection cassette,
   wherein the drug selection cassette is located upstream of the D_{H} gene segment operably linked to a 23-mer RSS, and
   wherein the drug selection cassette is optionally flanked by one or more site-specific recombination sites.
28. A targeting vector comprising a nucleotide molecule of any one of [1-27], comprising 5'- and 3'- homology arms configured to allow homologous recombination with an immunoglobulin heavy chain sequence, optionally wherein the immunoglobulin heavy chain sequence is at an endogenous rodent immunoglobulin heavy chain locus, comprises a human or humanized immunoglobulin heavy chain variable region, or a combination thereof.
29. A method of modifying an immunoglobulin heavy chain variable region to engineer D_{H}-D_{H} recombination, the method comprising:
   obtaining an immunoglobulin heavy chain variable region comprising a D_{H} region comprising one or more unrearranged D_{H} gene segments each of which unrearranged D_{H} gene segments are flanked on one side by a 12-mer RSS and on the other side by another12-mer RSS, and
   modifying the D_{H} region to further comprise at least one D_{H} gene segment operably linked to a 23-mer RSS.
30. The method of [29], wherein the one or more unrearranged D_{H} gene segments comprises one or more unrearranged human D_{H} gene segments.
31. The method of [30], wherein the one or more unrearranged D_{H} gene segments comprises all functional germline human D_{H} gene segments spanning between, and including, the germline human D_{H}1-1 and the germline human D_{H}7-27 gene segments, optionally in germline configuration.
32. The method of any one of [29-31], wherein modifying comprises replacing
   (a) at least one of the one or more unrearranged D_{H} gene segments with a D_{H} gene segment operably linked to a 23-mer RSS,
   (b) at least one of the two 12-mer RSS flanking an unrearranged D_{H} gene segment with a 23-mer RSS, or
   (c) a combination of (a) and (b).
33. The method of [32], wherein the immunoglobulin heavy chain variable region further comprises a J_{H} region comprising at least one unrearranged J_{H} gene segment,
   wherein the J_{H} region is operably linked to the D_{H} region, and
   wherein replacing at least one of the one or more unrearranged D_{H} gene segments with the D_{H} gene segment operably linked to the 23-mer RSS comprises deleting at least one unrearranged J_{H} gene segment comprised in the J_{H} region.
34. The method of [33], wherein the J_{H} region comprises a full repertoire of human germline J_{H} gene segments comprising a human germline J_{H}1 gene segment, a human germline J_{H}2 gene segment, a human germline J_{H}3 gene segment, a human germline J_{H}4 gene segment, a human germline J_{H}5 gene segment, and a human germline J_{H}6 gene segment, optionally wherein the human germline J_{H}1, J_{H}2, J_{H}3, J_{H}4, J_{H}5, and J_{H}6 gene segments are in germline configuration, and
   wherein deleting at least one germline J_{H} gene segment comprised in the J_{H} region comprises deleting the unrearranged human J_{H}1, J_{H}2, and J_{H}3 gene segments, and optionally further deleting the J_{H}4 and J_{H}5 gene segments.
35. The method of any one of [29 to 34], wherein the at least one D_{H} gene segment operably linked to a 23-mer RSS comprises
   (a) a human D_{H}3-3 gene segment operably linked to a 23-mer RSS, optionally wherein the 23-mer RSS is contiguous to the 5'-end of the D_{H}3-3 gene segment,
   (b) a human D_{H}2-2 gene segment operably linked to a 23-mer RSS, optionally wherein the 23-mer RSS is contiguous to the 3'-end of the D_{H}2-2 gene segment,
   (c) a human D_{H}2-8 gene segment operably linked to a 23-mer RSS, optionally wherein the 23-mer RSS is contiguous to the 3'-end of the D_{H}2-8 gene segment,
   (d) a human D_{H}2-15 gene segment operably linked to a 23-mer RSS, optionally wherein the 23-mer RSS is contiguous to the 3'-end of the D_{H}2-15 gene segment, or
   (e) any combination of (a)-(d).
36. The method of any one of [32-35], wherein the at least one unrearranged D_{H} gene segment that is replaced comprises the most 3' unrearranged D_{H} gene segment of the D_{H} region.
37. The method of any one of [32-36], wherein the at least one unrearranged D_{H} gene segment that is replaced comprises a D_{H} gene segment that corresponds to the at least one D_{H} gene segment operably linked to a 23-mer RSS.
38. An immunoglobulin heavy chain locus comprising the nucleotide molecule of any one of [1-27], the targeting vector of [28], or the immunoglobulin heavy chain variable region modified according to the method of any one of [29-37].
39. A rodent genome comprising the nucleic acid of any one of [1-27], the targeting vector of [28], the immunoglobulin heavy chain variable region modified according to the method of any one of [29-37], or the immunoglobulin heavy chain locus of [38], wherein the rodent genome is optionally a rodent germline genome.
40. A rodent germline genome comprising an immunoglobulin heavy chain variable region that comprises:
   (i) unrearranged heavy chain variable (V_{H}) gene segments,
   (ii) an engineered heavy chain variable region diversity (D_{H}) region, wherein the engineered D_{H} region comprises one or more germline D_{H} gene segments and one or more D_{H} gene segments operably linked to a 23-mer recombination signal sequence (RSS), and
   (iii) unrearranged heavy chain joining (J_{H}) gene segments,
   wherein (i)-(iii) are in operable linkage such that, upon recombination, the immunoglobulin heavy chain variable region comprises a rearranged heavy chain variable region sequence encoding an immunoglobulin heavy chain variable domain, optionally wherein the rearranged heavy chain variable region sequence is formed after a V_{H}(D_{H}-D_{H})J_{H} recombination event.
41. The rodent germline genome of [40], wherein the one or more D_{H} gene segments operably linked to a 23-mer recombination signal sequence (RSS) comprises a D_{H} segment operably linked to a 3' 23-mer RSS.
42. The rodent germline genome of [40] or [41], wherein the one or more D_{H} gene segments operably linked to a 23-mer recombination signal sequence (RSS) comprises a D_{H} gene segment operably linked to a 5' 23-mer RSS.
43. The rodent germline genome of any one of [40-42], wherein the immunoglobulin heavy chain variable region is a human immunoglobulin heavy chain variable region comprising only human V_{H}, D_{H}, and J_{H} gene segments.
44. The rodent germline genome of [43], wherein the human immunoglobulin heavy chain variable region is operably linked to an immunoglobulin heavy chain constant region.
45. The rodent germline genome of [44], wherein the immunoglobulin heavy chain constant region is an endogenous rodent immunoglobulin heavy chain constant region, optionally at an endogenous rodent immunoglobulin heavy chain locus.
46. The rodent germline genome of any one of [40-45], wherein the unrearranged V_{H} gene segments comprise a full repertoire of functional germline human V_{H} gene segments spanning between and including V_{H}3-74 to V_{H}6-1, optionally in germline configuration.
47. The rodent germline genome of any one of [40-46], wherein the unrearranged heavy chain J_{H} gene segments comprise a human J_{H}6 gene segment.
48. The rodent germline genome of any one of [40-47], wherein rodent germline genome lacks an endogenous Adam6 gene.
49. The rodent germline genome of any one of [40-47], further comprising one or more rodent Adam6 genes, optionally wherein the one or more rodent endogenous Adam6 genes comprises an endogenous rodent Adam6 gene.
50. The rodent germline genome of [49], wherein the one or more rodent Adam6 genes are inserted between two human V_{H} gene segments.
51. The rodent germline genome of [50], wherein the one or more rodent Adam6 genes are inserted between a human V_{H}1-2 gene segment and a human V_{H}6-1 gene segment.
52. The rodent germline genome of [49], wherein the one or more one or more rodent Adam6 genes are inserted in the place of a human Adam6 pseudogene.
53. The rodent germline genome of [49], wherein the one or more one or more rodent Adam6 genes are inserted between a human V_{H} gene segment and a human D_{H} gene segment.
54. The rodent germline genome of any one of [39-53], further comprising a terminal deoxynucleotidyl transferase gene.
55. The rodent germline genome of any one [39-54], characterized in that it is homozygous for the engineered D_{H} region.
56. The rodent germline genome of any one [39-54], characterized in that it is heterozygous for the engineered D_{H} region.
57. The rodent germline genome of any one of [39-56], wherein the rodent is a rat or mouse.
58. A rodent or rodent cell comprising the rodent germline genome of any one of [39-57].
59. The rodent or rodent cell of [58], wherein the rodent or rodent cell is a rat, a mouse, a rat cell, or a mouse cell.
60. The rodent or rodent cell of [58] or [59], wherein the rodent cell is a rodent embryonic stem cell.
61. A rodent or rodent embryo generated from the rodent embryonic stem cell of [60].
62. The rodent, rodent embryo, or rodent cell of any one of [58-61], further comprising a somatic genome that comprises a rearranged heavy chain V_{H}(D_{H}A-D_{H}B)J_{H} coding sequence,
   optionally wherein the rearranged heavy chain V_{H}(D_{H}A-D_{H}B)J_{H} gene segment is operably linked to an IgM constant region gene sequence and/or wherein D_{H}A or D_{H}B is derived from the D_{H} gene segment operably linked to a 23-mer RSS or a portion thereof and at least 9 base pairs of D_{H}A or D_{H}B is identical to at least 9 base pairs of the D_{H} gene segment operably linked to a 23-mer RSS or a portion thereof and each of.
63. The rodent of any one of [58-62], wherein at least 95% of all rearranged heavy chain VDJ coding sequences in the rodent have a CDR3 length of at least 10 amino acids, optionally wherein at least 70% of all rearranged heavy chain VDJ gene sequences in the rodent have a CDR3 length of at least 11 amino acids, optionally at least 15% of all rearranged heavy chain VDJ gene sequences in the rodent have a CDR3 length of at least 14 amino acids.
64. A rodent according to any one of [58-63], characterized in that the rodent exhibits at least a at least a 3-fold increased frequency of D_{H}-D_{H} recombination as compared to the frequency of D_{H}-D_{H} recombination in a reference rodent.
65. A method of making a rodent whose genome comprises an engineered D_{H} region, the method comprising generating a rodent from the embryonic stem cell of [60].
66. A method of making a rodent whose genome comprises an engineered D_{H} region, the method comprising
   (a) modifying the genome of a rodent embryonic stem cell to comprise a DNA fragment comprising one or more D_{H} gene segments each operably linked to a 23-mer RSS, optionally wherein the DNA fragment comprises the nucleotide molecule of any one of [1-27], the targeting vector of [28], immunoglobulin heavy chain variable region modified according to the method of any one of [30-37], or the immunoglobulin heavy chain of [38], and
   (b) generating a rodent using the modified rodent embryonic stem cell of (a).
67. A method of making a rodent whose genome an engineered D_{H} region, the method comprising
   modifying an unrearranged D_{H} region of an immunoglobulin heavy chain variable region to comprise at least one D_{H} segments operably linked to a 23-mer RSS, wherein the unrearranged D_{H} region further comprises one or more germline D_{H} gene segments.
   thereby making said rodent.
68. The method of [67], wherein modifying comprises replacing the one or more unrearranged human D_{H} gene segments, and optionally replacing one or more J_{H} gene segments, with the at least one D_{H} gene segment operably linked to a 23-mer RSS.
69. The method of [67] or [68], wherein the immunoglobulin heavy chain variable region is a human immunoglobulin heavy chain variable region, optionally wherein the human immunoglobulin heavy chain variable region comprises an unrearranged human immunoglobulin heavy chain V_{H} gene cluster comprising at least one V_{H} gene segment, an unrearranged human immunoglobulin heavy chain D_{H} region comprising one or more unrearranged human D_{H} gene segments, and an unrearranged human immunoglobulin heavy chain J_{H} gene cluster comprising at one unrearranged human J_{H} gene segment, wherein the unrearranged human immunoglobulin heavy chain D_{H} region is modified to comprise at least one D_{H} gene segment operably linked to a 23-mer RSS.
70. The method of [69], wherein the modifying step results in the immunoglobulin heavy chain variable region comprising
   (i) the full repertoire of functional human V_{H} gene segments, optionally all the functional human V_{H} gene segments spanning between and including V_{H}3-74 to V_{H}6-1, optionally wherein all the functional V_{H} gene segments are positioned in germline configuration,
   (ii) the full repertoire of unrearranged human D_{H} gene segments except for D_{H}7-27 which is replaced with the at least one D_{H} gene segment operably linked to a 23-mer RSS, and
   (iii) at least an unrearranged human J_{H}6 gene segment, and optionally at least an unrearranged human J_{H}4 gene segment, an unrearranged human J_{H}5 gene segment, and an unrearranged human J_{H}6 gene segment.
71. The method of [70], wherein the at least one D_{H} gene segment operably linked to a 23-mer RSS comprises a D_{H}3-3 gene segment operably linked to a 5'- 23-mer RSS.
72. The method of [69], wherein the modifying step results in the immunoglobulin heavy chain variable region comprising
   (i) a full repertoire of functional human V_{H} gene segments, optionally all functional V_{H} gene segments spanning between and including V_{H}3-74 to V_{H}6-1, optionally wherein all the functional V_{H} gene segments are positioned in germline configuration
   (ii) the full repertoire of unrearranged human D_{H} gene segments except for the unrearranged D_{H}2-2, D_{H}2-8, and D_{H}2-15 gene segments, each of which are replaced with at least one D_{H} gene segment operably linked to a 23-mer RSS, and
   (iii) the full repertoire of unrearranged human J_{H} gene segments, optionally an unrearranged human J_{H}1 gene segment, an unrearranged human J_{H}2 gene segment, an unrearranged human J_{H}3 gene segment, an unrearranged human J_{H}4 gene segment, an unrearranged human J_{H}5 gene segment, and an unrearranged human J_{H}6 gene segment.
73. The method of [72], wherein the at least one D_{H} gene segment operably linked to a 23-mer RSS comprises a human D_{H}2-2 gene segment operably linked to a 3' 23-mer RSS, a human D_{H}2-8 gene segment operably linked to a 3' 23-mer RSS, or a human D_{H}2-15 gene segment operably linked to a 3' 23-mer RSS, optionally wherein the unrearranged D_{H}2-2, D_{H}2-8, and D_{H}2-15 gene segments are respectively replaced with the human D_{H}2-2 gene segment operably linked to a 3'-end 23-mer RSS, the human D_{H}2-8 gene segment operably linked to a 3'-end 23-mer RSS, and the human D_{H}2-15 gene segment operably linked to a 3'-end 23-mer RSS.
74. The method of claim of any one of [67-73], wherein the immunoglobulin heavy chain variable region
   (a) further comprises one or more rodent Adam6 genes, optionally wherein the one or more rodent Adam6 genes are located between two unrearranged V_{H} gene segments, optionally between an unrearranged human V_{H}1-2 gene segment and an unrearranged human V_{H}6-1 gene segment, and/or
   (b) is operably linked to an immunoglobulin heavy chain constant region, optionally wherein the immunoglobulin heavy chain constant region is an endogenous rodent immunoglobulin heavy chain constant region, optionally an endogenous rodent immunoglobulin heavy chain constant region at an endogenous immunoglobulin heavy chain locus.
75. The method of any one of [65-74], wherein the rodent is a rat or a mouse.
76. A method of producing an antibody or obtaining a nucleic acid encoding same, the method comprising
   immunizing a rodent with an antigen, which rodent comprises a germline genome comprising an engineered D_{H} region that comprises one or more D_{H} segments that are each operably linked to a 23-mer RSS, optionally wherein the rodent is the rodent accordingly to any one of [58-64] and
   allowing the rodent to produce an immune response to the antigen including an antibody, or nucleic acid encoding same, that binds the antigen.
77. The method of [76], further comprising recovering the antibody, or nucleic acid encoding same, from the rodent or a rodent cell.
78. The method of [76], wherein the rodent cell is a B cell or a hybridoma.
79. The method of any one of [76-78], wherein one or more D_{H} segments that are each operably linked to a 23-mer RSS comprises a D_{H} gene segment operably linked to a 5' 23-mer RSS.
80. The method of any one of [76-79], wherein the one or more D_{H} segments that are each operably linked to a 23-mer RSS comprises a D_{H} gene segment operably linked to a 3' 23-mer RSS.
81. The method of any one of [76-80], wherein the rodent is a rat or a mouse.
82. A rodent genome, nucleic acid or immunoglobulin locus comprising a rearranged heavy chain V_{H}(D_{H}A-D_{H}B)J_{H} coding sequence operably linked to a rodent heavy chain constant region gene sequence, optionally wherein the rearranged heavy chain is somatically hypermutated and/or determined to be a V_{H}(D_{H}A-D_{H}B)J_{H} coding sequence since each of D_{H}A and D_{H}B respectively shows 40% identity to a first and second germline D_{H} gene segment, with a maximum of 1 nucleotide mutation.
83. The rodent genome, nucleic acid or immunoglobulin locus of [82], wherein the D_{H}B gene segment is derived from a human germline D_{H}3-3 gene segment.
84. The rodent genome, nucleic acid or immunoglobulin locus of [82], wherein the D_{H}A gene segment is derived from a human germline D_{H}2-2, D_{H}2-8 or D_{H}2-15 gene segment.
85. The rodent genome, nucleic acid or immunoglobulin locus of any one of [82-84], wherein the rearranged heavy chain V_{H}(D_{H}A-D_{H}B)J_{H} coding sequence encodes a CDR3 length of over 20 amino acids.
86. The rodent genome, nucleic acid or immunoglobulin locus of any one of [82-85], wherein the J_{H} gene segment is derived from a human germline J_{H}6 gene segment.
87. A rodent or rodent cell comprising the genome, nucleic acid, or immunoglobulin locus of any one of [82-86].
88. The rodent or rodent cell of [87], wherein the rodent is a rat or a mouse or the rodent cell is a rat cell or a mouse cell.
89. The rodent or rodent cell of [87] or [88], wherein the rodent cell is a rodent B cell.
90. A hybridoma comprising the rodent B cell of [89] fused with a myeloma cell.

## Claims

1. A method of obtaining a nucleic acid encoding an immunoglobulin heavy chain variable region, the method comprising obtaining from a rodent a nucleic acid comprising a rearranged heavy chain V_{H}(D_{H}A - D_{H}B)J_{H} coding nucleotide sequence, wherein the rodent comprises a germline genome comprising:
an engineered immunoglobulin heavy chain diversity (D_{H}) region ("engineered D_{H} region") comprising:
(i) a D_{H} gene segment immediately adjacent to a 23-mer RSS ("engineered D_{H} gene segment"); and
(ii) an unrearranged D_{H} gene segment flanked on its 5' end by a first 12-mer RSS and on its 3' end by a second 12-mer RSS ("unrearranged D_{H} gene segment"),
wherein (i) the engineered D_{H} gene segment and (ii) the unrearranged D_{H} gene segment are operably linked such that (i) the engineered D_{H} gene segment and (ii) the unrearranged D_{H} gene segment are able to join in a D_{H}-D_{H} recombination event according to the 12/23 rule,
wherein the D_{H}A-D_{H}B nucleotide sequence is derived from the engineered D_{H} region following the D_{H}-D_{H} recombination event,
optionally wherein the method further comprises immunizing the rodent with an antigen and allowing the rodent to produce an immune response to the antigen.

2. The method of claim 1, wherein D_{H}A or D_{H}B is derived from the D_{H} gene segment operably linked to a 23-mer RSS or a portion thereof and at least 9 base pairs of D_{H}A or D_{H}B is identical to at least 9 base pairs of the D_{H} gene segment operably linked to a 23-mer RSS or a portion thereof.

3. The method of claim 1, wherein each of D_{H}A and D_{H}B respectively shows 40% identity to a first and second germline D_{H} gene segment, with a maximum of 1 nucleotide mutation, optionally wherein the rearranged heavy chain V_{H}(D_{H}A D_{H}B)J_{H} coding nucleotide sequence is somatically hypermutated.

4. The method of any one of the preceding claims, wherein the rearranged heavy chain V_{H}(D_{H}A - D_{H}B)J_{H} coding nucleotide sequence comprises a human V_{H} gene segment, a human engineered D_{H} gene segment, a human unrearranged D_{H} gene segment, and a human J_{H} gene segment, optionally wherein:
(a) the D_{H}B gene segment is derived from the engineered D_{H} gene segment comprising a human germline D_{H}3-3 gene segment, optionally wherein the human J_{H} gene segment comprises a human germline J_{H}4 gene segment, a human germline J_{H}5 gene segment, or a human germline J_{H}6 gene segment; and/or
(b) the D_{H}A gene segment is derived from the engineered D_{H} gene segment comprising a human germline D_{H}2-2 gene segment, a human germline D_{H}2-8 gene segment, or a human germline D_{H}2-15 gene segment, optionally wherein the human J_{H} gene segment comprises a human germline J_{H}1 gene segment, a human germline J_{H}2 gene segment, a human germline J_{H}3 gene segment, a human germline J_{H}4 gene segment, a human germline J_{H}5 gene segment, or a human germline J_{H}6 gene segment.

5. The method of any one of the preceding claims, wherein the rearranged heavy chain V_{H}(D_{H}A - D_{H}B)J_{H} coding nucleotide sequence encodes a complementary determining region 3 (CDR3) length of over 20 amino acids.

6. The method of any one of the preceding claims, wherein the rearranged heavy chain V_{H}(D_{H}A - D_{H}B)J_{H} coding nucleotide sequence is operably linked to an immunoglobulin heavy chain constant region (C_{H}) nucleotide sequence or a portion thereof.

7. The method of claim 6, wherein the C_{H} nucleotide sequence or portion thereof is a rodent C_{H} nucleotide sequence or portion thereof.

8. The method of claim 7, wherein the rodent C_{H} nucleotide sequence or portion thereof comprises a rodent intronic enhancer region and a rodent IgM C_{H} nucleotide sequence.

9. A nucleic acid encoding an immunoglobulin heavy chain variable region, obtainable according to the method of any one of the preceding claims.

10. An immunoglobulin heavy chain locus comprising the nucleic acid of claim 9.

11. A rodent somatic genome comprising the nucleic acid of claim 9 or the immunoglobulin heavy chain locus of claim 10.

12. A somatic cell comprising in its genome the nucleic acid of claim 9, the immunoglobulin heavy chain locus of claim 10, or the rodent somatic genome of claim 11.

13. The somatic cell of claim 12, wherein the somatic cell is: (i) a rodent somatic cell; (ii) a rat somatic cell; or (iii) a mouse somatic cell.

14. The somatic cell of claim 12 or 13, wherein the somatic cell is a B cell.

15. A hybridoma comprising the B cell of claim 14 fused with a myeloma.
